# EUROPEAN PATENT SPECIFICATION

(11) **EP 3 021 372 B1**
(45) Date of publication and mention of the grant of the patent: **18.03.2020**
(21) Application number: 14823323.2
(22) Date of filing: 01.07.2014
(51) Int. Cl.: H01L 51/54, C07C 211/54

(54) **ORGANIC ELECTROLUMINESCENT ELEMENT**
ORGANISCHES ELEKTROLUMINESZENTES ELEMENT
ÉLÉMENT ÉLECTROLUMINESCENT ORGANIQUE

(30) Priority: 12.07.2013 JP 2013146010
(43) Date of publication of application: 18.05.2016
(60) Divisional application: 20154629.8
(73) Proprietor: Hodogaya Chemical Co., Ltd., Chuo-ku Tokyo 104-0028 (JP)
(72) Inventor: YOKOYAMA, Norimasa, Tokyo 104-0028 (JP); HAYASHI, Shuichi, Tokyo 104-0028 (JP); KABASAWA, Naoaki, Tokyo 104-0028 (JP)
(74) Representative: Müller-Boré & Partner Patentanwälte PartG mbB
(86) International application number: PCT/JP2014/003498
(87) International publication number: WO 2015/004875

(56) References cited:
- EP-A1- 2 500 346
- EP-A1- 2 527 334
- WO-A1-2006/114921
- WO-A1-2011/049123
- WO-A1-2011/059000
- WO-A1-2011/090149
- WO-A2-03/060956
- JP-A- 2008 016 827
- JP-A- 2011 088 836
- US-A1- 2006 232 198

## Description

The present invention relates to an organic electroluminescent device which is a preferred self-luminous device for various display devices. Specifically, this invention relates to organic electroluminescent devices (hereinafter referred to as organic EL devices) using specific arylamine compounds (and specific compounds having an anthracene ring structure).

The organic EL device is a self-luminous device and has been actively studied for their brighter, superior visibility and the ability to display clearer images in comparison with liquid crystal devices.

In 1987, C. W. Tang and colleagues at Eastman Kodak developed a laminated structure device using materials assigned with different roles, realizing practical applications of an organic EL device with organic materials. These researchers laminated an electron-transporting phosphor and a hole-transporting organic substance, and injected both charges into a phosphor layer to cause emission in order to obtain a high luminance of 1,000 cd/m² or more at a voltage of 10 V or less (refer to Patent Documents 1 and 2, for example).

To date, various improvements have been made for practical applications of the organic EL device. Various roles of the laminated structure are further subdivided to provide an electroluminescence device that includes an anode, a hole injection layer, a hole transport layer, a light emitting layer, an electron transport layer, an electron injection layer, and a cathode successively formed on a substrate, and high efficiency and durability have been achieved by the electroluminescence device (refer to Non-Patent Document 1, for example).

Further, there have been attempts to use triplet excitons for further improvements of luminous efficiency, and the use of a phosphorescence-emitting compound has been examined (refer to Non-Patent Document 2, for example).

Devices that use light emission caused by thermally activated delayed fluorescence (TADF) have also been developed. In 2011, Adachi et al. at Kyushu University, National University Corporation realized 5.3% external quantum efficiency with a device using a thermally activated delayed fluorescent material (refer to Non-Patent Document 3, for example).

The light emitting layer can be also fabricated by doping a charge-transporting compound generally called a host material, with a fluorescent compound, a phosphorescence-emitting compound, or a delayed fluorescent-emitting material. As described in the Non-Patent Document, the selection of organic materials in an organic EL device greatly influences various device characteristics such as efficiency and durability (refer to Non-Patent Document 2, for example).

In an organic EL device, charges injected from both electrodes recombine in a light emitting layer to cause emission. What is important here is how efficiently the hole and electron charges are transferred to the light emitting layer in order to form a device having excellent carrier balance. The probability of hole-electron recombination can be improved by improving hole injectability and electron blocking performance of blocking injected electrons from the cathode, and high luminous efficiency can be obtained by confining excitons generated in the light emitting layer. The role of a hole transport material is therefore important, and there is a need for a hole transport material that has high hole injectability, high hole mobility, high electron blocking performance, and high durability to electrons.

Heat resistance and amorphousness of the materials are also important with respect to the lifetime of the device. The materials with low heat resistance cause thermal decomposition even at a low temperature by heat generated during the drive of the device, which leads to the deterioration of the materials. The materials with low amorphousness cause crystallization of a thin film even in a short time and lead to the deterioration of the device. The materials in use are therefore required to have characteristics of high heat resistance and satisfactory amorphousness.

N,N'-diphenyl-N,N'-di(α-naphthyl)benzidine (hereinafter referred to as NPD) and various aromatic amine derivatives are known as the hole transport materials used for the organic EL device (refer to Patent Documents 1 and 2, for example). Although NPD has desirable hole transportability, its glass transition point (Tg), which is an index of heat resistance, is as low as 96°C, which causes the degradation of device characteristics by crystallization under a high-temperature condition (refer to Non-Patent Document 4, for example). The aromatic amine derivatives described in the Patent Documents include a compound known to have an excellent hole mobility of 10⁻³ cm²/Vs or higher (refer to Patent Documents 1 and 2, for example). However, since the compound is insufficient in terms of electron blocking performance, some of the electrons pass through the light emitting layer, and improvements in luminous efficiency cannot be expected. For such a reason, a material with higher electron blocking performance, a more stable thin-film state and higher heat resistance is needed for higher efficiency. Although an aromatic amine derivative having high durability is reported (refer to Patent Document 3, for example), the derivative is used as a charge transporting material used in an electrophotographic photoconductor, and there is no example of using the derivative in the organic EL device.

Arylamine compounds having a substituted carbazole structure are proposed as compounds improved in the characteristics such as heat resistance and hole injectability (refer to Patent Documents 4 and 5, for example). However, while the devices using these compounds for the hole injection layer or the hole transport layer have been improved in heat resistance, luminous efficiency and the like, the improvements are still insufficient. Further lower driving voltage and higher luminous efficiency are therefore needed.

In order to improve characteristics of the organic EL device and to improve the yield of the device production, it has been desired to develop a device having high luminous efficiency, low driving voltage and a long lifetime by using in combination the materials that excel in hole and electron injection/transport performances, stability as a thin film and durability, permitting holes and electrons to be highly efficiently recombined together.

Further, in order to improve characteristics of the organic EL device, it has been desired to develop a device that maintains carrier balance and has high efficiency, low driving voltage and a long lifetime by using in combination the materials that excel in hole and electron injection/transport performances, stability as a thin film and durability.

Patent Document 11 describes aromatic amine derivatives and an organic electroluminescent element comprising same.

Patent document 12 concerns organic electroluminescent devices comprising aromatic triamine compounds.

Patent Document 1: JP-A-8-048656
Patent Document 2: Japanese Patent No. 3194657
Patent Document 3: Japanese Patent No. 4943840
Patent Document 4: JP-A-2006-151979
Patent Document 5: WO2008/62636
Patent Document 6: JP-A-7-126615
Patent Document 7: JP-A-8-048656
Patent Document 8: JP-A-2005-108804
Patent Document 9: WO2011/059000
Patent Document 10: WO2003/060956
Patent Document 11: EP 2 527 334 A1
Patent Document 12 : US2006/0232198

Non-Patent Document 1: The Japan Society of Applied Physics, 9th Lecture Preprints, pp. 55 to 61 (2001)
Non-Patent Document 2: The Japan Society of Applied Physics, 9th Lecture Preprints, pp. 23 to 31 (2001)
Non-Patent Document 3: Appl. Phys. Let., 98, 083302 (2011)
Non-Patent Document 4: Organic EL Symposium, the 3rd Regular presentation Preprints, pp. 13 to 14 (2006)

An object of the present invention is to provide an organic EL device having high efficiency, low driving voltage and a long lifetime, by combining various materials for an organic EL device, which are excellent, as materials for an organic EL device having high efficiency and high durability, in hole and electron injection/transport performances, electron blocking ability, stability in a thin-film state and durability, so as to allow the respective materials to effectively reveal their characteristics.

Physical properties of the organic compound to be provided by the present invention include (1) good hole injection characteristics, (2) large hole mobility, (3) excellent electron blocking ability, (4) stability in a thin-film state, and (5) excellent heat resistance. Physical properties of the organic EL device to be provided by the present invention include (1) high luminous efficiency and high power efficiency, (2) low turn on voltage, (3) low actual driving voltage, and (4) a long lifetime.

To achieve the above object, the present inventors have noted that an arylamine material is excellent in hole injection and transport abilities, stability as a thin film and durability, have selected two specific kinds of arylamine compounds, and have produced various organic EL devices by combining a first hole transport material and a second hole transport material such that holes can be efficiently injected and transported into a light emitting layer. Then, they have intensively conducted characteristic evaluations of the devices. Also, they have noted that compounds having an anthracene ring structure are excellent in electron injection and transport abilities, stability as a thin film and durability, have selected two specific kinds of arylamine compounds and specific compounds having an anthracene ring structure, and have produced various organic EL devices by combining those compounds in good carrier balance. Then, they have intensively conducted characteristic evaluations of the devices. As a result, they have completed the present invention.

Specifically, according to the present invention, the following organic EL devices are provided.
1) An organic EL device having at least an anode, a hole injection layer, a first hole transport layer, a second hole transport layer, a light emitting layer, an electron transport layer and a cathode in this order, wherein the second hole transport layer includes an arylamine compound represented by the following general formula (1c-a) or (1c-b). 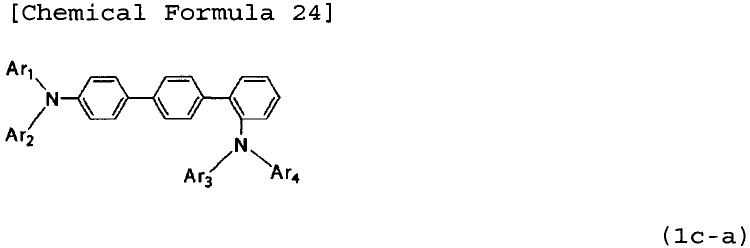 In the formula, Ar₁ to Ar₄ may be the same or different, and represent a substituted or unsubstituted aromatic hydrocarbon group, a substituted or unsubstituted aromatic heterocyclic group, or a substituted or unsubstituted condensed polycyclic aromatic group. 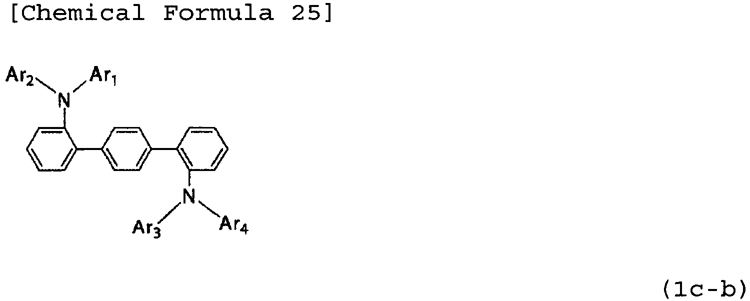 In the formula, Ar₁ to Ar₄ may be the same or different, and represent a substituted or unsubstituted aromatic hydrocarbon group, a substituted or unsubstituted aromatic heterocyclic group, or a substituted or unsubstituted condensed polycyclic aromatic group.
2) The organic EL device of 1), wherein the first hole transport layer includes an arylamine compound having a structure in which three to six triphenylamine structures are joined within a molecule via a single bond or a divalent group that does not contain a heteroatom.
3) The organic EL device of 2), wherein the arylamine compound having a structure in which three to six triphenylamine structures are joined within a molecule via a single bond or a divalent group that does not contain a heteroatom is an arylamine compound of the following general formula (2) having four triphenylamine structures within a molecule. 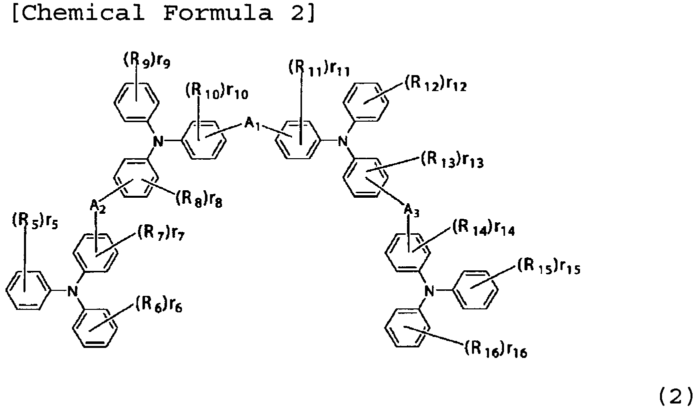 In the formula, R₁ to R₁₂ represent a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy. r₁ to r₁₂ may be the same or different, r₁, r₂, r₅, r₈, r₁₁, and r₁₂ representing 0 or an integer of 1 to 5, and r₃, r₄, r₆, r₇, r₉, and r₁₀ representing 0 or an integer of 1 to 4. When r₁, r₂, r₅, r₈, r₁₁, and r₁₂ are an integer of 2 to 5, or when r₃, r₄, r₆, r₇, r₉, and r₁₀ are an integer of 2 to 4, R₁ to R₁₂, a plurality of which bind to the same benzene ring, may be the same or different and may bind to each other via a single bond, substituted or unsubstituted methylene, an oxygen atom, or a sulfur atom to form a ring. A₁, A₂, and A₃ may be the same or different, and represent a divalent group represented by the following structural formulae (B) to (G), or a single bond. 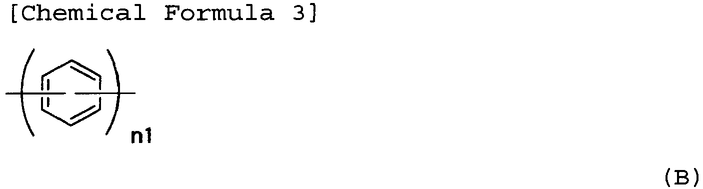 In the formula, nl represents an integer of 1 to 3. 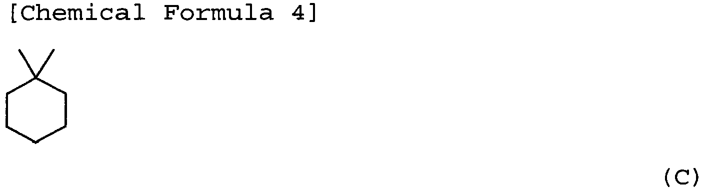 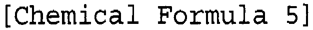 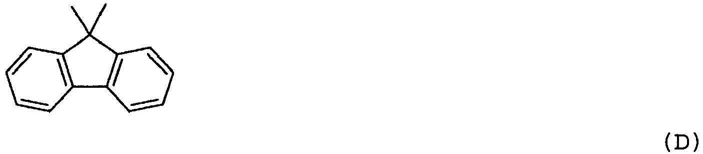 [Chemical Formula 6]

   -CH₂- (E) 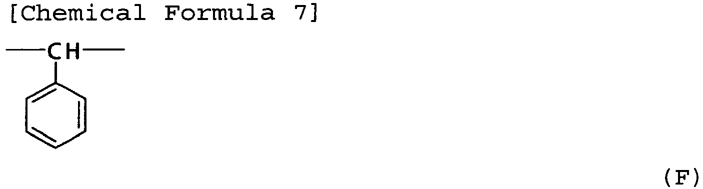 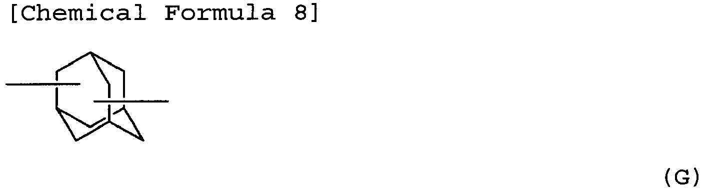
4) The organic EL device of 1), wherein the first hole transport layer includes an arylamine compound having a structure in which two triphenylamine structures are joined within a molecule via a single bond or a divalent group that does not contain a heteroatom.
5) The organic EL device of 4), wherein the arylamine compound having a structure in which two triphenylamine structures are joined within a molecule via a single bond or a divalent group that does not contain a heteroatom is an arylamine compound represented by the following general formula (3). 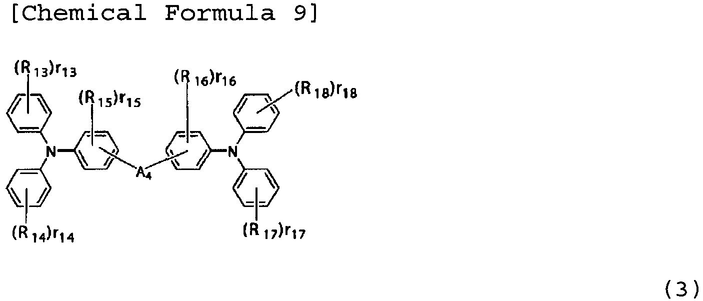 In the formula, R₁₃ to R₁₈ represent a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy. r₁₃ to r₁₈ may be the same or different, r₁₃, r₁₄, r₁₇, and r₁₈ representing 0 or an integer of 1 to 5, and r₁₅ and r₁₆ representing 0 or an integer of 1 to 4. When r₁₃, r₁₄, r₁₇, and r₁₈ are an integer of 2 to 5, or when r₁₅ and r₁₆ are an integer of 2 to 4, R₁₃ to R₁₈, a plurality of which bind to the same benzene ring, may be the same or different and may bind to each other via a single bond, substituted or unsubstituted methylene, an oxygen atom, or a sulfur atom to form a ring. A₄ represents a divalent group represented by the following structural formulae (C) to (G), or a single bond. 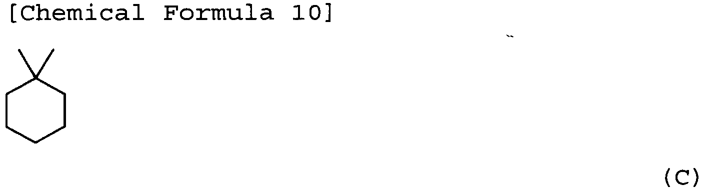 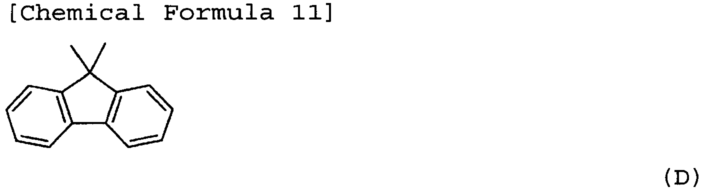 [Chemical Formula 12]

   -CH₂- (E) 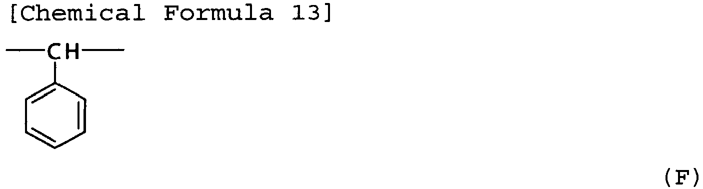 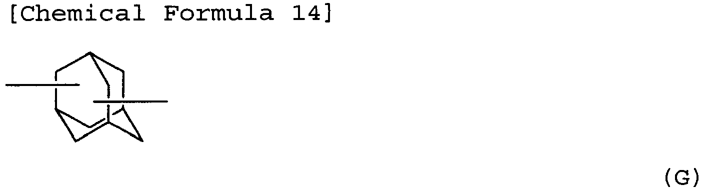
6) The organic EL device of 1), wherein the electron transport layer includes a compound of the following general formula (4) having an anthracene ring structure. 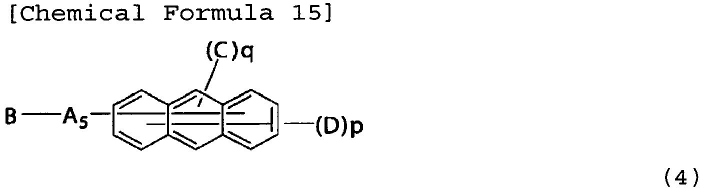 In the formula, A₅ represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond. B represents a substituted or unsubstituted aromatic heterocyclic group. C represents a substituted or unsubstituted aromatic hydrocarbon group, a substituted or unsubstituted aromatic heterocyclic group, or a substituted or unsubstituted condensed polycyclic aromatic group. D may be the same or different, and represents a hydrogen atom, a deuterium atom, a fluorine atom, a chlorine atom, cyano, trifluoromethyl, linear or branched alkyl of 1 to 6 carbon atoms, a substituted or unsubstituted aromatic hydrocarbon group, a substituted or unsubstituted aromatic heterocyclic group, or a substituted or unsubstituted condensed polycyclic aromatic group. p represents 7 or 8, and q represents 1 or 2 while maintaining a relationship that a sum of p and q is 9.
7) The organic EL device of 6), wherein the compound having an anthracene ring structure is a compound of the following general formula (4a) having an anthracene ring structure. 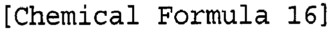 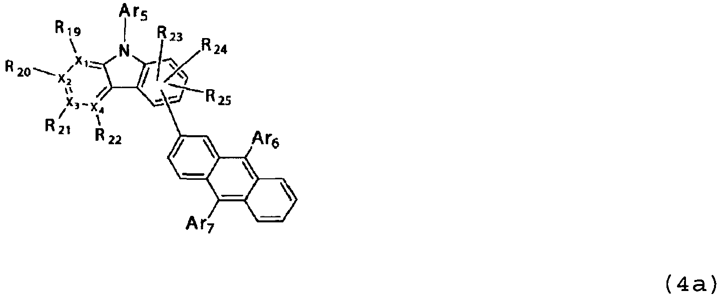 In the formula, Ars, Ar₆, and Ar₇ may be the same or different, and represent a substituted or unsubstituted aromatic hydrocarbon group, a substituted or unsubstituted aromatic heterocyclic group, or a substituted or unsubstituted condensed polycyclic aromatic group. R₁₉ to R₂₅ may be the same or different, and represent a hydrogen atom, a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, where R₁₉ to R₂₅ may bind to each other via a single bond, substituted or unsubstituted methylene, an oxygen atom, or a sulfur atom to form a ring. X₁, X₂, X₃, and X₄ represent a carbon atom or a nitrogen atom, where only one of X₁, X₂, X₃, and X₄ is a nitrogen atom, and, in this case, the nitrogen atom does not have the hydrogen atom or substituent for R₁₉ to R₂₂.
8) The organic EL device of 6), wherein the compound having an anthracene ring structure is a compound of the following general formula (4b) having an anthracene ring structure. 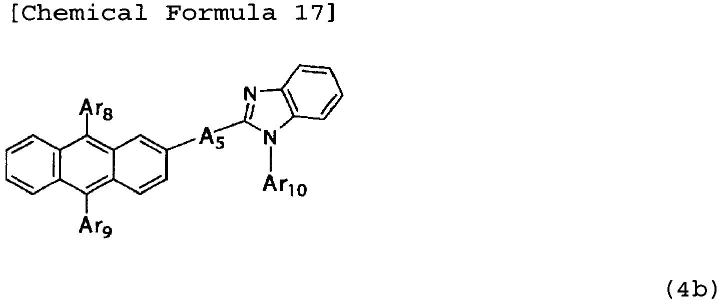 In the formula, A₅ represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond. Ar₈, Ar₉, and Ar₁₀ may be the same or different, and represent a substituted or unsubstituted aromatic hydrocarbon group, a substituted or unsubstituted aromatic heterocyclic group, or a substituted or unsubstituted condensed polycyclic aromatic group.

Specific examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1c-a) or (1c-b) include phenyl, biphenylyl, terphenylyl, naphthyl, anthryl, phenanthryl, fluorenyl, indenyl, pyrenyl, perylenyl, fluoranthenyl, triphenylenyl, pyridyl, furyl, pyrrolyl, thienyl, quinolyl, isoquinolyl, benzofuranyl, benzothienyl, indolyl, carbazolyl, benzoxazolyl, benzothiazolyl, quinoxalyl, benzoimidazolyl, pyrazolyl, dibenzofuranyl, dibenzothienyl, and carbolinyl. Ar₁ and Ar₂, or Ar₃ and Ar₄ may bind to each other via a single bond, substituted or unsubstituted methylene, an oxygen atom, or a sulfur atom to form a ring.

Specific examples of the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b) include a deuterium atom; cyano; nitro; halogen atoms such as a fluorine atom, a chlorine atom, a bromine atom, and an iodine atom; linear or branched alkyls of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxys of 1 to 6 carbon atoms such as methyloxy, ethyloxy, and propyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzoxazolyl, benzothiazolyl, quinoxalyl, benzoimidazolyl, pyrazolyl, dibenzofuranyl, dibenzothienyl, and carbolinyl; arylvinyls such as styryl and naphthylvinyl; and acyls such as acetyl and benzoyl. These substituents may be further substituted with the exemplified substituents above.

These substituents may bind to each other via a single bond, substituted or unsubstituted methylene, an oxygen atom, or a sulfur atom to form a ring.

The "aromatic heterocyclic group" in the "substituted or unsubstituted aromatic heterocyclic group" represented by Ar₁ to Ar₄ in the general formula (1c-a) or (1c-b) is preferably a sulfur-containing aromatic heterocyclic group such as thienyl, benzothienyl, benzothiazolyl, or dibenzothienyl; an oxygen-containing aromatic heterocyclic group such as furyl, benzofuranyl, benzoxazolyl, or dibenzofuranyl; or an N-substituted carbazolyl group having a substituent selected from the above exemplified aromatic hydrocarbon groups and condensed polycyclic aromatic groups.

Ar₁ to Ar₄ in the general formula (1c-a) or (1c-b) are preferably the "substituted or unsubstituted aromatic hydrocarbon group", the "substituted or unsubstituted sulfur-containing aromatic heterocyclic group", or the "substituted or unsubstituted condensed polycyclic aromatic group", far preferably, phenyl, biphenylyl, terphenylyl, naphthyl, phenanthryl, triphenylenyl, fluorenyl, or dibenzothienyl.

It is preferable that Ar₁ and Ar₂, or Ar₃ and Ar₄ in the general formula (1c-a) or (1c-b) be different groups, and it is far preferable that Ar₁ and Ar₂, and Ar₃ and Ar₄ in the general formula (1c-a) or (1c-b) be different groups. As bonding patterns of phenylene groups in the general formula (1c-a) or (1c-b), the bonding patterns include 1,2-bonding from the viewpoint of thin film stability influencing a device lifetime. The skeletons are those in which the phenylene groups are not bonded linearly, i.e. a 2,4''-diamino-[1,1';4',1"]terphenyl skeleton, and a 2,2"-diamino-[1,1';4',1"]terphenyl skeleton.

Specific examples of the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₁ to R₁₂ in the general formula (2) include methyl, ethyl, n-propyl, isopropyl, n-butyl, isobutyl, tert-butyl, n-pentyl, isopentyl, neopentyl, n-hexyl, cyclopentyl, cyclohexyl, 1-adamantyl, 2-adamantyl, vinyl, allyl, isopropenyl, and 2-butenyl. When a plurality of these groups bind to the same benzene ring (when r₁, r₂, r₅, r₈, r₁₁, or r₁₂ is an integer of 2 to 5, or when r₃, r₄, r₆, r₇, r₉, or r₁₀ is an integer of 2 to 4), these groups may bind to each other via a single bond, substituted or unsubstituted methylene, an oxygen atom, or a sulfur atom to form a ring.

Examples of the "substituent" in the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₁ to R₁₂ in the general formula (2) include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

Specific examples of the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₁ to R₁₂ in the general formula (2) include methyloxy, ethyloxy, n-propyloxy, isopropyloxy, n-butyloxy, tert-butyloxy, n-pentyloxy, n-hexyloxy, cyclopentyloxy, cyclohexyloxy, cycloheptyloxy, cyclooctyloxy, 1-adamantyloxy, and 2-adamantyloxy. When a plurality of these groups bind to the same benzene ring (when r₁, r₂, r₅, r₈, r₁₁, or r₁₂ is an integer of 2 to 5, or when r₃, r₄, r₆, r₇, r₉, or r₁₀ is an integer of 2 to 4), these groups may bind to each other via a single bond, substituted or unsubstituted methylene, an oxygen atom, or a sulfur atom to form a ring.

Examples of the "substituent" in the "linear or branched alkyloxy of 1 to 6 carbon atoms that has a substituent" or the "cycloalkyloxy of 5 to 10 carbon atoms that has a substituent" represented by R₁ to R₁₂ in the general formula (2) include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₁ to R₁₂ in the general formula (2) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1c-a) or (1c-b). When a plurality of these groups bind to the same benzene ring (when r₁, r₂, r₅, r₈, r₁₁, or r₁₂ is an integer of 2 to 5, or when r₃, r₄, r₆, r₇, r₉, or r₁₀ is an integer of 2 to 4), these groups may bind to each other via a single bond, substituted or unsubstituted methylene, an oxygen atom, or a sulfur atom to form a ring.

Examples of the "substituent" in the "substituted aromatic hydrocarbon group", the "substituted aromatic heterocyclic group", or the "substituted condensed polycyclic aromatic group" represented by R₁ to R₁₂ in the general formula (2) include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

Specific examples of the "aryloxy" in the "substituted or unsubstituted aryloxy" represented by R₁ to R₁₂ in the general formula (2) include phenyloxy, biphenylyloxy, terphenylyloxy, naphthyloxy, anthryloxy, phenanthryloxy, fluorenyloxy, indenyloxy, pyrenyloxy, and perylenyloxy. When a plurality of these groups bind to the same benzene ring (when r₁, r₂, r₅, r₈, r₁₁, or r₁₂ is an integer of 2 to 5, or when r₃, r₄, r₆, r₇, r₉, or r₁₀ is an integer of 2 to 4), these groups may bind to each other via a single bond, substituted or unsubstituted methylene, an oxygen atom, or a sulfur atom to form a ring.

Examples of the "substituent" in the "substituted aryloxy" represented by R₁ to R₁₂ in the general formula (2) include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

In the general formula (2), r₁ to r₁₂ may be the same or different, r₁, r₂, r₅, r₈, r₁₁, and r₁₂ representing 0 or an integer of 1 to 5, and r₃, r₄, r₆, r₇, r₉, and r₁₀ representing 0 or an integer of 1 to 4. When r₁, r₂, r₃, r₄, r₅, r₆, r₇, r₈, r₉, r₁₀, r₁₁, or r₁₂ is 0, R₁, R₂, R₃, R₄, R₅, R₆, R₇, R₈, R₉, R₁₀, R₁₁, or R₁₂ on the benzene ring does not exist, that is, the benzene ring is not substituted by a group represented by R₁, R₂, R₃, R₄, R₅, R₆, R₇, R₈, R₉, R₁₀, R₁₁, or R₁₂.

Examples of the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₁₃ to R₁₈ in the general formula (3) include the same groups exemplified as the groups for the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₁ to R₁₂ in the general formula (2), and possible embodiments may also be the same embodiments as the exemplified embodiments.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

Examples of the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₁₃ to R₁₈ in the general formula (3) include the same groups exemplified as the groups for the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₁ to R₁₂ in the general formula (2), and possible embodiments may also be the same embodiments as the exemplified embodiments.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₁₃ to R₁₈ in the general formula (3) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1c-a) or (1c-b). These groups may bind to each other via a single bond, substituted or unsubstituted methylene, an oxygen atom, or a sulfur atom to form a ring.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

Examples of the "aryloxy" in the "substituted or unsubstituted aryloxy" represented by R₁₃ to R₁₈ in the general formula (3) include the same groups exemplified as the groups for the "aryloxy" in the "substituted or unsubstituted aryloxy" represented by R₁ to R₁₂ in the general formula (2), and possible embodiments may also be the same embodiments as the exemplified embodiments.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

In the general formula (3), r₁₃ to r₁₈ may be the same or different, r₁₃, r₁₄, r₁₇, and r₁₈ representing 0 or an integer of 1 to 5, and r₁₅ and r₁₆ representing 0 or an integer of 1 to 4. When r₁₃, r₁₄, r₁₅, r₁₆, r₁₇, or r₁₈ is 0, R₁₃, R₁₄, R₁₅, R₁₆, R₁₇, or R₁₈ on the benzene ring does not exist, that is, the benzene ring is not substituted by a group represented by R₁₃, R₁₄, R₁₅, R₁₆, R₁₇, or R₁₈.

Specific examples of the "aromatic hydrocarbon", the "aromatic heterocyclic ring", or the "condensed polycyclic aromatics" of the "substituted or unsubstituted aromatic hydrocarbon", the "substituted or unsubstituted aromatic heterocyclic ring", or the "substituted or unsubstituted condensed polycyclic aromatics" in the "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by A₅ in the general formula (4) include benzene, biphenyl, terphenyl, tetrakisphenyl, styrene, naphthalene, anthracene, acenaphthylene, fluorene, phenanthrene, indane, pyrene, pyridine, pyrimidine, triazine, pyrrole, furan, thiophene, quinoline, isoquinoline, benzofuran, benzothiophene, indoline, carbazole, carboline, benzoxazole, benzothiazole, quinoxaline, benzimidazole, pyrazole, dibenzofuran, dibenzothiophene, naphthyridine, phenanthroline, and acridine.

The "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by A₅ in the general formula (4) is a divalent group that results from the removal of two hydrogen atoms from the above "aromatic hydrocarbon", "aromatic heterocyclic ring", or "condensed polycyclic aromatics".

Examples of the "substituent" of the "substituted aromatic hydrocarbon", the "substituted aromatic heterocyclic ring", or the "substituted condensed polycyclic aromatics" in the "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by A₅ in the general formula (4) include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

Specific examples of the "aromatic heterocyclic group" in the "substituted or unsubstituted aromatic heterocyclic group" represented by B in the general formula (4) include pyridyl, pyrimidyl, furyl, pyrrolyl, thienyl, quinolyl, isoquinolyl, benzofuranyl, benzothienyl, indolyl, carbazolyl, benzoxazolyl, benzothiazolyl, quinoxalyl, benzoimidazolyl, pyrazolyl, dibenzofuranyl, dibenzothienyl, and carbolinyl.

Specific examples of the "substituent" in the "substituted aromatic heterocyclic group" represented by B in the general formula (4) include a deuterium atom; cyano; nitro; halogen atoms such as a fluorine atom, a chlorine atom, a bromine atom, and an iodine atom; linear or branched alkyls of 1 to 6 carbon atoms such as methyl, ethyl, n-propyl, isopropyl, n-butyl, isobutyl, tert-butyl, n-pentyl, isopentyl, neopentyl, and n-hexyl; cycloalkyls of 5 to 10 carbon atoms such as cyclopentyl, cyclohexyl, 1-adamantyl, and 2-adamantyl; linear or branched alkyloxys of 1 to 6 carbon atoms such as methyloxy, ethyloxy, and propyloxy; cycloalkyloxys of 5 to 10 carbon atoms such as cyclopentyloxy, cyclohexyloxy, 1-adamantyloxy, and 2-adamantyloxy; alkenyls such as allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthryl, fluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzoxazolyl, benzothiazolyl, quinoxalyl, benzoimidazolyl, pyrazolyl, dibenzofuranyl, dibenzothienyl, and carbolinyl; aryloxys such as phenyloxy, biphenylyloxy, naphthyloxy, anthryloxy, and phenanthryloxy; arylvinyls such as styryl and naphthylvinyl; and acyls such as acetyl and benzoyl. These substituents may be further substituted with the exemplified substituents above.

These substituents may bind to each other via a single bond, substituted or unsubstituted methylene, an oxygen atom, or a sulfur atom to form a ring.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by C in the general formula (4) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1c-a) or (1c-b). When a plurality of these groups bind to the same anthracene ring (when q is 2), these groups may be the same or different.

Examples of the "substituent" in the "substituted aromatic hydrocarbon group", the "substituted aromatic heterocyclic group", or the "substituted condensed polycyclic aromatic group" represented by C in the general formula (4) include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

Specific examples of the "linear or branched alkyl of 1 to 6 carbon atoms" represented by D in the general formula (4) include methyl, ethyl, n-propyl, isopropyl, n-butyl, isobutyl, tert-butyl, n-pentyl, isopentyl, neopentyl, and n-hexyl. The plurality of D may be the same or different, and these groups may bind to each other via a single bond, substituted or unsubstituted methylene, an oxygen atom, or a sulfur atom to form a ring.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by D in the general formula (4) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1c-a) or (1c-b). The plurality of D may be the same or different, and these groups may bind to each other via a single bond, substituted or unsubstituted methylene, an oxygen atom, or a sulfur atom to form a ring.

Examples of the "substituent" in the "substituted aromatic hydrocarbon group", the "substituted aromatic heterocyclic group", or the "substituted condensed polycyclic aromatic group" represented by D in the general formula (4) include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

A₅ in the general formula (4) is preferably the "divalent group of a substituted or unsubstituted aromatic hydrocarbon", the "divalent group of substituted or unsubstituted condensed polycyclic aromatics", or a single bond, far preferably, a divalent group derived from benzene, biphenyl, terphenyl, naphthalene, anthracene, fluorene, or phenanthrene; or a single bond, particularly preferably, a divalent group derived from benzene, biphenyl, naphthalene, fluorene, or phenanthrene; or a single bond.

B in the general formula (4) is preferably a nitrogen-containing aromatic heterocyclic group such as pyridyl, pyrimidyl, pyrrolyl, quinolyl, isoquinolyl, indolyl, carbazolyl, benzoxazolyl, benzothiazolyl, quinoxalyl, benzoimidazolyl, pyrazolyl, or carbolinyl, far preferably, pyridyl, pyrimidyl, quinolyl, isoquinolyl, indolyl, benzoimidazolyl, pyrazolyl, or carbolinyl.

C in the general formula (4) is preferably the "substituted or unsubstituted aromatic hydrocarbon group" or the "substituted or unsubstituted condensed polycyclic aromatic group", far preferably, phenyl, biphenylyl, naphthyl, anthracenyl, phenanthryl, or fluorenyl.

In the general formula (4), A₅ is preferably bonded to the 2-position of the anthracene ring.

From the viewpoint of stability of a compound, it is preferable that q be 2 (p be 7).

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₅, Ar₆, and Ar₇ in the general formula (4a) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1c-a) or (1c-b).

Examples of the "substituent" in the "substituted aromatic hydrocarbon group", the "substituted aromatic heterocyclic group", or the "substituted condensed polycyclic aromatic group" represented by Ar₅, Ar₆, and Ar₇ in the general formula (4a) include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

Examples of the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₁₉ to R₂₅ in the general formula (4a) include the same groups exemplified as the groups for the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₁ to R₁₂ in the general formula (2), and possible embodiments may also be the same embodiments as the exemplified embodiments.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

Examples of the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₁₉ to R₂₅ in the general formula (4a) include the same groups exemplified as the groups for the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₁ to R₁₂ in the general formula (2), and possible embodiments may also be the same embodiments as the exemplified embodiments.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₁₉ to R₂₅ in the general formula (4a) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1c-a) or (1c-b). These groups may bind to each other via a single bond, substituted or unsubstituted methylene, an oxygen atom, or a sulfur atom to form a ring.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

Specific examples of the "aryloxy" in the "substituted or unsubstituted aryloxy" represented by R₁₉ to R₂₅ in the general formula (4a) include the same groups exemplified as the groups for the "aryloxy" in the "substituted or unsubstituted aryloxy" represented by R₁ to R₁₂ in the general formula (2), and possible embodiments may also be the same embodiments as the exemplified embodiments.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

In the general formula (4a), X₁, X₂, X₃, and X₄ represent a carbon atom or a nitrogen atom, and only one of X₁, X₂, X₃, and X₄ is a nitrogen atom. When one of X₁, X₂, X₃, and X₄ is a nitrogen atom, the nitrogen atom does not have the hydrogen atom or substituent for R₁₉ to R₂₂. That is, R₁₉ does not exist when X₁ is a nitrogen atom, R₂₀ does not exist when X₂ is a nitrogen atom, R₂₁ does not exist when X₃ is a nitrogen atom, and R₂₂ does not exist when X₄ is a nitrogen atom.

Ar₅, Ar₆, and Ar₇ in the general formula (4a) are preferably the "substituted or unsubstituted aromatic hydrocarbon group" or the "substituted or unsubstituted condensed polycyclic aromatic group", far preferably, phenyl, biphenylyl, naphthyl, anthracenyl, phenanthryl, or fluorenyl.

In the general formula (4a), it is preferable that only X₃ be a nitrogen atom (the group R₂₁ not exist) .

Among the compounds of the general formula (4a) having an anthracene ring structure, the compounds of the following general formula (4a') having an anthracene ring structure are preferable.

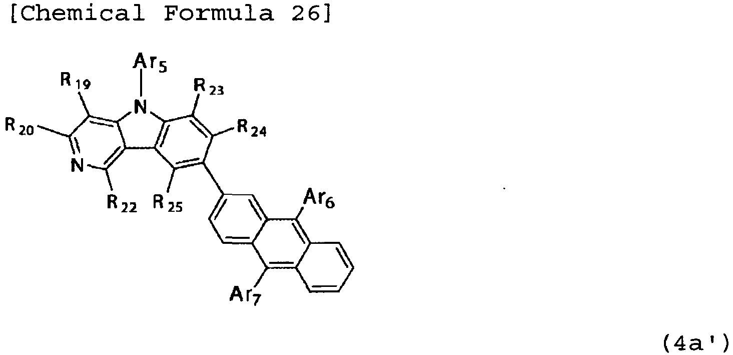

In the formula, Ar₅, Ar₆, Ar₇, R₁₉, R₂₀, and R₂₂ to R₂₅ are as defined in the general formula (4a).

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₈, Ar₉, and Ar₁₀ in the general formula (4b) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1c-a) or (1c-b).

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1c-a) or (1c-b), and possible embodiments may also be the same embodiments as the exemplified embodiments.

A₅ in the general formula (4b) is preferably the "divalent group of a substituted or unsubstituted aromatic hydrocarbon", the "divalent group of substituted or unsubstituted condensed polycyclic aromatics", or a single bond, far preferably, a divalent group derived from benzene, biphenyl, terphenyl, naphthalene, anthracene, fluorene, or phenanthrene, particularly preferably, a divalent group derived from benzene, biphenyl, naphthalene, fluorene, or phenanthrene.

Ar₈, Ar₉, and Ar₁₀ in the general formula (4b) are preferably the "substituted or unsubstituted aromatic hydrocarbon group" or the "substituted or unsubstituted condensed polycyclic aromatic group", far preferably, phenyl, biphenylyl, naphthyl, anthracenyl, phenanthryl, or fluorenyl.

In the structural formula (B) in the general formula (2), n1 represents an integer of 1 to 3.

With respect to p and q in the general formula (4), p represents 7 or 8, and q represents 1 or 2 while maintaining a relationship that a sum of p and q (p + q) is 9.

Among the compounds of the general formula (4) having an anthracene ring structure, the compounds of the general formula (4a) or the general formula (4b) having an anthracene ring structure are far preferably used.

The arylamine compounds of the general formula (1c-a) or (1c-b), the arylamine compounds of the general formula (2) having a structure in which four triphenylamine structures are joined within a molecule via a single bond or a divalent group that does not contain a heteroatom, or the arylamine compounds of the general formula (3) having a structure in which two triphenylamine structures are joined within a molecule via a single bond or a divalent group that does not contain a heteroatom, for use in the organic EL device of the present invention, can be used as a constitutive material of a hole injection layer or a hole transport layer of an organic EL device.

The arylamine compounds of the general formula (1c-a) or (1c-b), the arylamine compounds of the general formula (2) having a structure in which four triphenylamine structures are joined within a molecule via a single bond or a divalent group that does not contain a heteroatom, or the arylamine compounds of the general formula (3) having a structure in which two triphenylamine structures are joined within a molecule via a single bond or a divalent group that does not contain a heteroatom, have high hole mobility and are therefore preferred compounds as material of a hole injection layer or a hole transport layer.

The compounds of the general formula (4) having an anthracene ring structure, for use in the organic EL device of the present invention, can be used as a constitutive material of an electron transport layer of an organic EL device.

The compounds of the general formula (4) having an anthracene ring structure excel in electron injection and transport abilities and are therefore preferred compounds as material of an electron transport layer.

The organic EL device of the present invention combines materials for an organic EL device exceling in hole and electron injection/transport performances, stability as a thin film and durability, taking carrier balance into consideration. Therefore, compared with the conventional organic EL devices, hole transport efficiency to the light emitting layer from the hole transport layer is improved (and electron transport efficiency to the light emitting layer from the electron transport layer is also improved in an embodiment using specific compounds having an anthracene ring structure). As a result, luminous efficiency is improved and driving voltage is decreased, and durability of the organic EL device can thereby be improved.

Thus, an organic EL device having high efficiency, low driving voltage and a long lifetime can be attained in the present invention.

The organic EL device of the present invention can achieve an organic EL device having high efficiency, low driving voltage and a long lifetime as a result of attaining efficient hole injection/transport into a light emitting layer by selecting a combination of two specific kinds of arylamine compounds which excel in hole and electron injection/transport performances, stability as a thin film and durability and can effectively exhibit hole injection/transport roles. An organic EL device having high efficiency, low driving voltage and a long lifetime can be achieved by selecting two specific kinds of arylamine compounds and specific compounds having an anthracene ring structure, and combining those compounds so as to achieve good carrier balance. The organic EL device of the present invention can improve luminous efficiency, driving voltage and durability of the conventional organic EL devices.

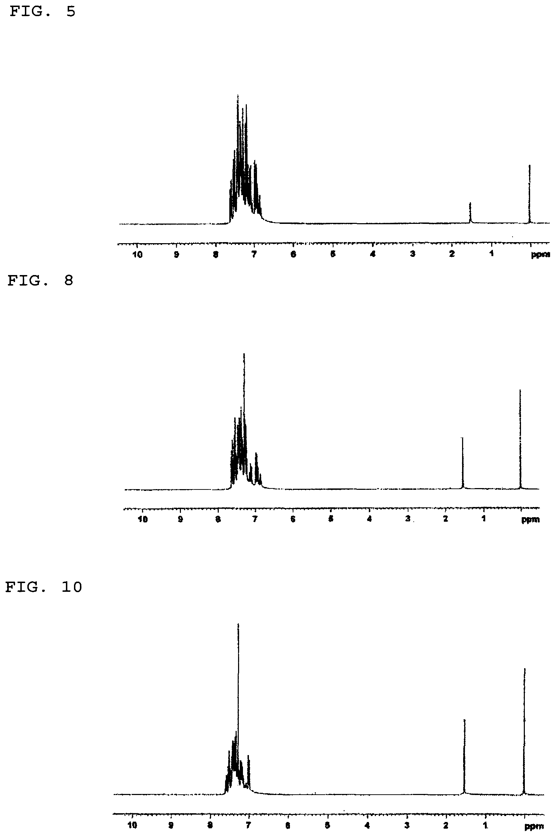

FIG. 5 is a ¹H-NMR chart of the compound (1-24) of Example 5 of the present invention.

FIG. 8 is a ¹H-NMR chart of the compound (1-27) of Example 8 of the present invention.

FIG. 10 is a ¹H-NMR chart of the compound (1-30) of Example 10 of the present invention.

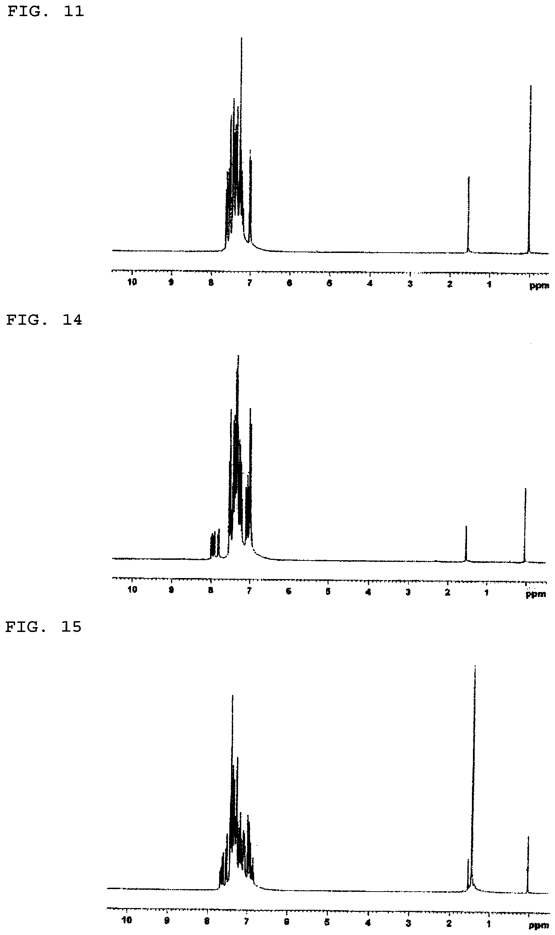

FIG. 11 is a ¹H-NMR chart of the compound (1-31) of Example 11 of the present invention.

FIG. 14 is a ¹H-NMR chart of the compound (1-36) of Example 14 of the present invention.

FIG. 15 is a ¹H-NMR chart of the compound (1-37) of Example 15 of the present invention.

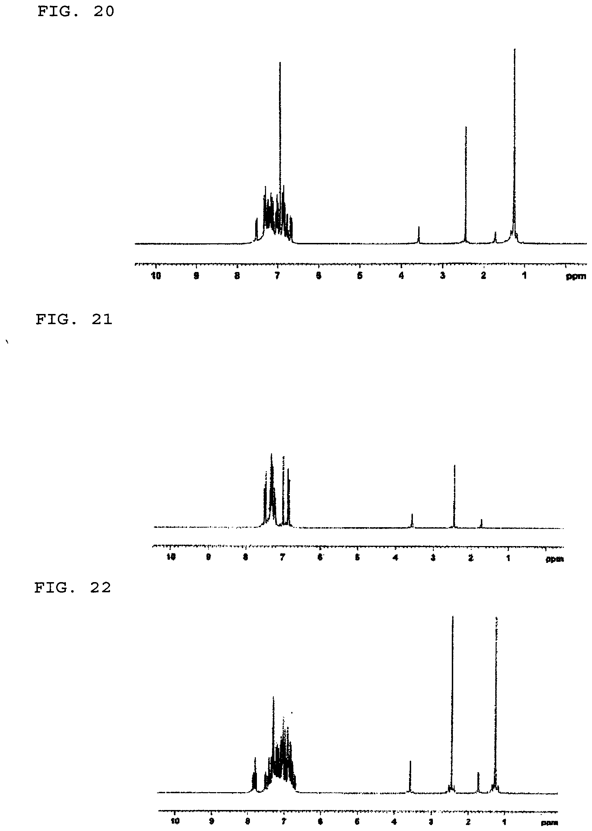

FIG. 20 is a ¹H-NMR chart of the compound (1-49) of Example 20 of the present invention.

FIG. 21 is a ¹H-NMR chart of the compound (1-50) of Example 21 of the present invention.

FIG. 22 is a ¹H-NMR chart of the compound (1-55) of Example 22 of the present invention.

FIG. 23 is a diagram illustrating the configuration of the organic EL devices of Examples 29, 31 to 33, 36, 37, 40 and Comparative Examples 1 and 2.

The following presents specific examples of preferred compounds among the arylamine compounds of the general formula (1c-a) or (1c-b) preferably used in the organic EL device of the present invention. The present invention, however, is not restricted to these compounds.

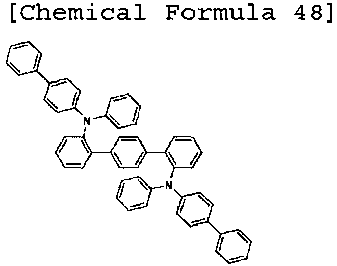

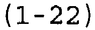

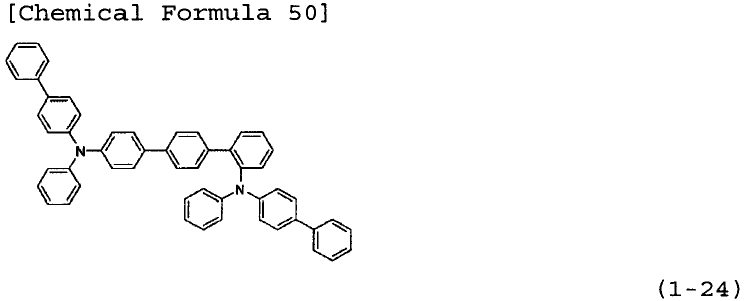

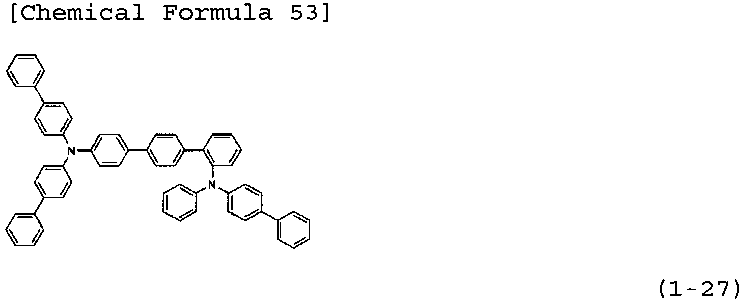

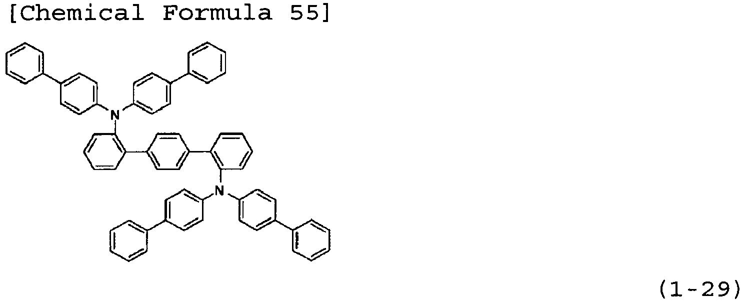

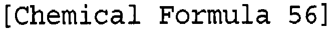

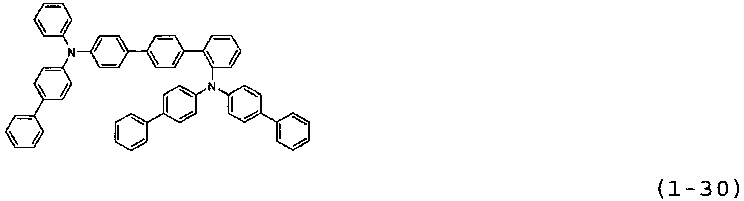

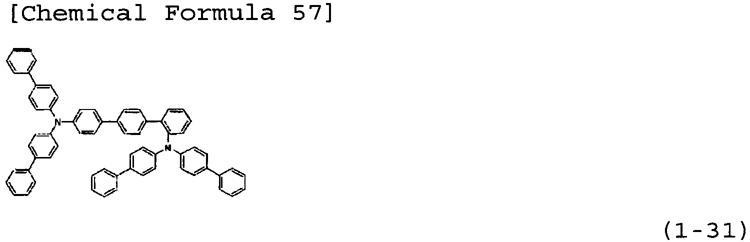

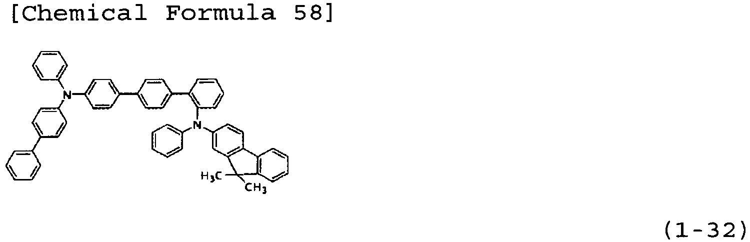

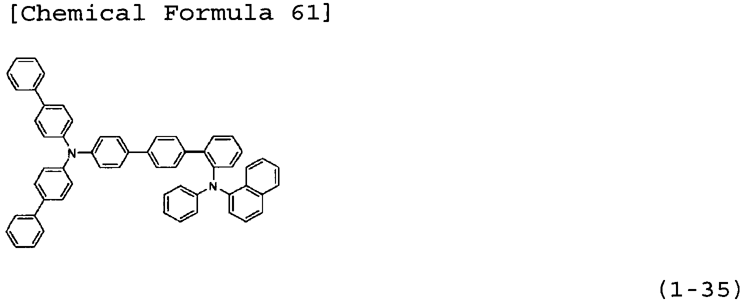

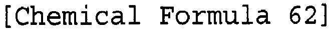

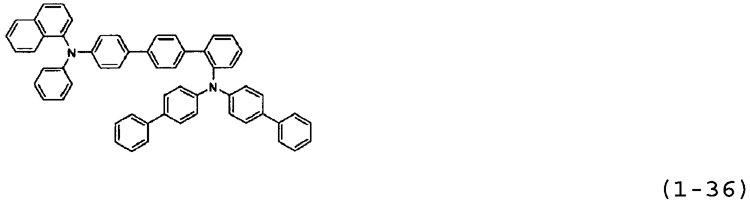

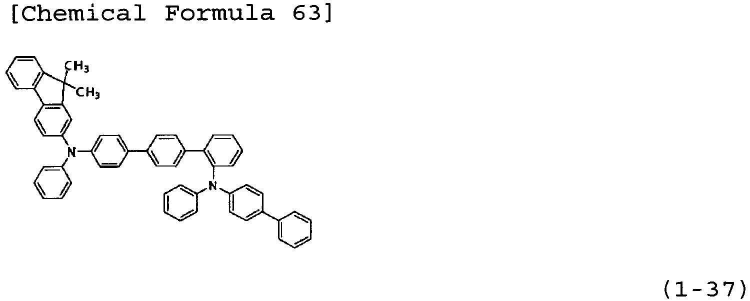

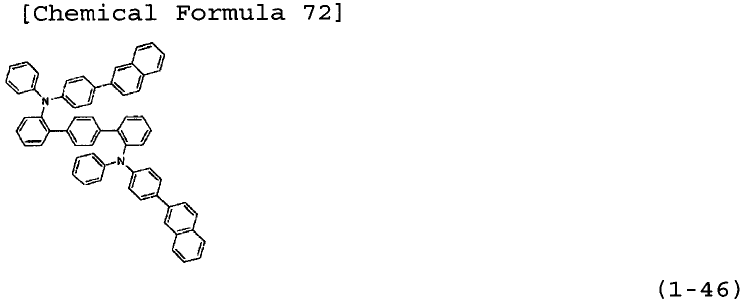

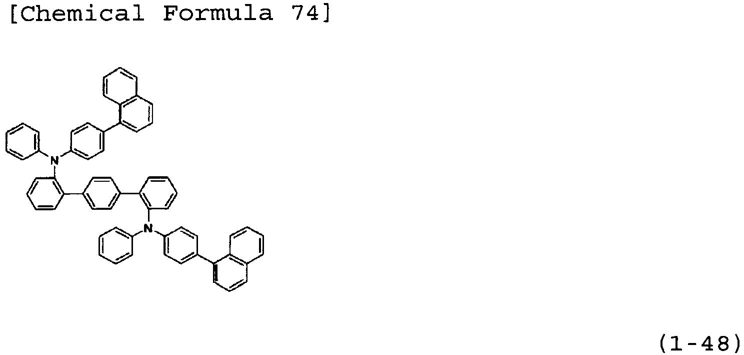

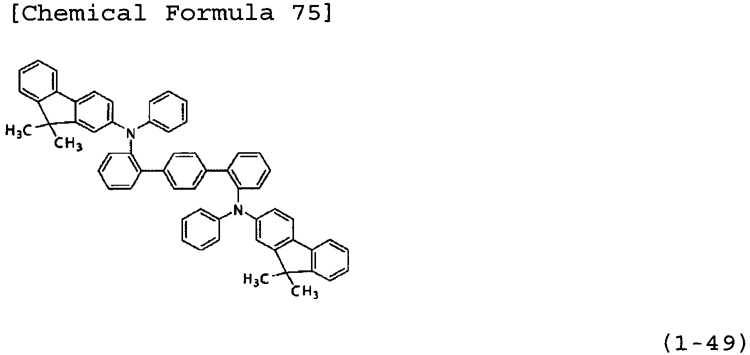

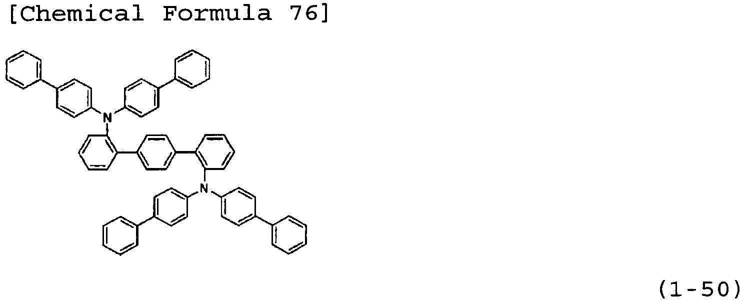

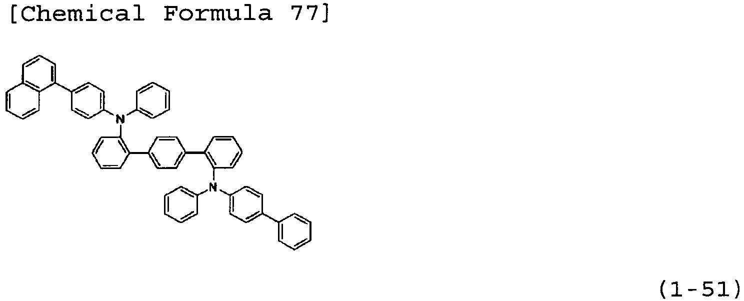

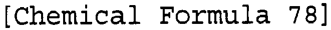

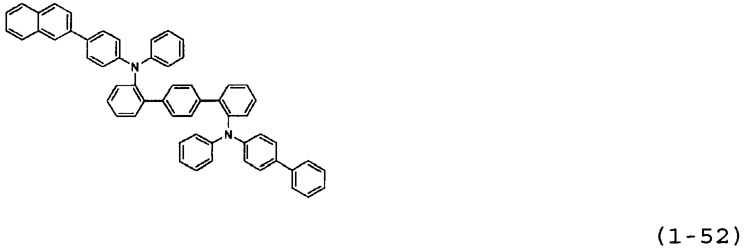

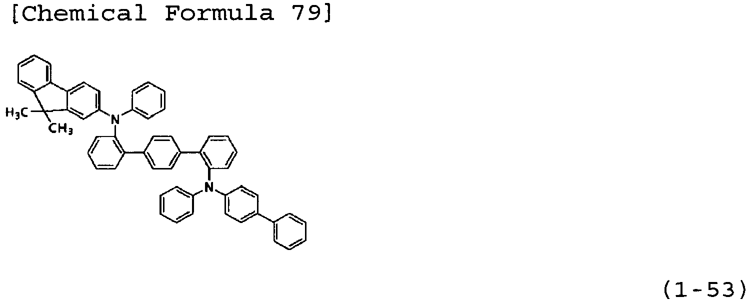

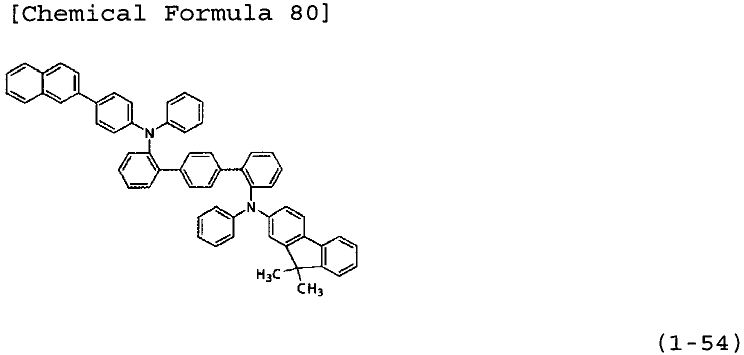

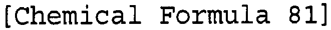

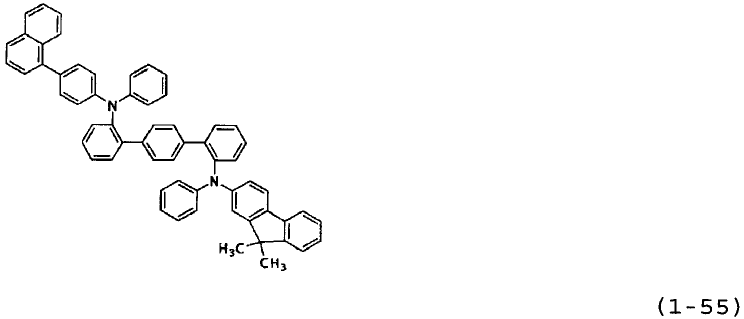

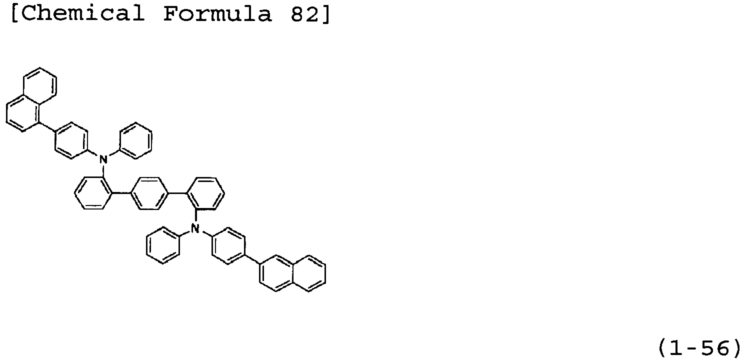

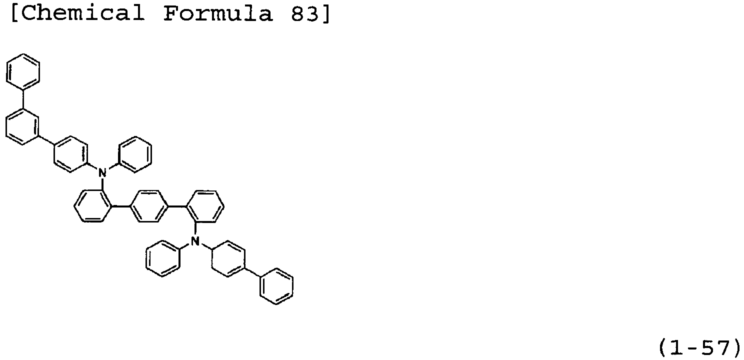

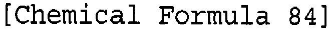

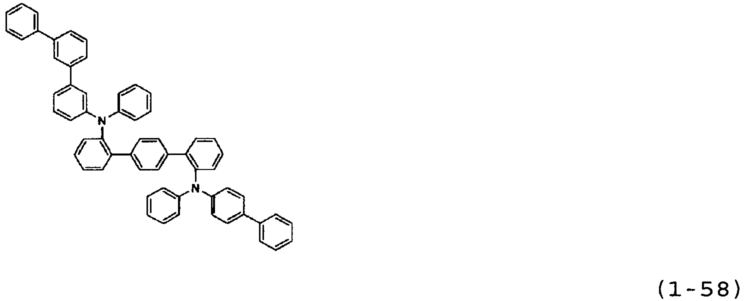

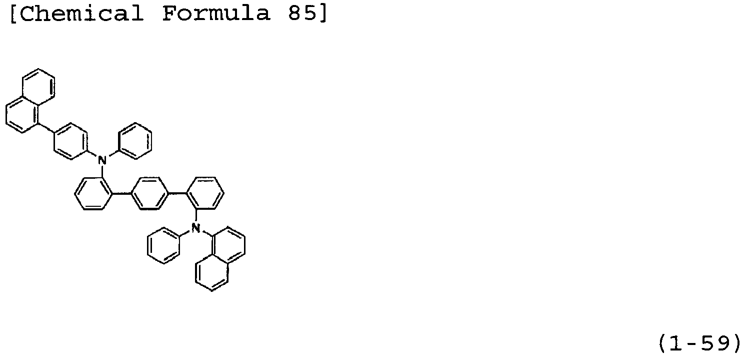

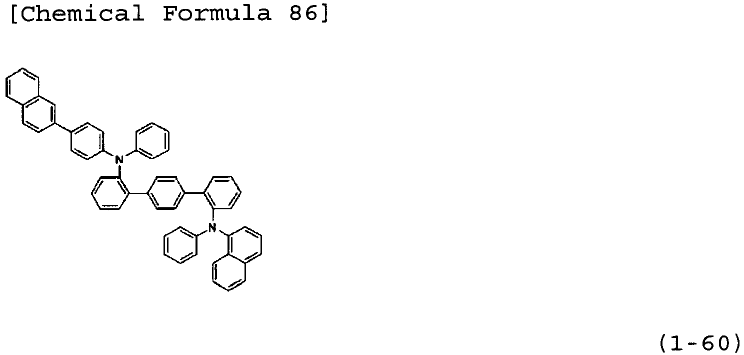

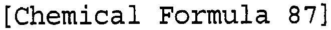

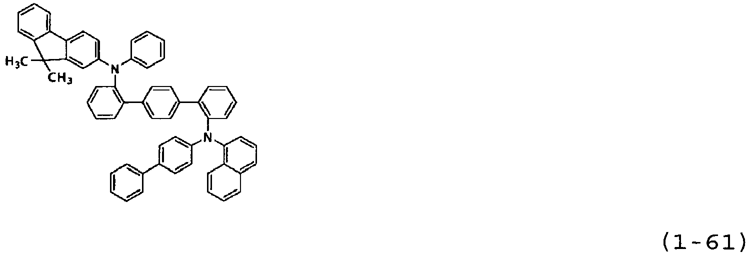

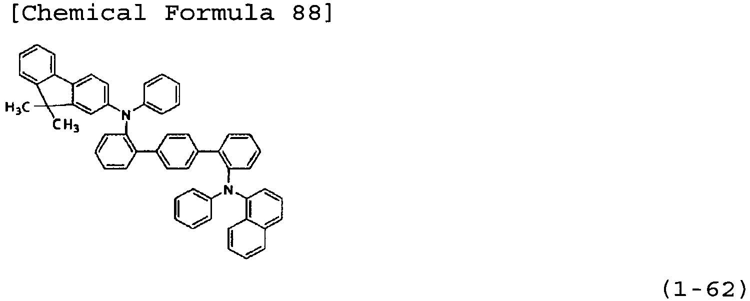

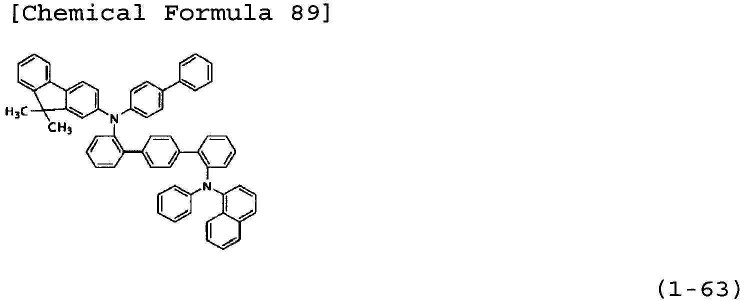

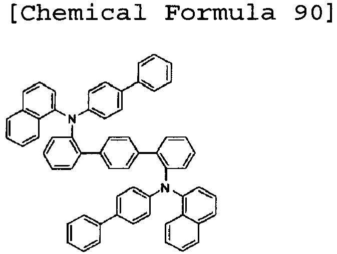

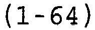

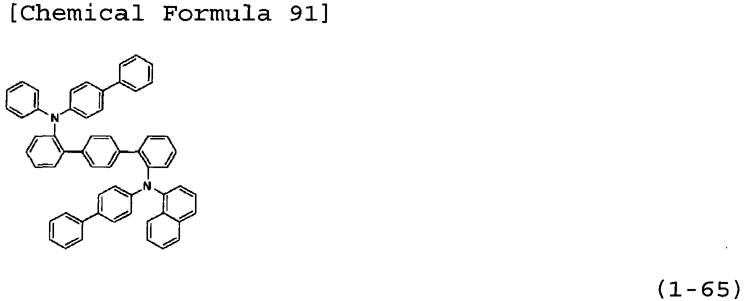

Among the arylamine compounds preferably used in the organic EL device of the present invention and having a structure in which three to six triphenylamine structures are joined within a molecule via a single bond or a divalent group that does not contain a heteroatom, the far preferably used compounds are the arylamine compounds of the general formula (2) having a structure in which four triphenylamine structures are joined within a molecule via a single bond or a divalent group that does not contain a heteroatom. Following presents specific examples of preferred compounds among the arylamine compounds of the general formula (2) having a structure in which four triphenylamine structures are joined within a molecule via a single bond or a divalent group that does not contain a heteroatom. The present invention, however, is not restricted to these compounds.

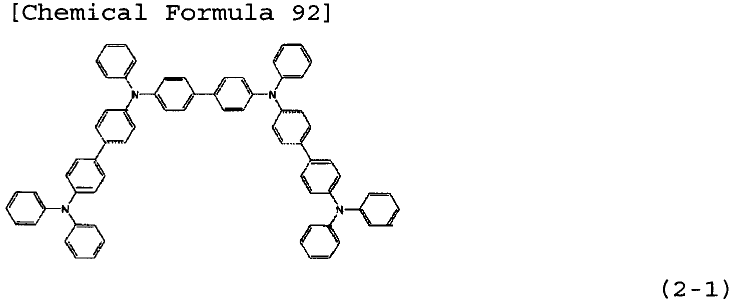

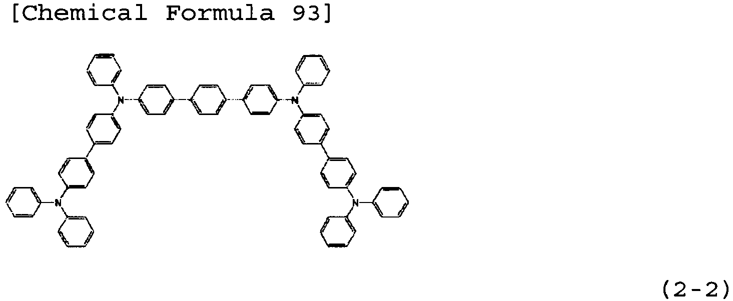

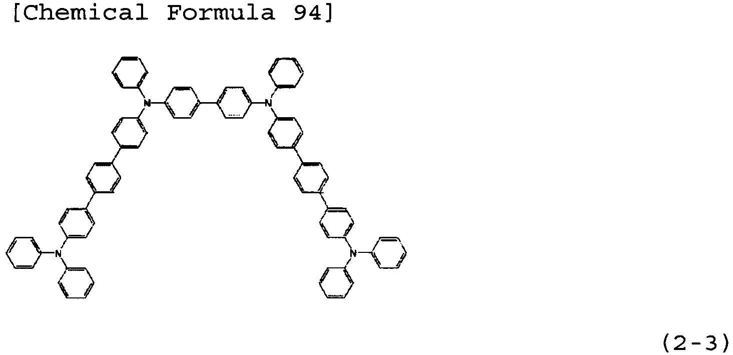

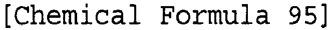

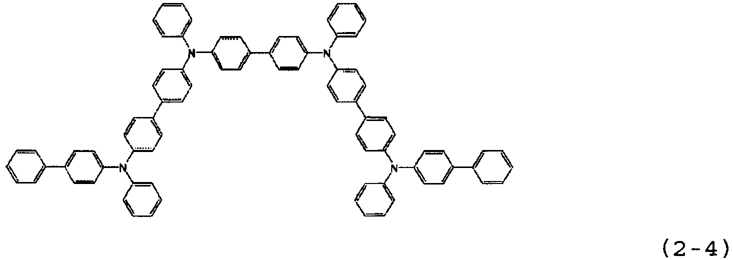

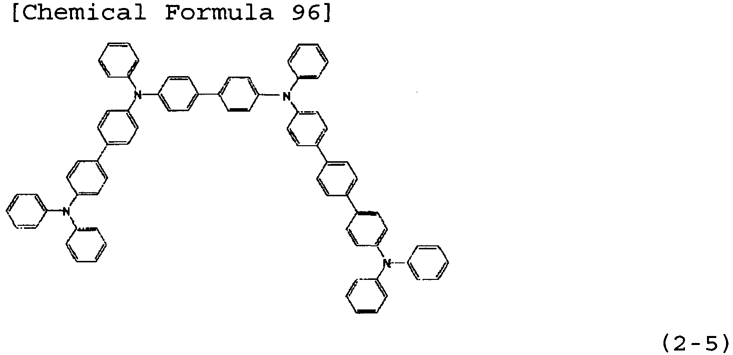

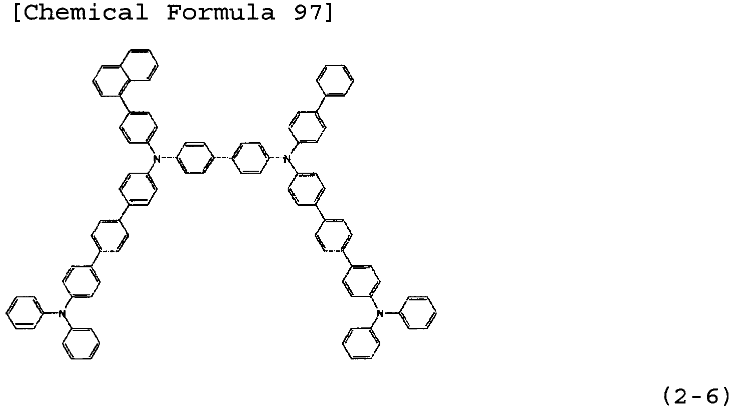

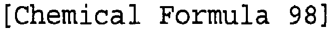

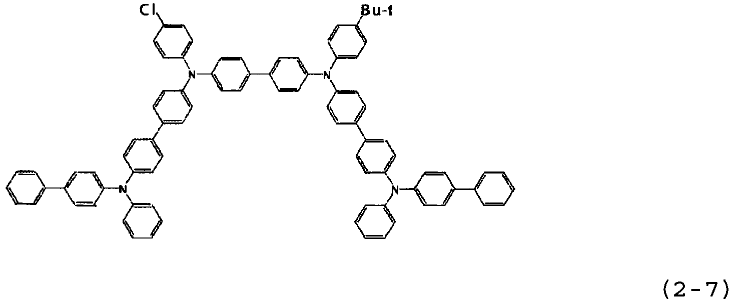

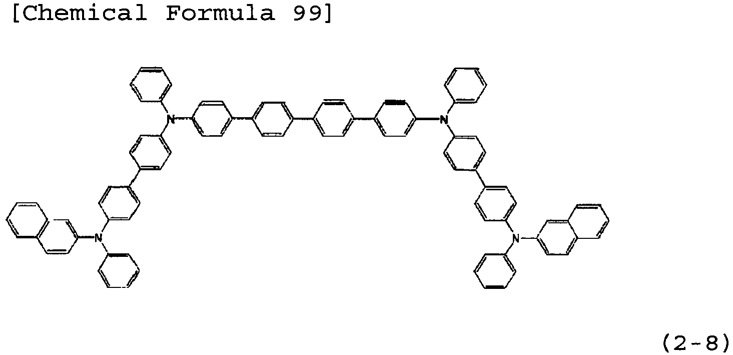

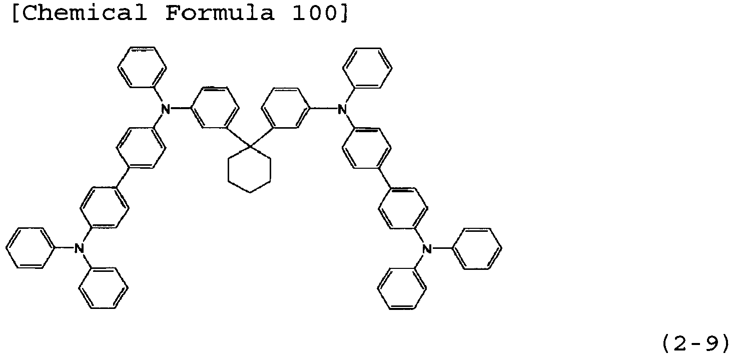

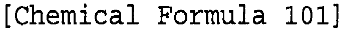

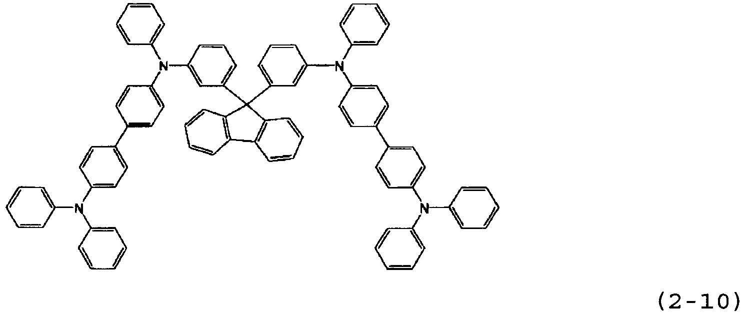

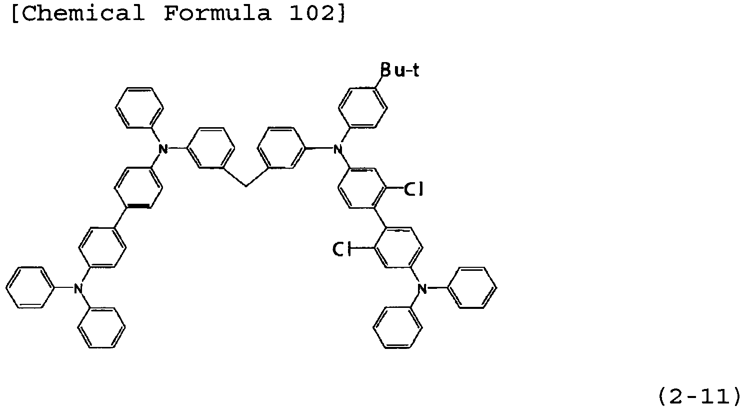

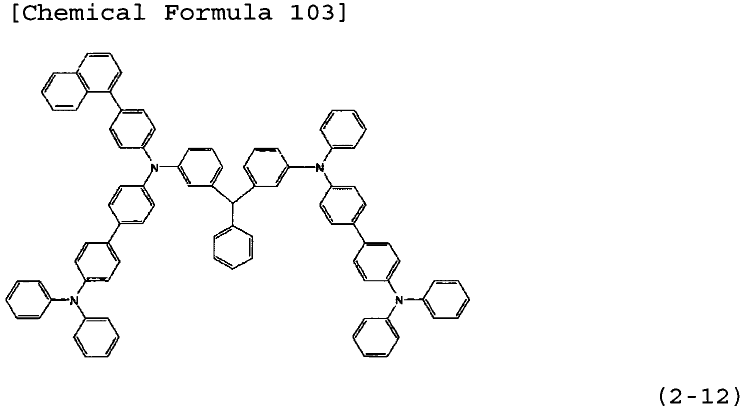

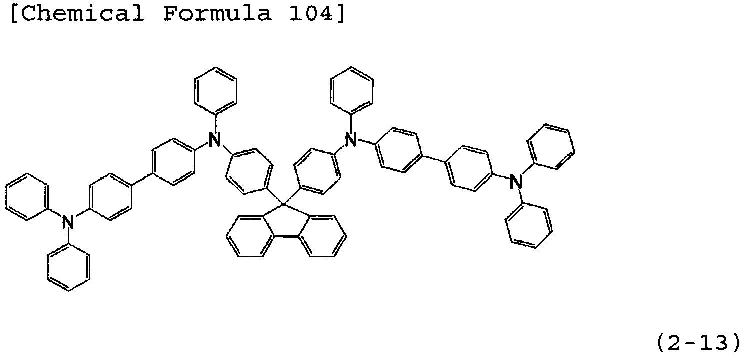

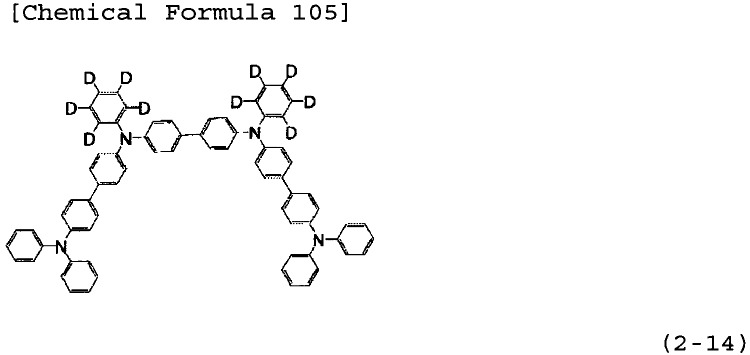

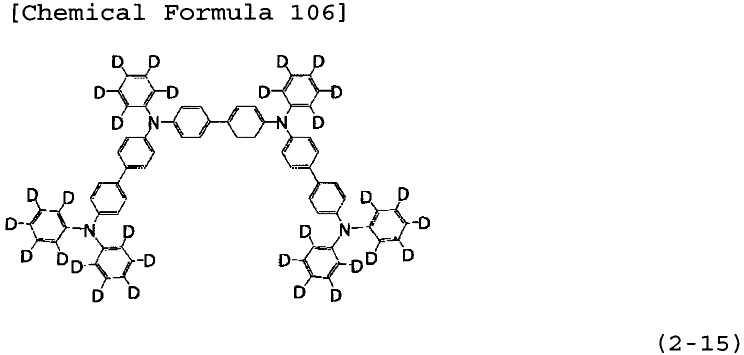

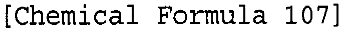

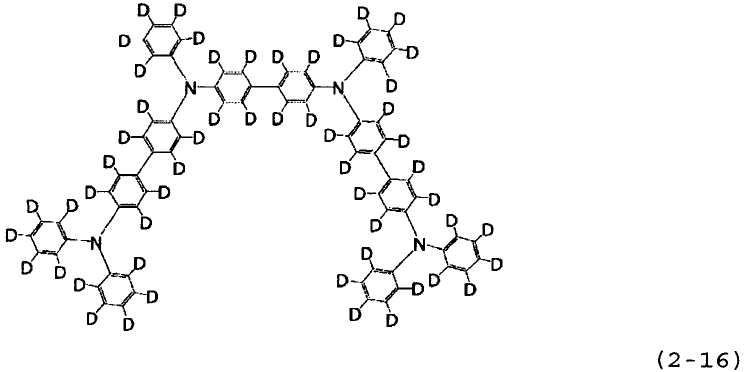

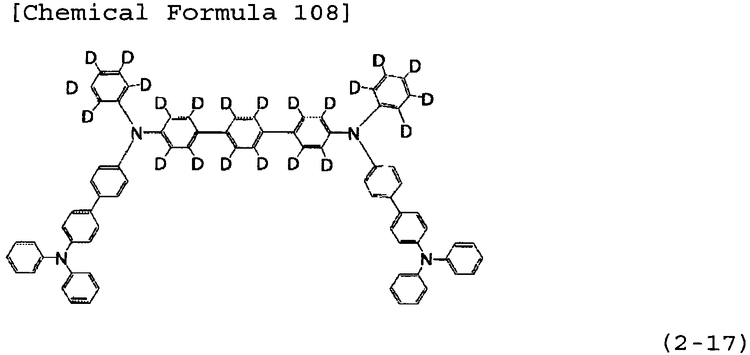

Among the arylamine compounds preferably used in the organic EL device of the present invention and having a structure in which three to six triphenylamine structures are joined within a molecule via a single bond or a divalent group that does not contain a heteroatom, the following presents specific examples of preferred compounds besides the arylamine compounds of the general formula (2) having a structure in which four triphenylamine structures are joined within a molecule via a single bond or a divalent group that does not contain a heteroatom. The present invention, however, is not restricted to these compounds.

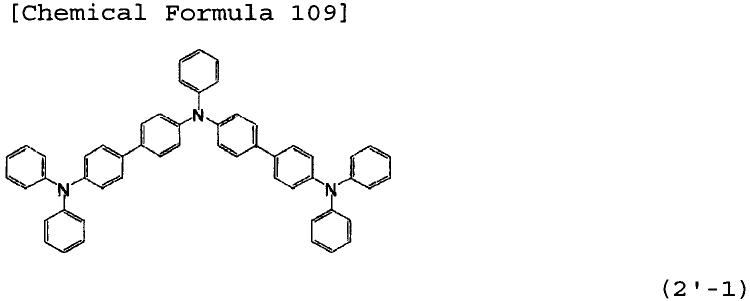

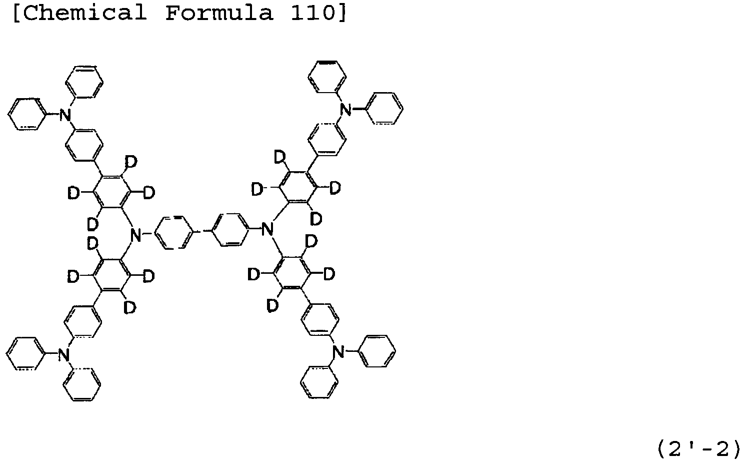

The following presents specific examples of preferred compounds among the arylamine compounds of the general formula (3) preferably used in the organic EL device of the present invention and having a structure in which two triphenylamine structures are joined within a molecule via a single bond or a divalent group that does not contain a heteroatom. The present invention, however, is not restricted to these compounds.

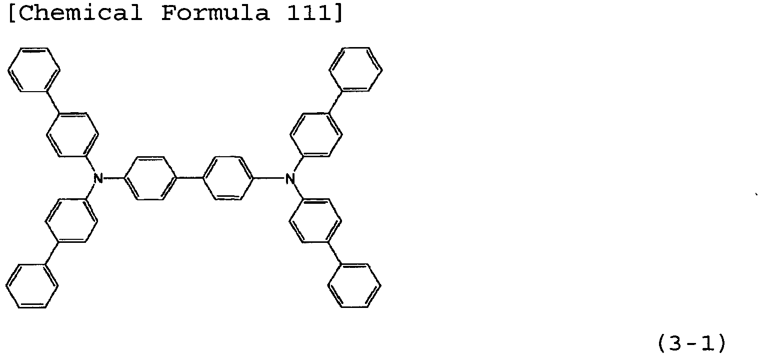

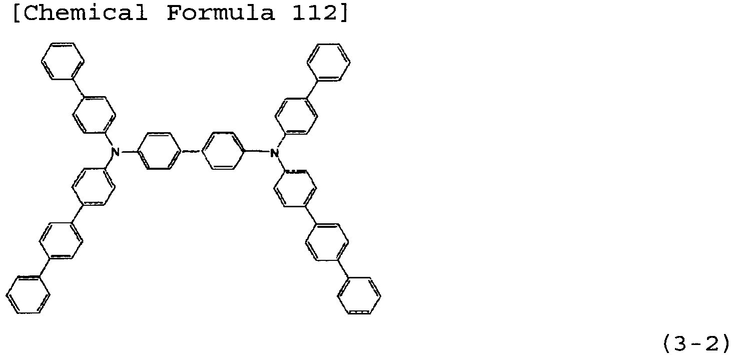

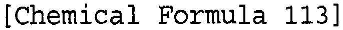

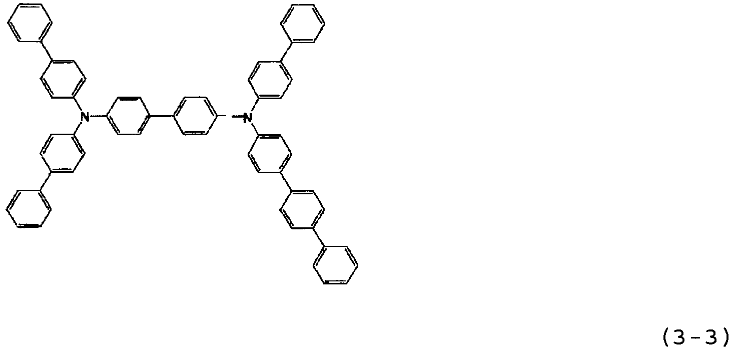

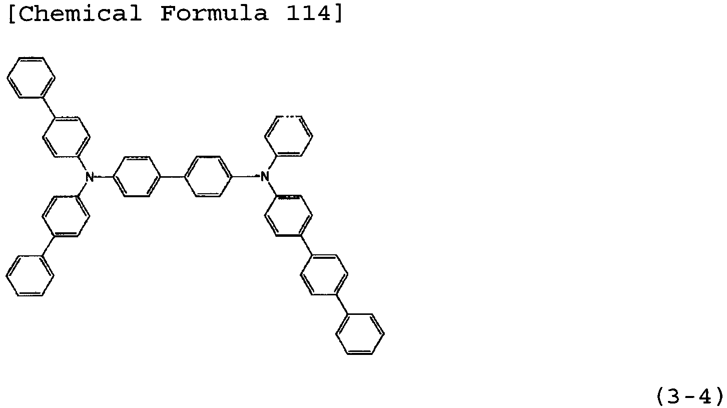

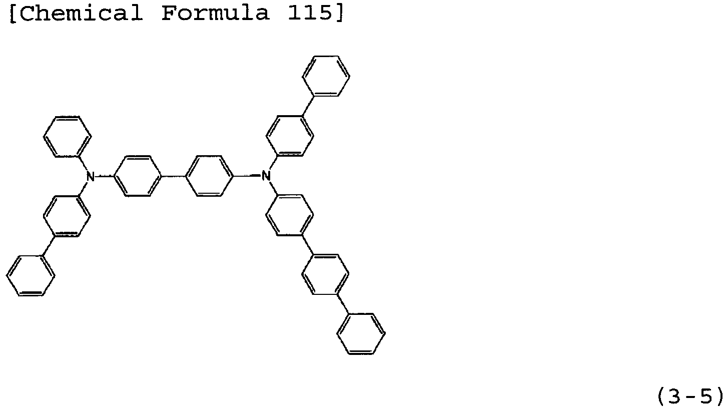

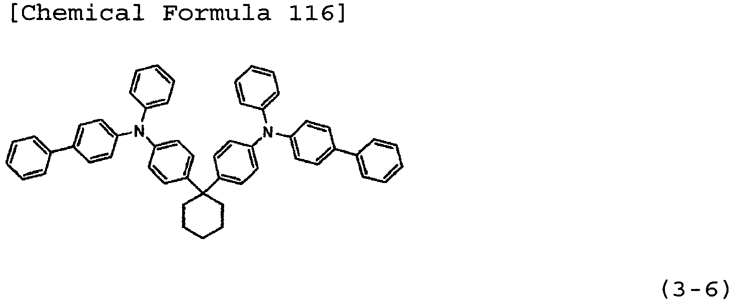

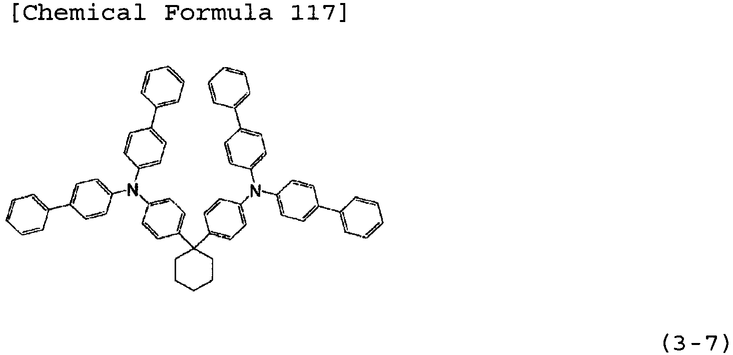

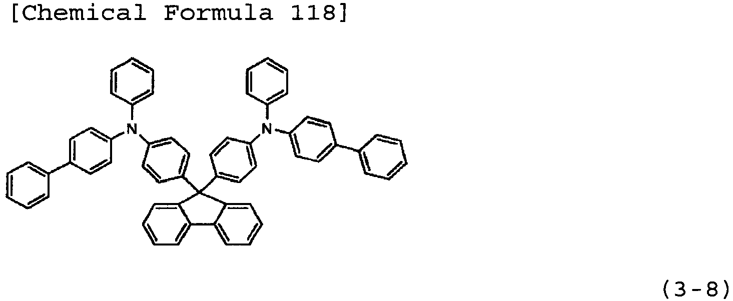

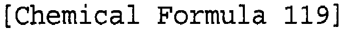

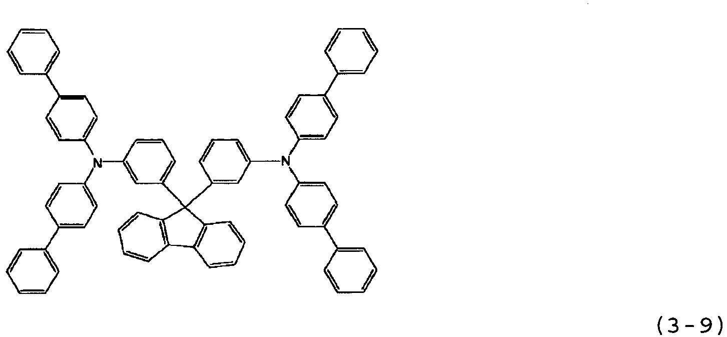

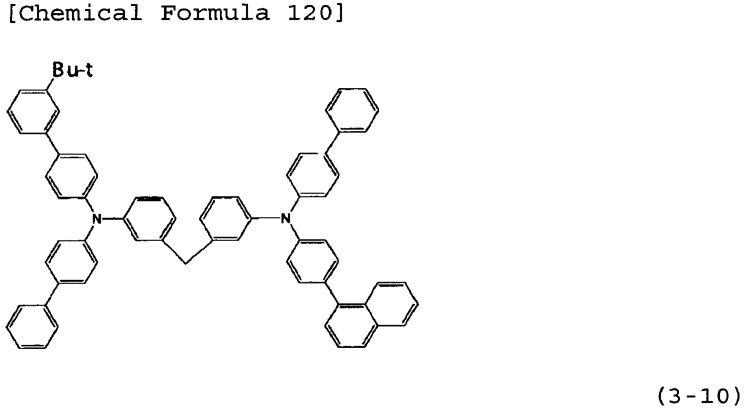

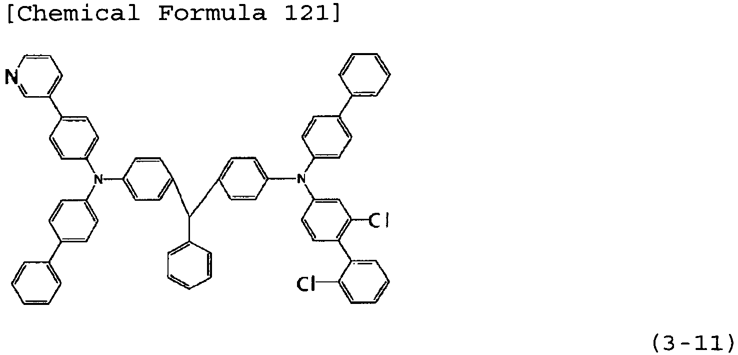

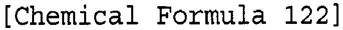

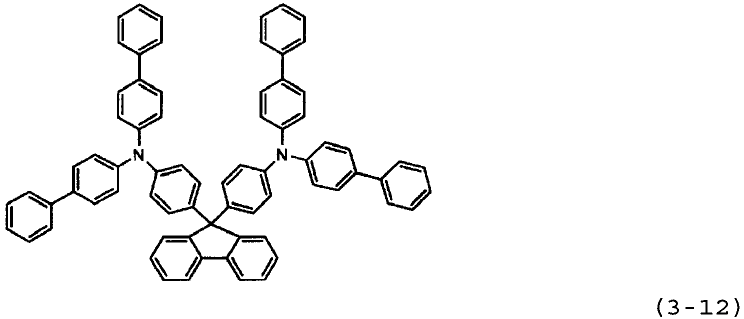

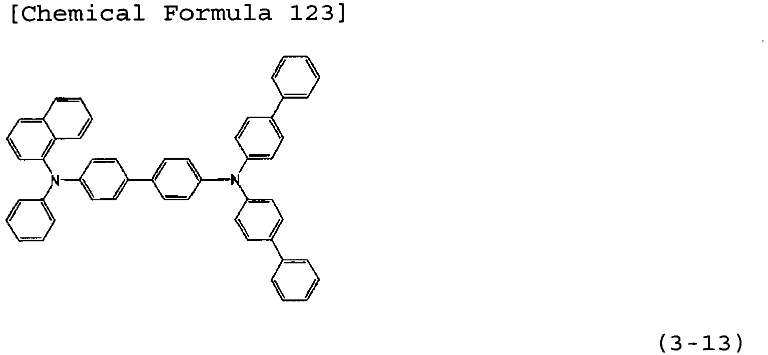

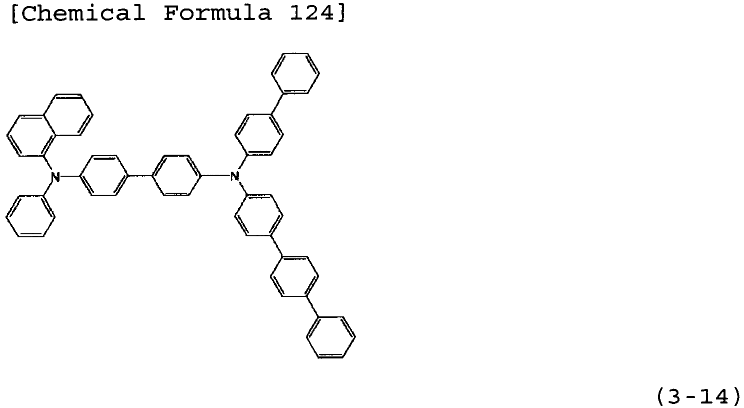

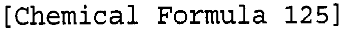

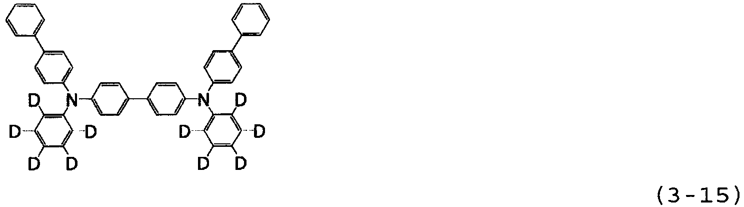

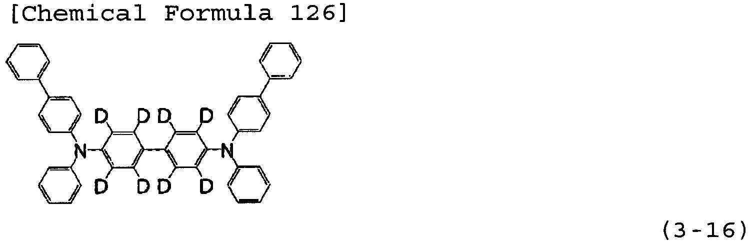

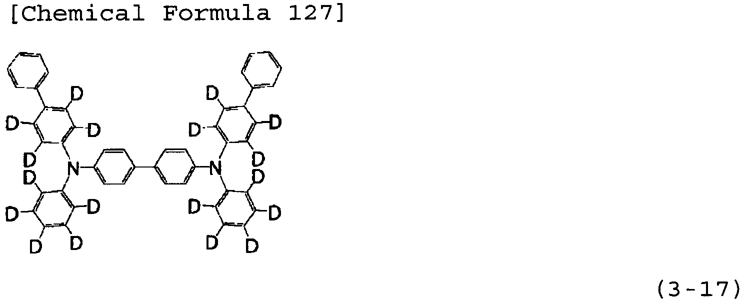

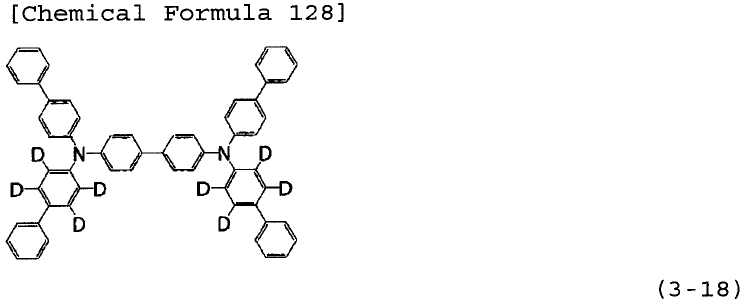

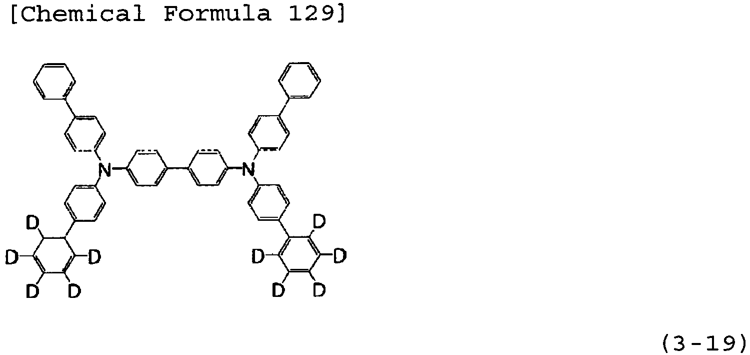

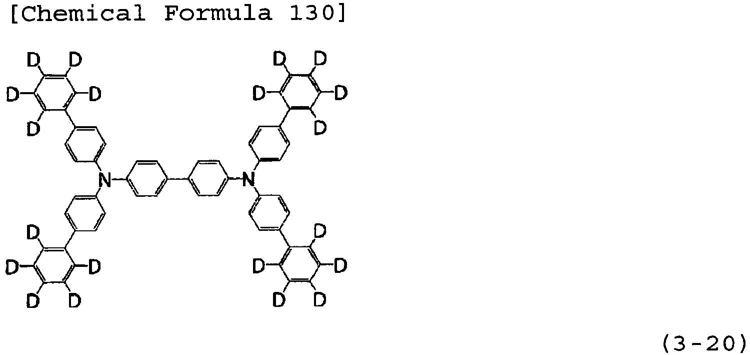

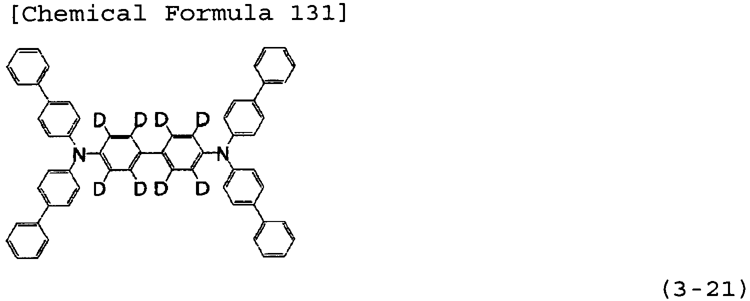

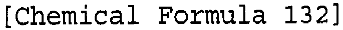

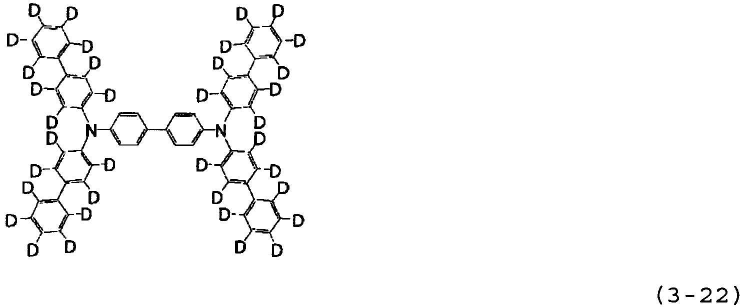

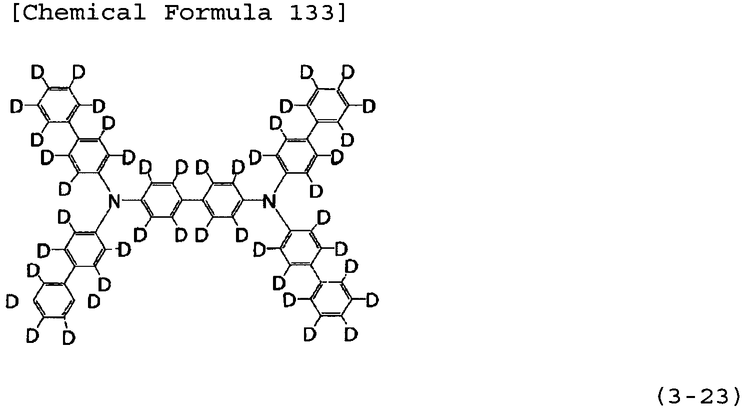

Among the arylamine compounds used in the organic EL device of the present invention and having two triphenylamine structures within a molecule, the following presents specific examples of preferred compounds besides the arylamine compounds of the general formula (3) having a structure in which two triphenylamine structures are joined within a molecule via a single bond or a divalent group that does not contain a heteroatom. The present invention, however, is not restricted to these compounds.

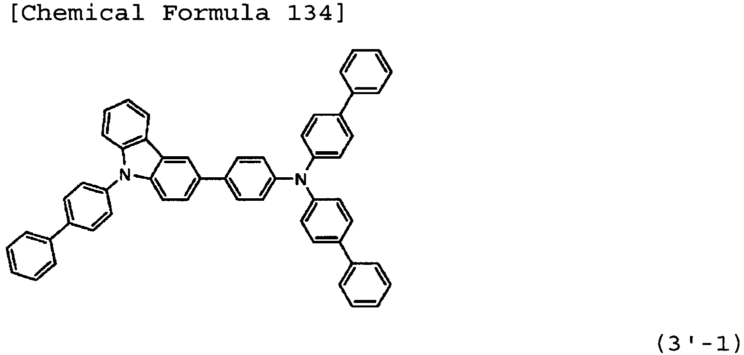

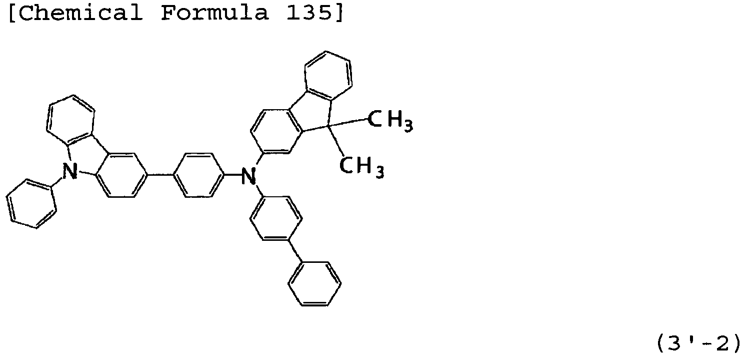

The arylamine compounds having a structure in which three to six triphenylamine structures are joined within a molecule via a single bond or a divalent group that does not contain a heteroatom, or the arylamine compounds having a structure in which two triphenylamine structures are joined within a molecule via a single bond or a divalent group that does not contain a heteroatom, can be synthesized by a known method (refer to Patent Documents 6 to 8, for example).

The following presents specific examples of preferred compounds among the compounds of the general formula (4a) preferably used in the organic EL device of the present invention and having an anthracene ring structure. The present invention, however, is not restricted to these compounds.

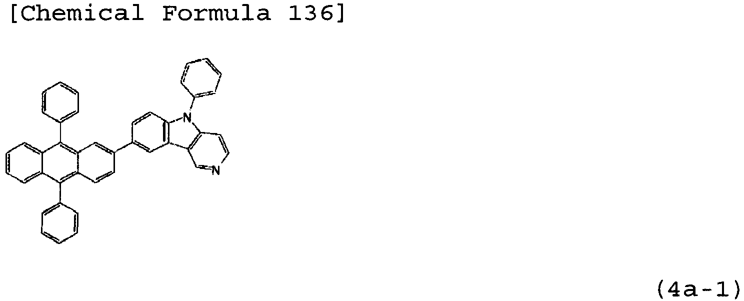

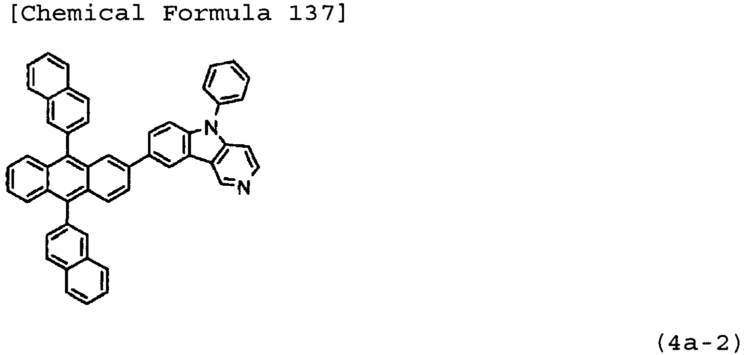

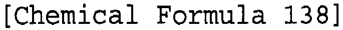

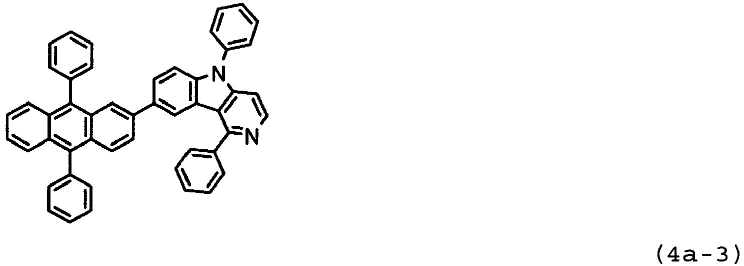

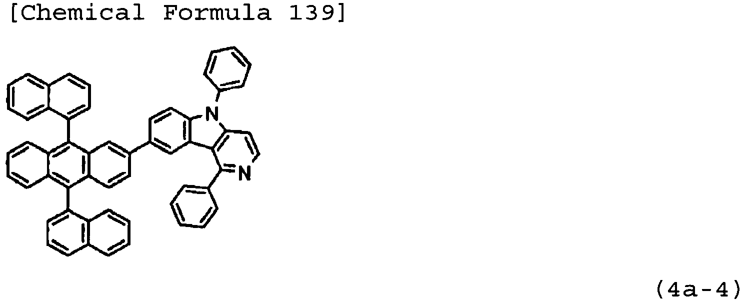

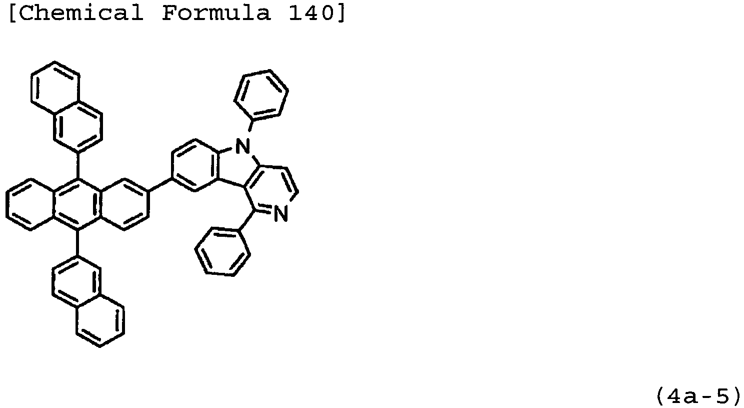

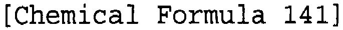

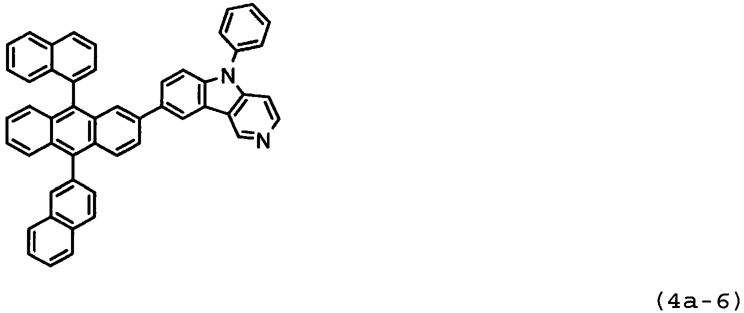

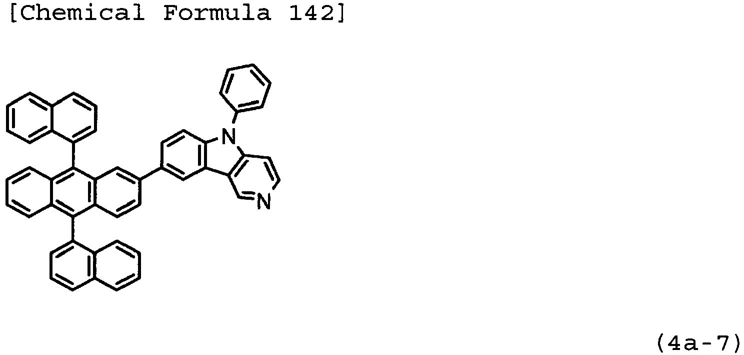

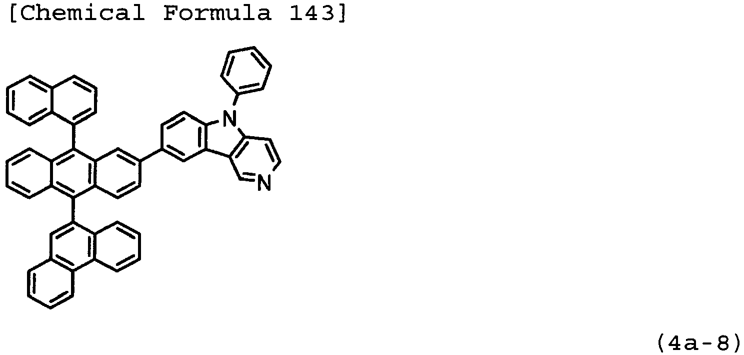

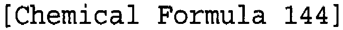

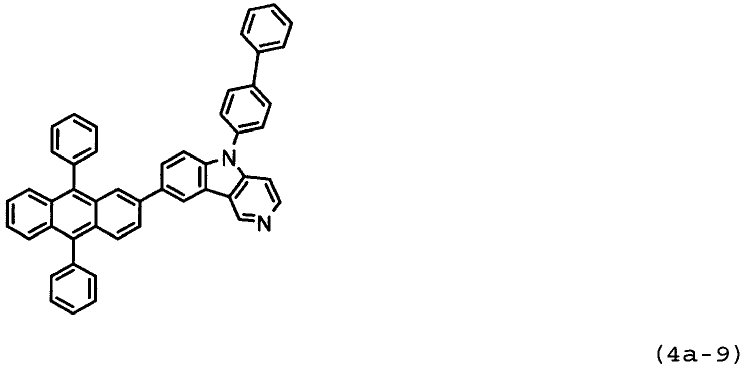

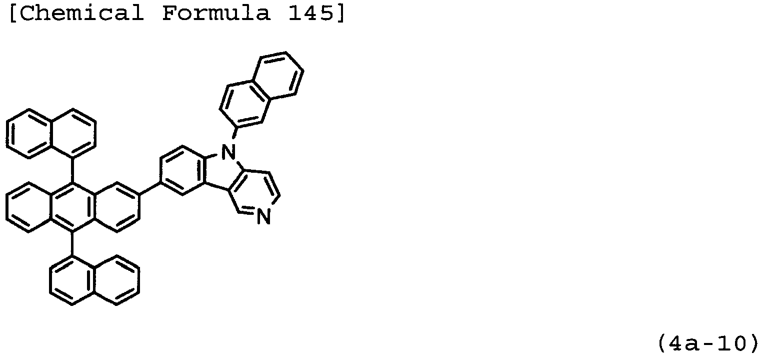

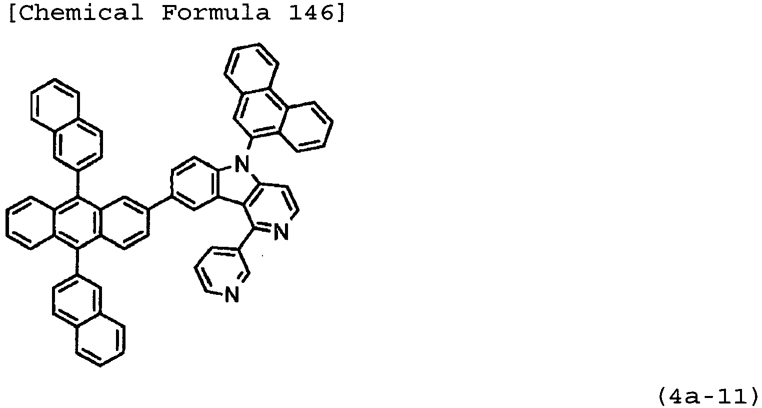

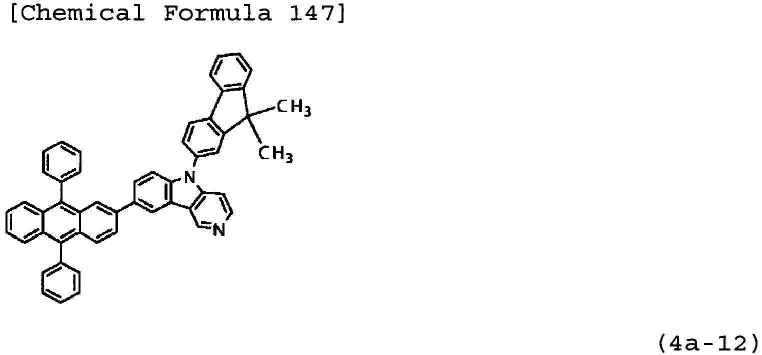

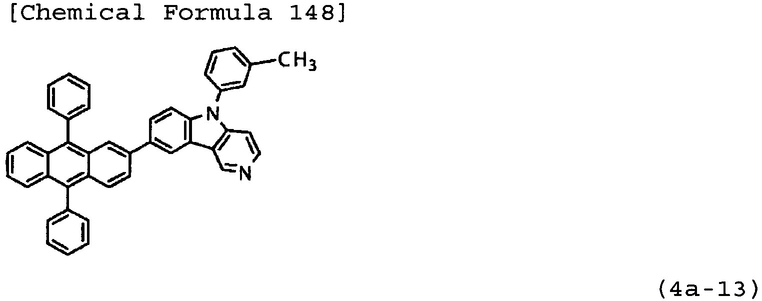

The following presents specific examples of preferred compounds among the compounds of the general formula (4b) preferably used in the organic EL device of the present invention and having an anthracene ring structure. The present invention, however, is not restricted to these compounds.

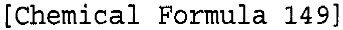

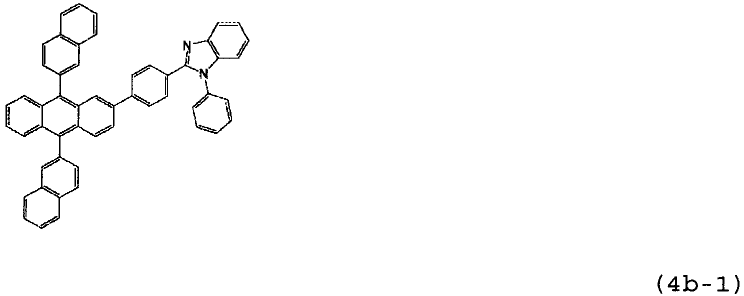

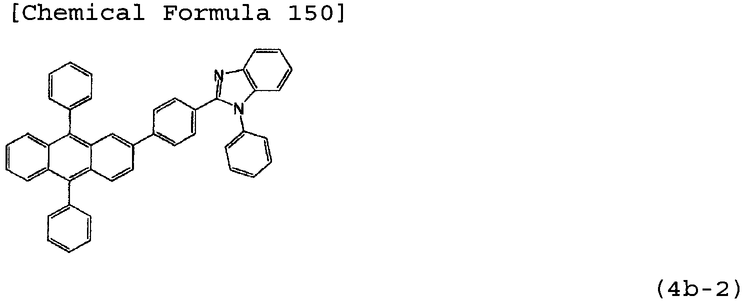

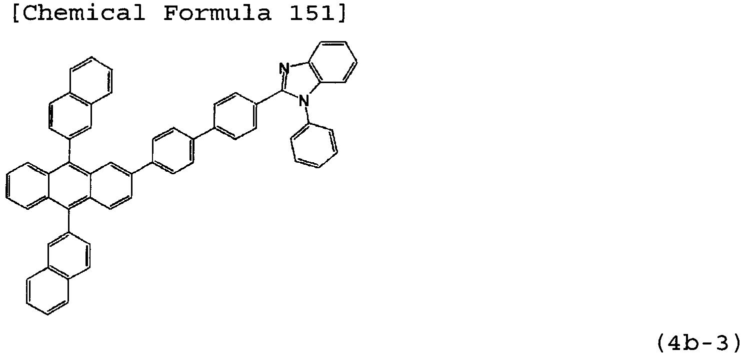

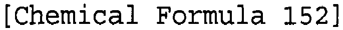

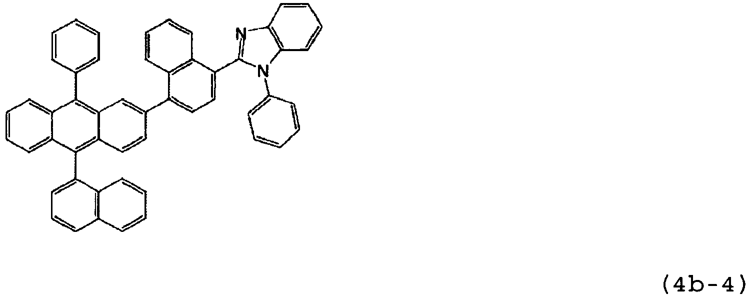

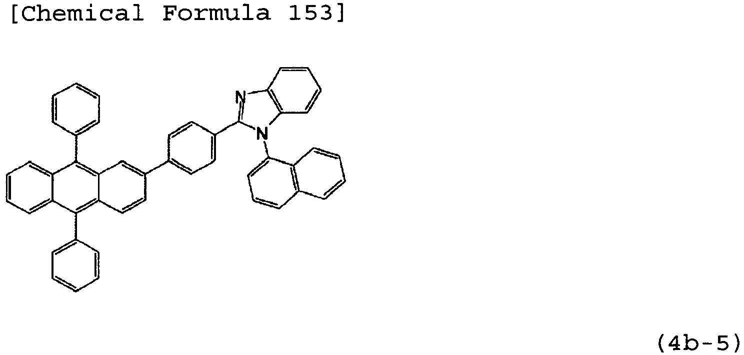

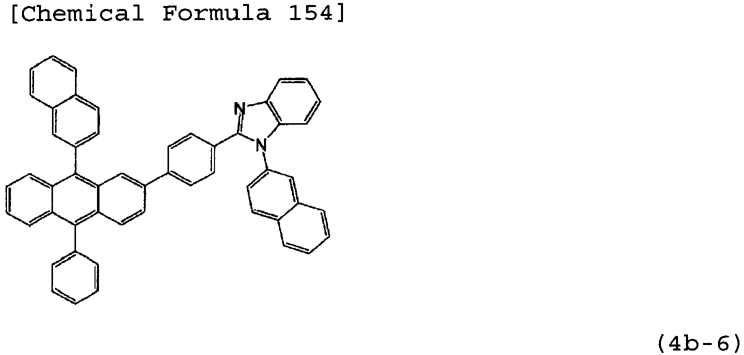

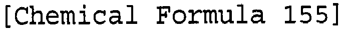

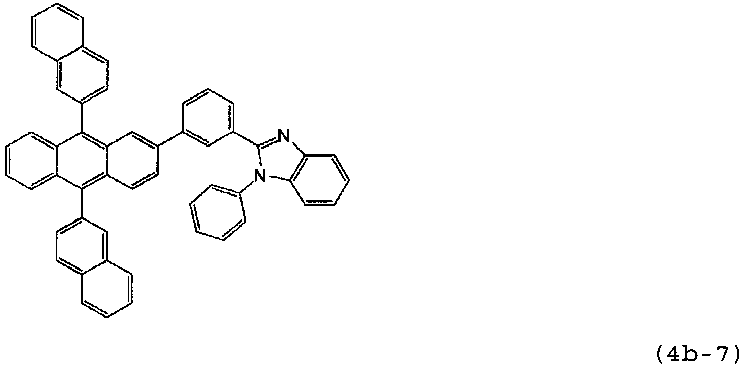

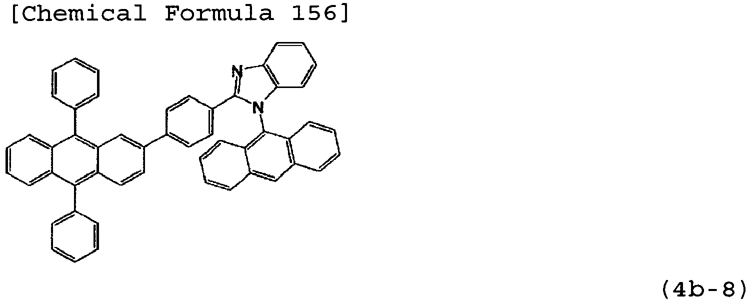

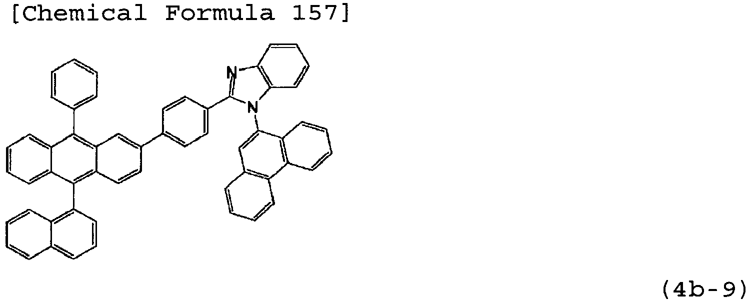

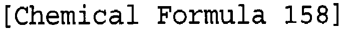

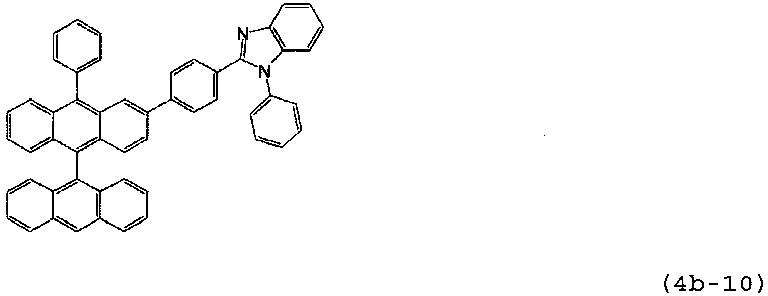

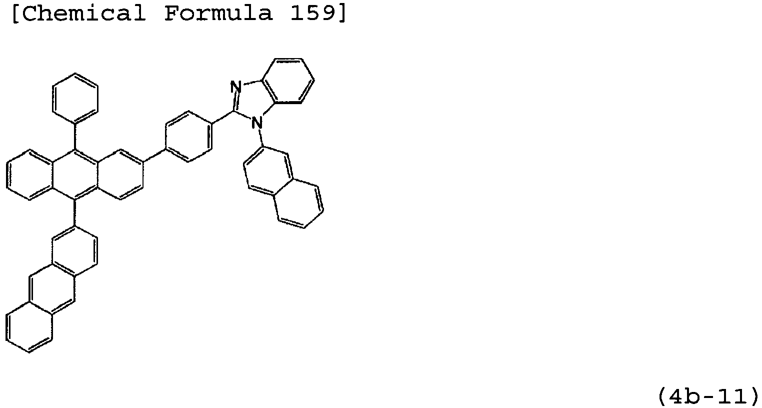

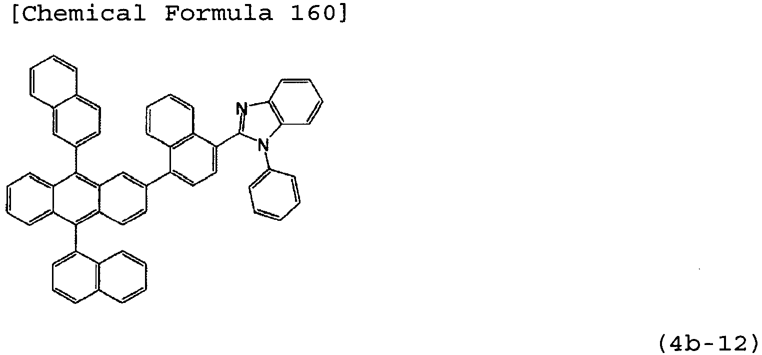

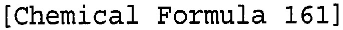

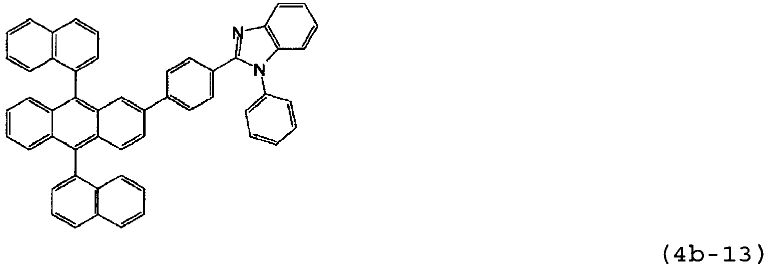

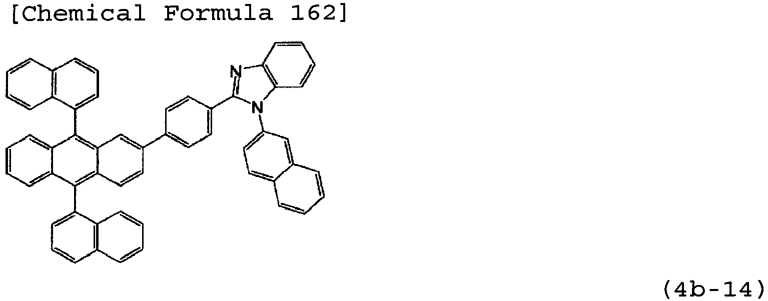

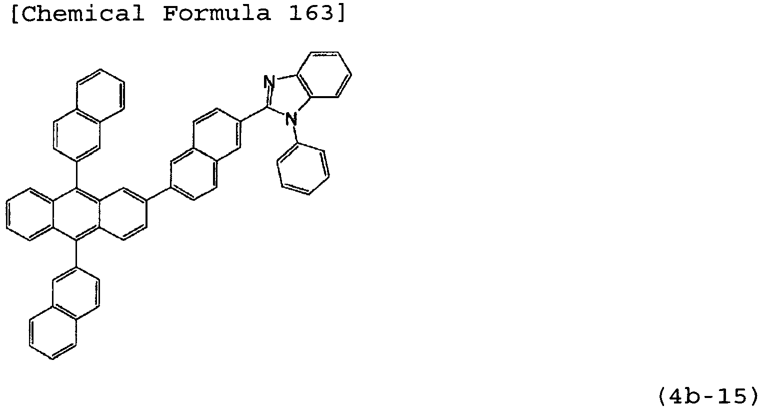

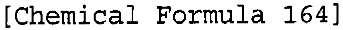

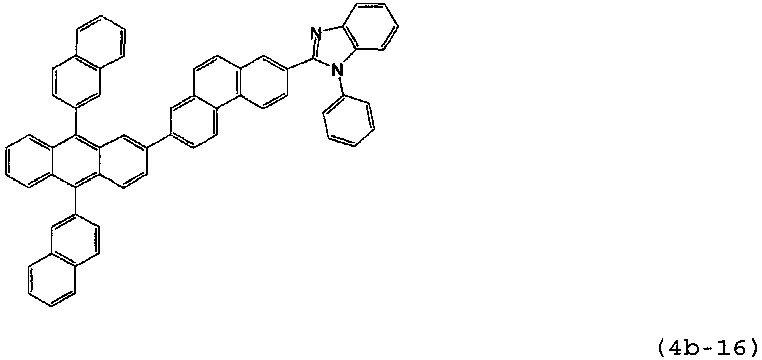

The compounds described above having an anthracene ring structure can be synthesized by a known method (refer to Patent Documents 9 to 10, for example).

The arylamine compounds of the general formula (1c-a) or (1c-b) were purified by methods such as column chromatography, adsorption using, for example, a silica gel, activated carbon, or activated clay, and recrystallization or crystallization using a solvent. The compounds were identified by an NMR analysis. A melting point, a glass transition point (Tg), and a work function were measured as material property values. The melting point can be used as an index of vapor deposition, the glass transition point (Tg) as an index of stability in a thin-film state, and the work function as an index of hole transportability.

The melting point and the glass transition point (Tg) were measured by a high-sensitive differential scanning calorimeter (DSC3100S produced by Bruker AXS) using powder.

For the measurement of the work function, a 100 nm-thick thin film was fabricated on an ITO substrate, and an ionization potential measuring device (PYS-202 produced by Sumitomo Heavy Industries, Ltd.) was used.

The organic EL device of the present invention may have a structure including an anode, a hole injection layer, a first hole transport layer, a second hole transport layer, a light emitting layer, an electron transport layer, and a cathode successively formed on a substrate, optionally with an electron blocking layer between the second hole transport layer and the light emitting layer, a hole blocking layer between the light emitting layer and the electron transport layer, and an electron injection layer between the electron transport layer and the cathode. Some of the organic layers in the multilayer structure may be omitted, or may serve more than one function. For example, a single organic layer may serve as the hole injection layer and the first hole transport layer, or as the electron injection layer and the electron transport layer.

Electrode materials with high work functions such as ITO and gold are used as the anode of the organic EL device of the present invention. The hole injection layer of the organic EL device of the present invention may be made of, for example, material such as starburst-type triphenylamine derivatives and various triphenylamine tetramers; porphyrin compounds as represented by copper phthalocyanine; accepting heterocyclic compounds such as hexacyano azatriphenylene; and coating-type polymer materials, in addition to the arylamine compounds of the general formula (1c-a) or (1c-b), the arylamine compounds of the general formula (2) having a structure in which four triphenylamine structures are joined within a molecule via a single bond or a divalent group that does not contain a heteroatom, and the arylamine compounds of the general formula (3) having a structure in which two triphenylamine structures are joined within a molecule via a single bond or a divalent group that does not contain a heteroatom. These materials may be formed into a thin film by a vapor deposition method or other known methods such as a spin coating method and an inkjet method.

Examples of material used for the first hole transport layer of the organic EL device of the present invention can be benzidine derivatives such as N,N'-diphenyl-N,N'-di(m-tolyl)benzidine (hereinafter referred to as TPD), N,N'-diphenyl-N,N'-di(a-naphthyl)benzidine (hereinafter referred to as NPD), and N,N,N',N'-tetrabiphenylylbenzidine; 1,1-bis[4-(di-4-tolylamino)phenyl]cyclohexane (TAPC); and various triphenylamine trimers and tetramers, in addition to the arylamine compounds of the general formula (2) having a structure in which four triphenylamine structures are joined within a molecule via a single bond or a divalent group that does not contain a heteroatom, and the arylamine compounds of the general formula (3) having a structure in which two triphenylamine structures are joined within a molecule via a single bond or a divalent group that does not contain a heteroatom. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. Examples of material used for the hole injection/transport layer can be coating-type polymer materials such as poly(3,4-ethylenedioxythiophene) (PEDOT)/poly(styrene sulfonate) (PSS). These materials may be formed into a thin-film by a vapor deposition method or other known methods such as a spin coating method and an inkjet method.

Further, material used for the hole injection layer or the first hole transport layer may be obtained by p-doping trisbromophenylamine hexachloroantimony or the like into the material commonly used for these layers, or may be, for example, polymer compounds each having a structure of a benzidine derivative such as TPD as a part of the compound structure.

The arylamine compounds of the general formula (1c-a) or (lc-b) are used as the second hole transport layer of the organic EL device of the present invention. These materials may be formed into a thin-film by a vapor deposition method or other known methods such as a spin coating method and an inkjet method.

Examples of material used for the electron blocking layer of the organic EL device of the present invention can be compounds having an electron blocking effect, including, for example, carbazole derivatives such as 4,4',4''-tri(N-carbazolyl)triphenylamine (hereinafter referred to as TCTA), 9,9-bis[4-(carbazol-9-yl)phenyl]fluorene, 1,3-bis(carbazol-9-yl)benzene (hereinafter referred to as mCP), and 2,2-bis(4-carbazol-9-ylphenyl)adamantane (Ad-Cz) ; and compounds having a triphenylsilyl group and a triarylamine structure, as represented by 9- [4-(carbazol-9-yl)phenyl]-9-[4-(triphenylsilyl)phenyl]-9H-fluorene, in addition to the arylamine compounds of the general formula (2) having a structure in which four triphenylamine structures are joined within a molecule via a single bond or a divalent group that does not contain a heteroatom, and the arylamine compounds of the general formula (3) having a structure in which two triphenylamine structures are joined within a molecule via a single bond or a divalent group that does not contain a heteroatom. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

Examples of material used for the light emitting layer of the organic EL device of the present invention can be various metal complexes, anthracene derivatives, bis(styryl)benzene derivatives, pyrene derivatives, oxazole derivatives, and polyparaphenylene vinylene derivatives, in addition to quinolinol derivative metal complexes such as Alq₃. Further, the light emitting layer may be made of a host material and a dopant material. Examples of the host material can be thiazole derivatives, benzimidazole derivatives, and polydialkyl fluorene derivatives, in addition to the above light-emitting materials. Examples of the dopant material can be quinacridone, coumarin, rubrene, perylene, pyrene, derivatives thereof, benzopyran derivatives, indenophenanthrene derivatives, rhodamine derivatives, and aminostyryl derivatives. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer.

Further, the light-emitting material may be a phosphorescent material. Phosphorescent materials as metal complexes of metals such as iridium and platinum may be used. Examples of the phosphorescent materials include green phosphorescent materials such as Ir(ppy)₃, blue phosphorescent materials such as FIrpic and FIr6, and red phosphorescent materials such as Btp₂Ir(acac). Here, carbazole derivatives such as 4,4'-di(N-carbazolyl)biphenyl (CBP), TCTA, and mCP may be used as the hole injecting and transporting host material. Compounds such as p-bis(triphenylsilyl)benzene (UGH2) and 2,2',2''-(1,3,5-phenylene)-tris(1-phenyl-1H-benzimidazole) (TPBI) may be used as the electron transporting host material. In this way, a high-performance organic EL device can be produced.

In order to avoid concentration quenching, the doping of the host material with the phosphorescent light-emitting material should preferably be made by co-evaporation in a range of 1 to 30 weight percent with respect to the whole light emitting layer.

Further, Examples of the light-emitting material may be delayed fluorescent-emitting material such as a CDCB derivative of PIC-TRZ, CC2TA, PXZ-TRZ, 4CzIPN or the like (refer to Non-Patent Document 3, for example).

These materials may be formed into a thin-film by using a vapor deposition method or other known methods such as a spin coating method and an inkjet method.

The hole blocking layer of the organic EL device of the present invention may be formed by using hole blocking compounds such as various rare earth complexes, triazole derivatives, triazine derivatives, and oxadiazole derivatives, in addition to the metal complexes of phenanthroline derivatives such as bathocuproin (BCP), and the metal complexes of quinolinol derivatives such as aluminum(III) bis(2-methyl-8-quinolinate)-4-phenylphenolate (hereinafter referred to as BAlq). These materials may also serve as the material of the electron transport layer. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

Material used for the electron transport layer of the organic EL device of the present invention can be the compounds of the general formula (4) having an anthracene ring structure, far preferably, the compounds of the general formula (4a) or (4b) having an anthracene ring structure. Other examples of material can be metal complexes of quinolinol derivatives such as Alq₃ and BAlq, various metal complexes, triazole derivatives, triazine derivatives, oxadiazole derivatives, pyridine derivatives, pyrimidine derivatives, benzimidazole derivatives, thiadiazole derivatives, anthracene derivatives, carbodiimide derivatives, quinoxaline derivatives, pyridoindole derivatives, phenanthroline derivatives, and silole derivatives. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

Examples of material used for the electron injection layer of the organic EL device of the present invention can be alkali metal salts such as lithium fluoride and cesium fluoride; alkaline earth metal salts such as magnesium fluoride; and metal oxides such as aluminum oxide. However, the electron injection layer may be omitted in the preferred selection of the electron transport layer and the cathode.

The cathode of the organic EL device of the present invention may be made of an electrode material with a low work function such as aluminum, or an alloy of an electrode material with an even lower work function such as a magnesium-silver alloy, a magnesium-indium alloy, or an aluminum-magnesium alloy.

The following describes an embodiment of the present invention in more detail based on Examples. The present invention, however, is not restricted to the following Examples.

### Example 5

### <Synthesis of 2,4''-bis{(biphenyl-4-yl)-phenylamino}-1,1':4',1''-terphenyl (Compound 1-24)>

4-Bromo-4'-{(biphenyl-4-yl)-phenylamino}-biphenyl (16.8 g), (biphenyl-4-yl)-{2-(4,4,5,5-tetramethyl-[1,3,2]dioxaborolan-2-yl)phenyl}-phenylamine (19.0 g), potassium carbonate (7.4 g), water (26 ml), toluene (200 ml), and ethanol (50 ml) were added into a nitrogen-substituted reaction vessel and aerated with nitrogen gas under ultrasonic irradiation for 30 minutes. After adding tetrakis(triphenylphosphine)palladium (0.87 g), the mixture was heated and refluxed for 20 hours while being stirred. After the mixture was cooled to a room temperature, an organic layer was collected by liquid separation, then dried over anhydrous magnesium sulfate and concentrated to obtain a crude product. After the crude product was purified by column chromatography (support: silica gel, eluent: heptane/toluene), the purified product was crystallized with an ethyl acetate/methanol mixed solvent to obtain a white powder of 2,4''-bis{(biphenyl-4-yl)-phenylamino}-1,1':4',1''-terphenyl (Compound 1-24; 20.8 g; yield 82%).

The structure of the obtained white powder was identified by NMR.
¹H-NMR (CDCl₃) detected 40 hydrogen signals, as follows.
δ (ppm) = 7.61 (2H), 7.56-6.83 (38H).

### Example 8

### <Synthesis of 4-{bis(biphenyl-4-yl)amino}-2''-{(biphenyl-4-yl)-phenylamino}-1,1':4',1''-terphenyl (Compound 1-27)>

2-{(biphenyl-4-yl)-phenylamino}-4''-bromo-1,1':4',1''-terphenyl (12.1 g), bis(biphenyl-4-yl)amine (8.0 g), tris(dibenzylideneacetone)palladium (0.6 g), tri-tert-butylphosphine (0.22 g), and tert-butoxy sodium (6.3 g) were added into a nitrogen-substituted reaction vessel, heated and refluxed for 3 hours while being stirred. After the mixture was cooled to a room temperature, methanol (600 ml) was added, and a precipitated crude product was collected by filtration. The crude product was dissolved in toluene, and after insoluble matter was removed by filtration, purification by crystallization with methanol was carried out. Then, recrystallization with a THF/methanol mixed solvent was carried out to obtain a white powder of 4-{bis(biphenyl-4-yl)amino}-2''-{(biphenyl-4-yl)-phenylamino}-1,1':4',1''-terphenyl (Compound 1-27; 15 g; yield 87%).

The structure of the obtained white powder was identified by NMR.
¹H-NMR (CDCl₃) detected 44 hydrogen signals, as follows.
δ (ppm) = 7.62 (4H), 7.58-6.91 (38H), 6.87 (2H).

### Example 10

### <Synthesis of 2-{bis(biphenyl-4-yl)amino}-4''-{(biphenyl-4-yl)-phenylamino}-1,1':4',1''-terphenyl (Compound 1-30)>

The reaction was carried out under the same conditions as those of Example 5, except that (biphenyl-4-yl)-{2-(4,4,5,5-tetramethyl-[1,3,2]dioxaborolan-2-yl)phenyl}-phenylamine was replaced with bis(biphenyl-4-yl)-{2-(4,4,5,5-tetramethyl-[1,3,2]dioxaborolan-2-yl)phenyl}amine. As a result, a white powder of 2-{bis(biphenyl-4-yl)amino}-4''-{(biphenyl-4-yl)-phenylaminol-1,1':4',1''-terphenyl (Compound 1-30; 15.7 g; yield 94%) was obtained.

The structure of the obtained white powder was identified by NMR.
¹H-NMR (CDCl₃) detected 44 hydrogen signals, as follows.
δ (ppm) = 7.60 (2H), 7.56-6.97 (42H).

### Example 11

### <Synthesis of 2,4''-bis{bis(biphenyl-4-yl)amino}-1,1':4',1''-terphenyl (Compound 1-31)>

The reaction was carried out under the same conditions as those of Example 5, except that 4-bromo-4'-{(biphenyl-4-yl)-phenylamino}-biphenyl was replaced with 4-bromo-4'-{bis(biphenyl-4-yl)amino}-biphenyl, and (biphenyl-4-yl)-{2-(4,4,5,5-tetramethyl-[1,3,2]dioxaborolan-2-yl)phenyl}-phenylamine was replaced with 2-{bis(biphenyl-4-yl)amino}phenylboronic acid. As a result, a white powder of 2,4''-bis{bis(biphenyl-4-yl)aminol-1,1':4',1''-terphenyl (Compound 1-31; 12 g; yield 76%) was obtained.

The structure of the obtained white powder was identified by NMR.
¹H-NMR (CDCl₃) detected 48 hydrogen signals, as follows. δ (ppm) = 7.65-6.98 (48H).

### Example 14

### <Synthesis of 2-{bis(biphenyl-4-yl)amino}-4''-{(naphthalen-1-yl)-phenylamino}-1,1':4',1''-terphenyl (Compound 1-36)>

The reaction was carried out under the same conditions as those of Example 5, except that 4-bromo-4'-{(biphenyl-4-yl)-phenylamino}-biphenyl was replaced with 4-bromo-4'-{(naphthalen-1-yl)-phenylamino}-biphenyl, and (biphenyl-4-yl)-{2-(4,4,5,5-tetramethyl-[1,3,2]dioxaborolan-2-yl)phenyl}-phenylamine was replaced with 2-{bis(biphenyl-4-yl)amino}phenylboronic acid. As a result, a white powder of 2-{bis(biphenyl-4-yl)amino}-4''-{(naphthalen-1-yl)-phenylamino}-1,1':4',1''-terphenyl (Compound 1-36; 12.8 g; yield 75%) was obtained.

The structure of the obtained white powder was identified by NMR.
¹H-NMR (CDCl₃) detected 42 hydrogen signals, as follows.
δ (ppm) = 7.99 (2H), 7.93 (2H), 7.81 (2H), 7.57-6.96 (36H).

### Example 15

### <Synthesis of 2-{(biphenyl-4-yl)-phenylamino}-4''-{(9,9-dimethyl-9H-fluoren-2-yl)-phenylamino}-1,1':4',1''-terphenyl (Compound 1-37)>

The reaction was carried out under the same conditions as those of Example 5, except that 4-bromo-4'-{(biphenyl-4-yl)-phenylamino}-biphenyl was replaced with 4-bromo-4'-{(9,9-dimethyl-9H-fluoren-2-yl)-phenylamino}-biphenyl. As a result, a white powder of 2-{(biphenyl-4-yl)-phenylamino}-4''-{(9,9-dimethyl-9H-fluoren-2-yl)-phenylamino}-1,1':4',1''-terphenyl (Compound 1-37; 11.7 g; yield 73%) was obtained.

The structure of the obtained white powder was identified by NMR.
¹H-NMR (CDCl₃) detected 44 hydrogen signals, as follows.
δ (ppm) = 7.68 (1H), 7.64-6.84 (37H), 1.48 (6H).

### Example 20

### <Synthesis of 2,2''-bis{(9,9-dimethyl-9H-fluoren-2-yl)-phenylamino}-1,1':4',1''-terphenyl (Compound 1-49)>

1,4-Dibromobenzene (6.51 g), 2-{(9,9-dimethyl-9H-fluoren-2-yl)-phenylamino}-phenylboronic acid (26.9 g), potassium carbonate (11.4 g), water (50 ml), toluene (200 ml), and ethanol (50 ml) were added into a nitrogen-substituted reaction vessel and aerated with nitrogen gas under ultrasonic irradiation for 30 minutes. The mixture was heated after adding tetrakis(triphenylphosphine)palladium (0.95 g), and stirred at 70°C for 12 hours. The mixture was cooled to a room temperature, and methanol (200 ml) was added. A precipitated solid was collected by filtration, and the solid was dissolved under heat after adding 1,2-dichlorobenzene (400 ml). Silica gel (20 g) was added, and the mixture was stirred for 30 minutes. After insoluble matter was removed by filtration, a precipitate formed by adding methanol (500 ml) was collected by filtration. The precipitate was dissolved by adding 1,2-dichlorobenzene (100 ml), and a crude product precipitated by adding toluene (100 ml) and methanol (100 ml) was collected by filtration. The crude product was washed under reflux with methanol (250 ml) to obtain a white powder of 2,2"-bis{(9,9-dimethyl-9H-fluoren-2-yl)-phenylamino}-1,1':4',1''-terphenyl (Compound 1-49; 13.7 g; yield 76%) was obtained.

The structure of the obtained white powder was identified by NMR.
¹H-NMR (THF-d₈) detected 48 hydrogen signals, as follows.
δ (ppm) = 7.53 (2H), 7.35-6.81 (30H), 6.76 (2H), 6.67 (2H), 1.29 (12H).

### Example 21

### <Synthesis of 2,2"-bis{bis(biphenyl-4-yl)amino}-1,1':4',1"-terphenyl (Compound 1-50)>

1,4 -Dibromobenzene (6.51 g), 2-{bis(biphenyl-4-yl)amino}-phenylboronic acid (26.9 g), potassium carbonate (11.4 g), water (50 ml), toluene (200 ml), and ethanol (50 ml) were added into a nitrogen-substituted reaction vessel and aerated with nitrogen gas under ultrasonic irradiation for 30 minutes. The mixture was heated after adding tetrakis(triphenylphosphine)palladium (0.95 g), and stirred at 70°C for 12 hours. The mixture was cooled to a room temperature, and methanol (200 ml) was added. A precipitated solid was collected by filtration, and the solid was dissolved under heat after adding 1,2-dichlorobenzene (400 ml). Silica gel (20 g) was added, and the mixture was stirred for 30 minutes. After insoluble matter was removed by filtration, a precipitate formed by adding methanol (500 ml) was collected by filtration. The precipitate was dissolved by adding 1,2-dichlorobenzene (100 ml), and a crude product precipitated by adding toluene (100 ml) and methanol (100 ml) was collected by filtration. The crude product was washed under reflux with methanol (250 ml) to obtain a white powder of 2,2"-bis{bis(biphenyl-4-yl)amino}-1,1':4',1''-terphenyl (Compound 1-50; 15.7 g; yield 78%) was obtained.

The structure of the obtained white powder was identified by NMR.
¹H-NMR (THF-d₈) detected 48 hydrogen signals, as follows.
δ (ppm) = 7.51-7.45 (8H), 7.33-7.18 (28H), 7.00 (4H), 6.90-6.82 (8H).

### Example 22

### <Synthesis of 2-{(9,9-dimethyl-9H-fluoren-2-yl)-phenylamino}-2"-[{4-(naphthalen-1-yl)phenyl}-phenylamino]-1,1':4',1"-terphenyl (Compound 1-55)>

The reaction was carried out under the same conditions as those of Example 5, except that 4-bromo-4'-{(biphenyl-4-yl)-phenylamino}-biphenyl was replaced with 4-bromo-2'-{4-(naphthalen-1-yl)phenyl}-phenylamino}-biphenyl, and (biphenyl-4-yl)-{2-(4,4,5,5-tetramethyl-[1,3,2]dioxaborolan-2-yl)phenyl)-phenylamine was replaced with 2-{(9,9-dimethyl-9H-fluoren-2-yl)-phenylamino}-phenylboronic acid. As a result, a white powder of 2-{(9,9-dimethyl-9H-fluoren-2-yl)-phenylamino}-2''-[{4-(naphthalen-1-yl)phenyl}-phenylamino]-1,1':4',1"-terphenyl (Compound 1-55; 7.3 g; yield 48%) was obtained.

The structure of the obtained white powder was identified by NMR.
¹H-NMR (THF-d₈) detected 46 hydrogen signals, as follows.
δ (ppm) = 7.89-7.76 (3H), 7.55-6.69 (37H), 1.29 (6H).

### Example 23

The melting points and the glass transition points of the arylamine compounds of the general formula (1c-a) or (1c-b) were determined using a high-sensitive differential scanning calorimeter (DSC3100SA produced by Bruker AXS).

| | Melting point | Glass transition point |
|---|---|---|
| Compound of Example 5 | No melting point observed | 107°C |
| Compound of Example 8 | No melting point observed | 119°C |
| Compound of Example 10 | No melting point observed | 118°C |
| Compound of Example 11 | No melting point observed | 133°C |
| Compound of Example 14 | No melting point observed | 117°C |
| Compound of Example 15 | 218°C | 114°C |
| Compound of Example 20 | 269°C | 117°C |
| Compound of Example 21 | 277°C | 122°C |
| Compound of Example 22 | No melting point observed | 117°C |

The arylamine compounds of the general formula (1c-a) or (1c-b) have glass transition points of 100°C or higher, demonstrating that the compounds have a stable thin-film state.

### Example 24

A 100 nm-thick vapor-deposited film was fabricated on an ITO substrate using the arylamine compounds of the general formula (1c-a) or (1c-b), and a work function was measured using an ionization potential measuring device (PYS-202 produced by Sumitomo Heavy Industries, Ltd.).

| | Work function |
|---|---|
| Compound of Example 5 | 5.76 eV |
| Compound of Example 8 | 5.71 eV |
| Compound of Example 10 | 5.72 eV |
| Compound of Example 11 | 5.70 eV |
| Compound of Example 14 | 5.70 eV |
| Compound of Example 15 | 5.67 eV |

As the results show, the arylamine compounds of the general formula (1c-a) or (1c-b) have desirable energy levels compared to the work function 5.4 eV of common hole transport materials such as NPD and TPD, and thus possess desirable hole transportability.

### Example 29

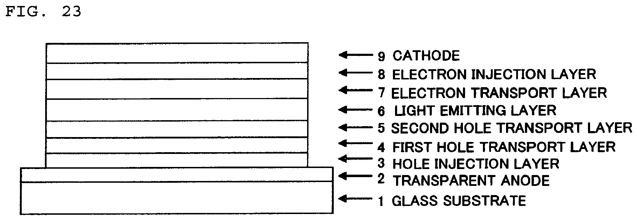

The organic EL device, as shown in FIG. 23, was fabricated by vapor-depositing a hole injection layer 3, a first hole transport layer 4, a second hole transport layer 5, a light emitting layer 6, an electron transport layer 7, an electron injection layer 8, and a cathode (aluminum electrode) 9 in this order on a glass substrate 1 on which an ITO electrode was formed as a transparent anode 2 beforehand.

Specifically, the glass substrate 1 having ITO (film thickness of 150 nm) formed thereon was subjected to ultrasonic washing in isopropyl alcohol for 20 minutes and then dried for 10 minutes on a hot plate heated to 200°C. After UV ozone treatment for 15 minutes, the glass substrate with ITO was installed in a vacuum vapor deposition apparatus, and the pressure was reduced to 0.001 Pa or lower. HIM-1 of the structural formula below was then formed in a film thickness of 5 nm as the hole injection layer 3 so as to cover the transparent anode 2. The first hole transport layer 4 was formed on the hole injection layer 3 by forming Compound 3-1 of the structural formula below in a film thickness of 60 nm. The second hole transport layer 5 was formed on the first hole transport layer 4 by forming the compound (1-24) of Example 5 in a film thickness of 5 nm. Then, the light emitting layer 6 was formed on the second hole transport layer 5 in a film thickness of 20 nm by dual vapor deposition of EMD-1 (NUBD370 produced by SFC Co., Ltd.) and EMH-1 (ABH113 produced by SFC Co., Ltd.) at a vapor deposition rate ratio of EMD-1:EMH-1 = 5:95. The electron transport layer 7 was formed on the light emitting layer 6 in a film thickness of 30 nm by dual vapor deposition of Compound 4b-1 of the structural formula below and ETM-1 of the structural formula below at a vapor deposition rate ratio of Compound 4b-1:ETM-1 = 50:50. The electron injection layer 8 was formed on the electron transport layer 7 by forming lithium fluoride in a film thickness of 1 nm. Finally, the cathode 9 was formed by vapor-depositing aluminum in a thickness of 100 nm. The characteristics of the thus fabricated organic EL device were measured in the atmosphere at an ordinary temperature. Table 1 summarizes the results of emission characteristics measurements performed by applying a DC voltage to the fabricated organic EL device.

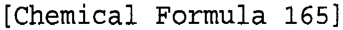

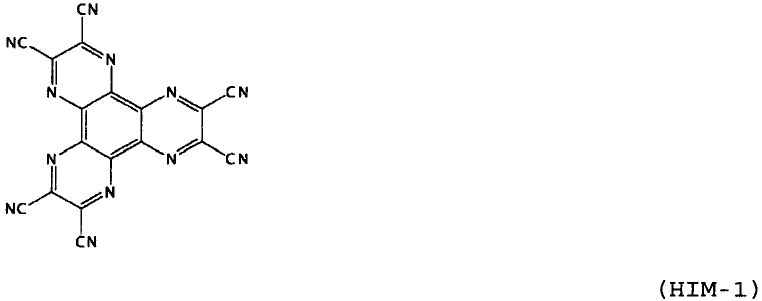

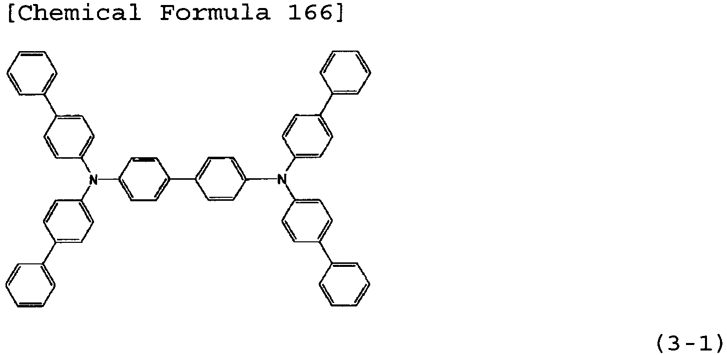

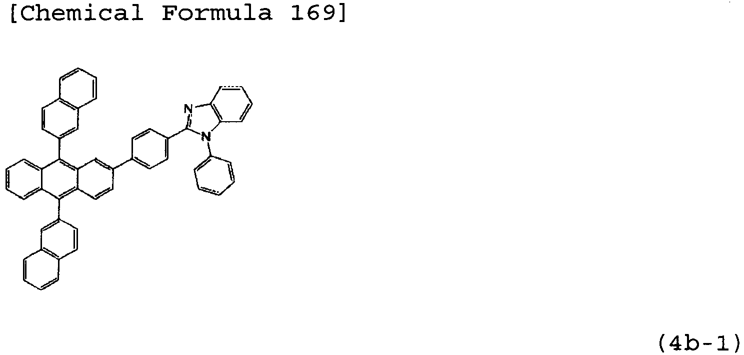

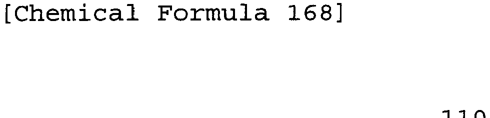

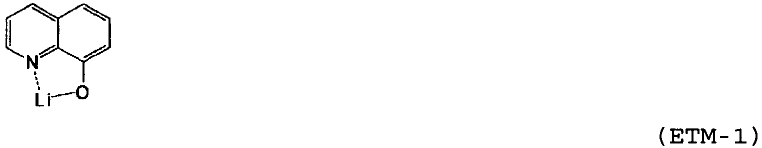

### Example 31

An organic EL device was fabricated under the same conditions used in Example 29, except that the second hole transport layer 5 was formed by forming the compound (1-27) of Example 8 in a film thickness of 5 nm, instead of using the compound (1-24) of Example 5. The characteristics of the organic EL device thus fabricated were measured in the atmosphere at an ordinary temperature. Table 1 summarizes the results of emission characteristics measurements performed by applying a DC voltage to the fabricated organic EL device.

### Example 32

An organic EL device was fabricated under the same conditions used in Example 29, except that the second hole transport layer 5 was formed by forming the compound (1-30) of Example 10 in a film thickness of 5 nm, instead of using the compound (1-24) of Example 5. The characteristics of the organic EL device thus fabricated were measured in the atmosphere at an ordinary temperature. Table 1 summarizes the results of emission characteristics measurements performed by applying a DC voltage to the fabricated organic EL device.

### Example 33

An organic EL device was fabricated under the same conditions used in Example 29, except that the second hole transport layer 5 was formed by forming the compound (1-31) of Example 11 in a film thickness of 5 nm, instead of using the compound (1-24) of Example 5. The characteristics of the organic EL device thus fabricated were measured in the atmosphere at an ordinary temperature. Table 1 summarizes the results of emission characteristics measurements performed by applying a DC voltage to the fabricated organic EL device.

### Example 36

An organic EL device was fabricated under the same conditions used in Example 29, except that the second hole transport layer 5 was formed by forming the compound (1-36) of Example 14 in a film thickness of 5 nm, instead of using the compound (1-24) of Example 5. The characteristics of the organic EL device thus fabricated were measured in the atmosphere at an ordinary temperature. Table 1 summarizes the results of emission characteristics measurements performed by applying a DC voltage to the fabricated organic EL device.

### Example 37

An organic EL device was fabricated under the same conditions used in Example 29, except that the second hole transport layer 5 was formed by forming the compound (1-37) of Example 15 in a film thickness of 5 nm, instead of using the compound (1-24) of Example 5. The characteristics of the organic EL device thus fabricated were measured in the atmosphere at an ordinary temperature. Table 1 summarizes the results of emission characteristics measurements performed by applying a DC voltage to the fabricated organic EL device.

### Example 40

An organic EL device was fabricated under the same conditions used in Example 29, except that the second hole transport layer 5 was formed by forming the compound (1-50) of Example 21 in a film thickness of 5 nm, instead of using the compound (1-24) of Example 5. The characteristics of the organic EL device thus fabricated were measured in the atmosphere at an ordinary temperature. Table 1 summarizes the results of emission characteristics measurements performed by applying a DC voltage to the fabricated organic EL device.

### Comparative Example 1

For comparison, an organic EL device was fabricated under the same conditions used in Example 29, except that Compound 4b-1 was replaced with Compound 4a-1 and that the second hole transport layer 5 was formed by forming Compound 3-1 of the structural formula in a film thickness of 5 nm, instead of using the compound (1-24) of Example 5, after the first hole transport layer 4 was formed by forming Compound 3-1 of the structural formula in a film thickness of 60 nm. The characteristics of the organic EL device thus fabricated were measured in the atmosphere at an ordinary temperature. Table 1 summarizes the results of emission characteristics measurements performed by applying a DC voltage to the fabricated organic EL device.

### Comparative Example 2

For comparison, an organic EL device was fabricated under the same conditions used in Example 29, except using Compound 3'-2 of the structural formula below instead of Compound 3-1 of the structural formula as material of the first hole transport layer 4, and further except performing dual vapor deposition of EMD-2 (SBD160 produced by SFC Co., Ltd.) and EMH-2 (ABH401 produced by SFC Co., Ltd.) at a vapor deposition rate ratio of EMD-2:EMH-2 = 5:95 instead of using EMD-1 (NUBD370 produced by SFC Co., Ltd.) and EMH-1 (ABH113 produced by SFC Co., Ltd.) as material of the light emitting layer 6 and except that the second hole transport layer 5 was formed by forming Compound 3'-2 of the structural formula in a film thickness of 5 nm, instead of using the compound (1-24) of Example 5, after the first hole transport layer 4 was formed by forming Compound 3'-2 of the structural formula in a film thickness of 60 nm. Furthermore, except that Compound 4b-1 was replaced with Compound 4a-1 of the structural formula below as material of the electron transport layer 7. The characteristics of the organic EL device thus fabricated were measured in the atmosphere at an ordinary temperature. Table 1 summarizes the results of emission characteristics measurements performed by applying a DC voltage to the fabricated organic EL device.

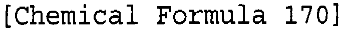

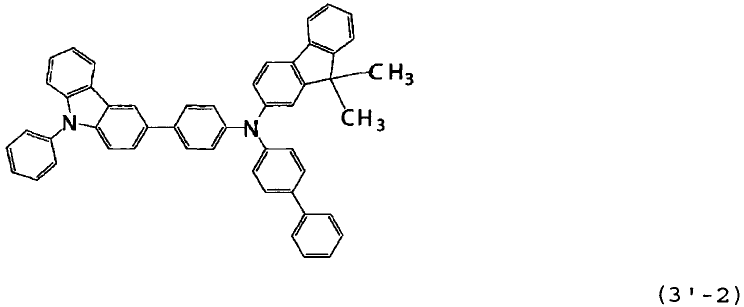

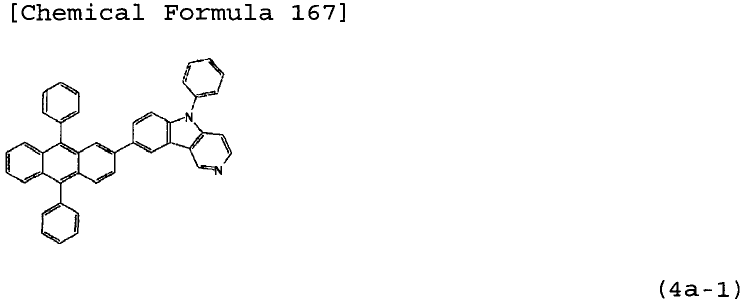

Table 1 summarizes the results of device lifetime measurements performed with organic EL devices fabricated in Examples 25 to 43 and Comparative Examples 1 to 2. A device lifetime was measured as the time elapsed until the emission luminance of 2,000 cd/m² (initial luminance) at the start of emission was attenuated to 1,900 cd/m² (corresponding to attenuation to 95% when taking the initial luminance as 100%) when carrying out constant current driving.

**[Table 1]**

| | First hole transport layer | Second hole transport layer | Light emitting layer | Electron transport layer | Voltage [V] (@10mA/cm²) | Luminance [cd/m²] (@10mA/cm²) | Current efficiency [cd/A] (@10mA/cm²) | Power efficiency [lm/W] (@10mA/cm²) | Device lifetime (Attenuatior to 95%) |
|---|---|---|---|---|---|---|---|---|---|
| Ex. 29 | Compound 3-1 | Compound 1-24 | EMD-1/EMH-1 | Compound 4b-1/ETM-1 | 3.94 | 722 | 7.21 | 5.76 | 148 h |
| Ex. 31 | Compound 3-1 | Compound 1-27 | EMD-1/EMH-1 | Compound 4b-1/ETM-1 | 3.94 | 725 | 7.24 | 5.78 | 104 h |
| Ex. 32 | Compound 3-1 | Compound 1-30 | EMD-1/EMH-1 | Compound 4b-1/ETM-1 | 4.00 | 748 | 7.48 | 5.78 | 179 h |
| Ex. 33 | Compound 3-1 | Compound 1-31 | EMD-1/EMH-1 | Compound 4b-1/ETM-1 | 4.00 | 747 | 7.46 | 5.87 | 110 h |
| Ex. 36 | Compound 3-1 | Compound 1-36 | EMD-1/EMH-1 | Compound 4b-1/ETM-1 | 3.92 | 719 | 7.18 | 5.77 | 161 h |
| Ex. 37 | Compound 3-1 | Compound 1-37 | EMD-1/EMH-1 | Compound 4b-1/ETM-1 | 4.00 | 742 | 7.42 | 5.82 | 119 h |
| Ex. 40 | Compound 3-1 | Compound 1-50 | EMD-1/EMH-1 | Compound 4b-1/ETM-1 | 3.88 | 722 | 7.21 | 5.83 | 141 h |
| Com. Ex. 1 | Compound 3-1 | Compound 3-1 | EMD-1/EMH-1 | Compound 4a-1/ETM-1 | 4.00 | 669 | 6.69 | 5.25 | 60 h |
| Com. Ex. 2 | Compound 3'-2 | Compound 3'-2 | EMD-2/EMH-2 | Compound 4a-1/ETM-1 | 4.01 | 680 | 6.80 | 5.34 | 57 h |

As shown in Table 1, the current efficiency upon passing a current with a current density of 10 mA/cm² was 7.18 to 7.48 cd/A for the organic EL devices in Examples 29, 31 to 33, 36, 37 and 40, which was higher than 6.69 to 6.80 cd/A for the organic EL devices in Comparative Examples 1 to 2. Further, the power efficiency was 5.76 to 5.83 lm/W for the organic EL devices in Examples 29, 31 to 33, 36, 37 and 40, which was higher than 5.25 to 5.34 lm/W for the organic EL devices in Comparative Examples 1 to 2. Table 1 also shows that the device lifetime (attenuation to 95%) was 104 to 179 hours for the organic EL devices in Examples 29, 31 to 33, 36, 37 and 40, showing achievement of a far longer lifetime than 57 to 60 hours for the organic EL devices in Comparative Examples 1 to 2.

In the organic EL devices of the present invention, the combination of two specific kinds of arylamine compounds and specific compounds having an anthracene ring structure can improve carrier balance inside the organic EL devices and achieve high luminous efficiency and a long lifetime, compared to the conventional organic EL devices.

In the organic EL devices of the present invention with the combination of two specific kinds of arylamine compounds and specific compounds having an anthracene ring structure, luminous efficiency and durability of an organic EL device can be improved to attain potential applications for, for example, home electric appliances and illuminations.

### Description of Reference Numeral

- 1: Glass substrate
- 2: Transparent anode
- 3: Hole injection layer
- 4: First hole transport layer
- 5: Second hole transport layer
- 6: Light emitting layer
- 7: Electron transport layer
- 8: Electron injection layer
- 9: Cathode

## Claims

1. An organic electroluminescent device comprising at least an anode (2), a hole injection layer (3), a first hole transport layer (4), a second hole transport layer (5), a light emitting layer (6), an electron transport layer (7) and a cathode (9) in this order, **characterised in that** the second hole transport layer (5) comprises an arylamine compound represented by the following general formula (1c-a) or (1c-b): 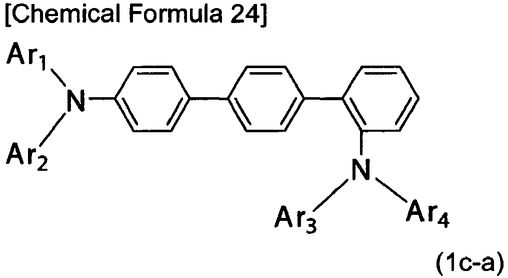 wherein Ar₁ to Ar₄ may be the same or different, and represent a substituted or unsubstituted aromatic hydrocarbon group, a substituted or unsubstituted aromatic heterocyclic group, or a substituted or unsubstituted condensed polycyclic aromatic group; 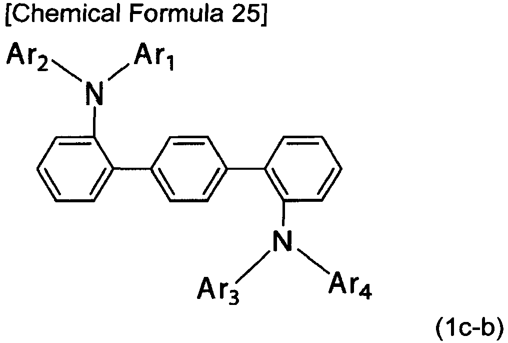 wherein Ar₁ to Ar₄ may be the same or different, and represent a substituted or unsubstituted aromatic hydrocarbon group, a substituted or unsubstituted aromatic heterocyclic group, or a substituted or unsubstituted condensed polycyclic aromatic group.

2. The organic electroluminescent device according to claim 1, wherein the first hole transport layer (4) comprises an arylamine compound having a structure in which three to six triphenylamine structures are joined within a molecule via a single bond or a divalent group that does not contain a heteroatom.

3. The organic electroluminescent device according to claim 2, wherein the arylamine compound having a structure in which three to six triphenylamine structures are joined within a molecule via a single bond or a divalent group that does not contain a heteroatom is an arylamine compound of the following general formula (2) having four triphenylamine structures within a molecule, 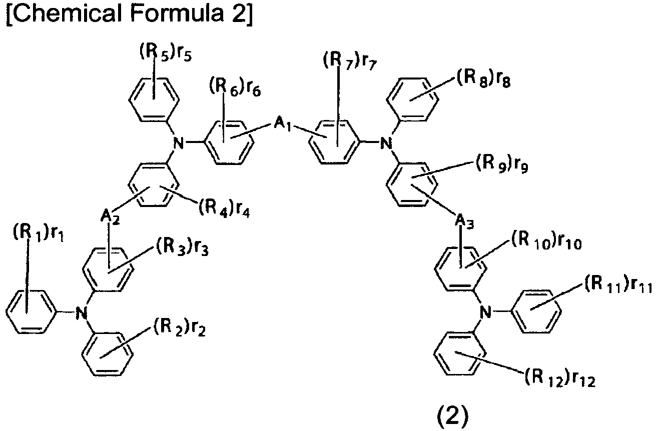 wherein R₁ to R₁₂ represent a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy; r₁ to r₁₂ may be the same or different, r₁, r₂, r₅, r₈, r₁₁, and r₁₂ representing 0 or an integer of 1 to 5, and r₃, r₄, r₆, r₇, r₉, and r₁₀ representing 0 or an integer of 1 to 4, where when r₁, r₂, r₅, r₈, r₁₁, and r₁₂ are an integer of 2 to 5, or when r₃, r₄, r₆, r₇, r₉, and r₁₀ are an integer of 2 to 4, R₁ to R₁₂, a plurality of which bind to the same benzene ring, may be the same or different and may bind to each other via a single bond, substituted or unsubstituted methylene, an oxygen atom, or a sulfur atom to form a ring; and A₁, A₂, and A₃ may be the same or different, and represent a divalent group represented by the following structural formulae (B) to (G), or a single bond, 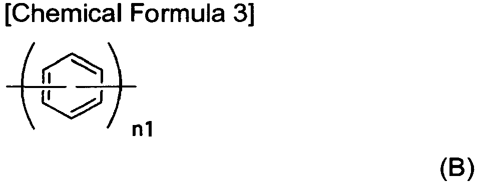 wherein n1 represents an integer of 1 to 3, 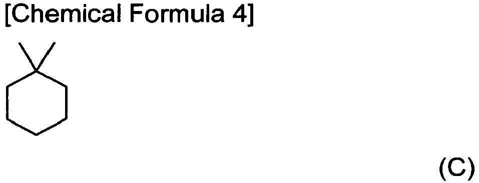 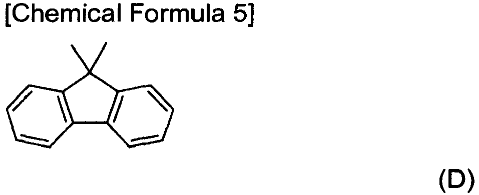 [Chemical Formula 6]
-CH₂- (E)

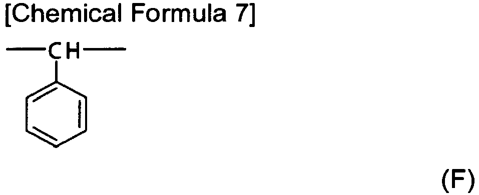

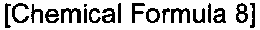

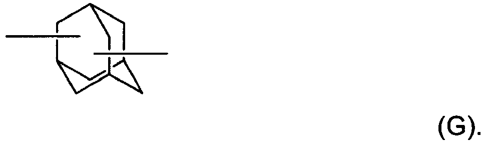

4. The organic electroluminescent device according to claim 1, wherein the first hole transport layer (4) comprises an arylamine compound having a structure in which two triphenylamine structures are joined within a molecule via a single bond or a divalent group that does not contain a heteroatom.

5. The organic electroluminescent device according to claim 4, wherein the arylamine compound having a structure in which two triphenylamine structures are joined within a molecule via a single bond or a divalent group that does not contain a heteroatom is an arylamine compound represented by the following general formula (3): 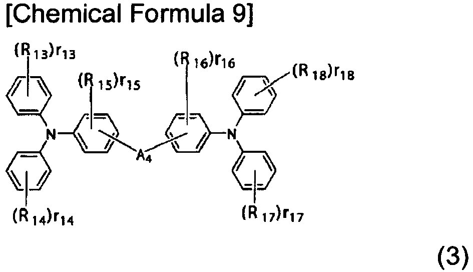 wherein R₁₃ to R₁₈ represent a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy; r₁₃ to r₁₈ may be the same or different, r₁₃, r₁₄, r₁₇, and r₁₈ representing 0 or an integer of 1 to 5, and r₁₅ and r₁₆ representing 0 or an integer of 1 to 4, where when r₁₃, r₁₄, r₁₇, and r₁₈ are an integer of 2 to 5, or when r₁₅ and r₁₆ are an integer of 2 to 4, R₁₃ to R₁₈, a plurality of which bind to the same benzene ring, may be the same or different and may bind to each other via a single bond, substituted or unsubstituted methylene, an oxygen atom, or a sulfur atom to form a ring; and A₄ represents a divalent group represented by the following structural formulae (C) to (G), or a single bond, 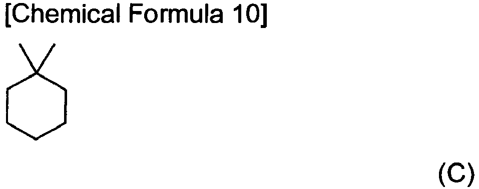 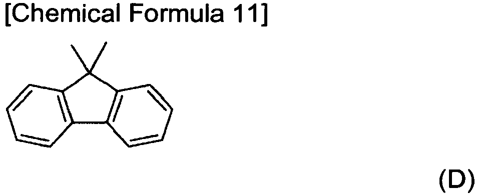 [Chemical Formula 12]
-CH₂- (E)

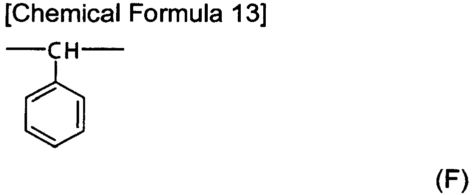

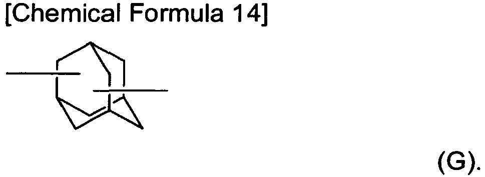

6. The organic electroluminescent device according to claim 1, wherein the electron transport layer (7) comprises a compound of the following general formula (4) having an anthracene ring structure, 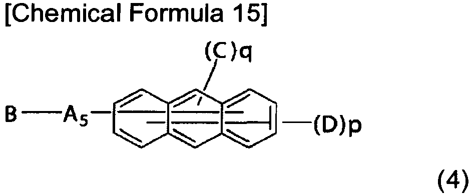 wherein A₅ represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond; B represents a substituted or unsubstituted aromatic heterocyclic group; C represents a substituted or unsubstituted aromatic hydrocarbon group, a substituted or unsubstituted aromatic heterocyclic group, or a substituted or unsubstituted condensed polycyclic aromatic group; D may be the same or different, and represents a hydrogen atom, a deuterium atom, a fluorine atom, a chlorine atom, cyano, trifluoromethyl, linear or branched alkyl of 1 to 6 carbon atoms, a substituted or unsubstituted aromatic hydrocarbon group, a substituted or unsubstituted aromatic heterocyclic group, or a substituted or unsubstituted condensed polycyclic aromatic group; and p represents 7 or 8, and q represents 1 or 2 while maintaining a relationship that a sum of p and q is 9.

7. The organic electroluminescent device according to claim 6, wherein the compound having an anthracene ring structure is a compound of the following general formula (4a) having an anthracene ring structure, 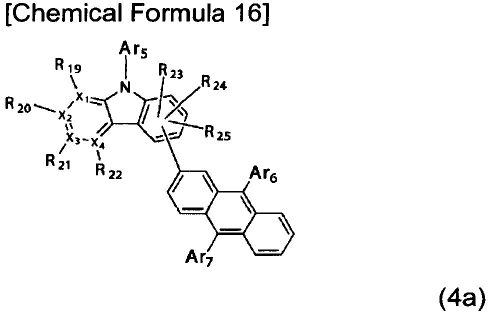 wherein Ar₅, Ar₆, and Ar₇ may be the same or different, and represent a substituted or unsubstituted aromatic hydrocarbon group, a substituted or unsubstituted aromatic heterocyclic group, or a substituted or unsubstituted condensed polycyclic aromatic group; R₁₉ to R₂₅ may be the same or different, and represent a hydrogen atom, a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, where R₁₉ to R₂₅ may bind to each other via a single bond, substituted or unsubstituted methylene, an oxygen atom, or a sulfur atom to form a ring; and X₁, X₂, X₃, and X₄ represent a carbon atom or a nitrogen atom, where only one of X₁, X₂, X₃, and X₄ is a nitrogen atom, and, in this case, the nitrogen atom does not have the hydrogen atom or substituent for R₁₉ to R₂₂.

8. The organic electroluminescent device according to claim 6, wherein the compound having an anthracene ring structure is a compound of the following general formula (4b) having an anthracene ring structure, 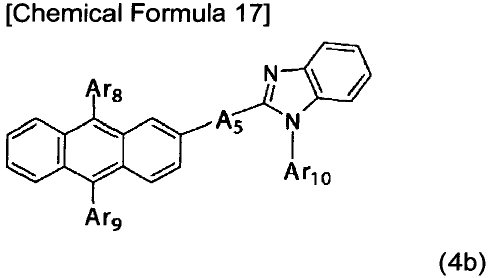 wherein A₅ represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond; and Ar₈, Ar₉, and Ar₁₀ may be the same or different, and represent a substituted or unsubstituted aromatic hydrocarbon group, a substituted or unsubstituted aromatic heterocyclic group, or a substituted or unsubstituted condensed polycyclic aromatic group.

## Patentansprüche

1. Organische elektrolumineszierende Vorrichtung, umfassend mindestens eine Anode (2), eine Lochinjektionsschicht (3), eine erste Lochtransportschicht (4), eine zweite Lochtransportschicht (5), eine lichtemittierende Schicht (6), eine Elektronentransportschicht (7) und eine Kathode (9) in dieser Reihenfolge, **dadurch gekennzeichnet, dass** die zweite Lochtransportschicht (5) eine Arylaminverbindung, dargestellt durch die nachstehende allgemeine Formel (1c-a) oder (1c-b), umfasst: 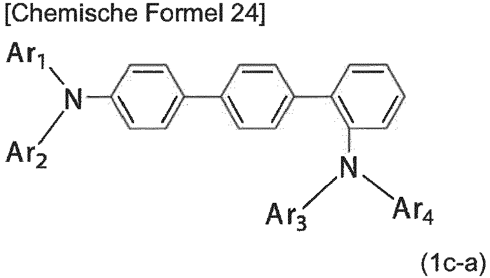 wobei Ar₁ bis Ar₄ gleich oder verschieden sein können und eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellen; 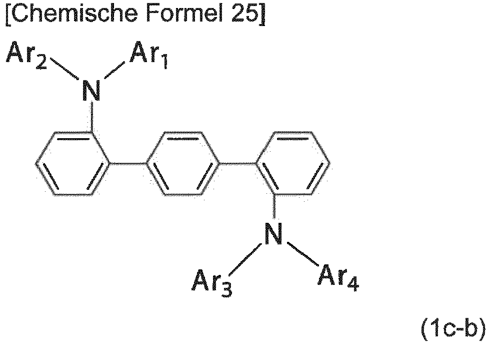 wobei Ar₁ bis Ar₄ gleich oder verschieden sein können und eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellen.

2. Organische elektrolumineszierende Vorrichtung nach Anspruch 1, wobei die erste Lochtransportschicht (4) eine Arylaminverbindung mit einer Struktur umfasst, in der drei bis sechs Triphenylaminstrukturen innerhalb eines Moleküls über eine Einfachbindung oder eine zweiwertige Gruppe, die kein Heteroatom enthält, verbunden sind.

3. Organische elektrolumineszierende Vorrichtung nach Anspruch 2, wobei die Arylaminverbindung mit einer Struktur, in der drei bis sechs Triphenylaminstrukturen innerhalb eines Moleküls über eine Einfachbindung oder eine zweiwertige Gruppe, die kein Heteroatom enthält, verbunden sind, eine Arylaminverbindung der nachstehenden allgemeinen Formel (2) mit vier Triphenylaminstrukturen innerhalb eines Moleküls ist, 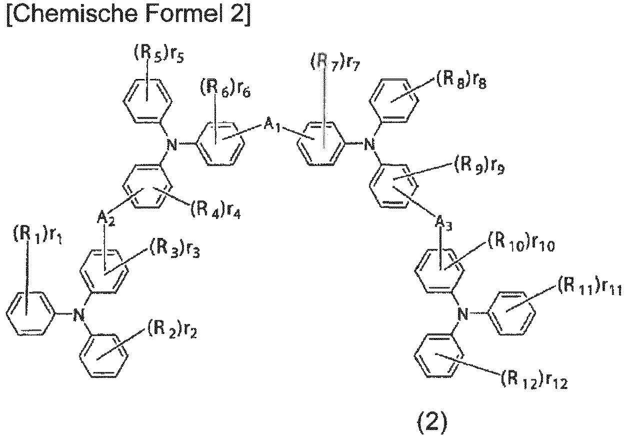 wobei R₁ bis R₁₂ ein Deuteriumatom, ein Fluoratom, ein Chloratom, Cyano, Nitro, unverzweigtes oder verzweigtes Alkyl mit 1 bis 6 Kohlenstoffatomen, das einen Substituenten aufweisen kann, Cycloalkyl mit 5 bis 10 Kohlenstoffatomen, das einen Substituenten aufweisen kann, unverzweigtes oder verzweigtes Alkenyl mit 2 bis 6 Kohlenstoffatomen, das einen Substituenten aufweisen kann, unverzweigtes oder verzweigtes Alkyloxy mit 1 bis 6 Kohlenstoffatomen, das einen Substituenten aufweisen kann, Cycloalkyloxy mit 5 bis 10 Kohlenstoffatomen, das einen Substituenten aufweisen kann, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder substituiertes oder unsubstituiertes Aryloxy darstellen; r₁ bis r₁₂ gleich oder verschieden sein können, r₁, r₂, r₅, r₈, r₁₁ und r₁₂ 0 oder eine ganze Zahl von 1 bis 5 darstellen, und r₃, r₄, r₆, r₇, r₉ und r₁₀ 0 oder eine ganze Zahl von 1 bis 4 darstellen, wobei wenn r₁, r₂, r₅, r₈, r₁₁ und r₁₂ eine ganze Zahl von 2 bis 5 sind, oder wenn r₃, r₄, r₆, r₇, r₉ und r₁₀ eine ganze Zahl von 2 bis 4 sind, R₁ bis R₁₂, von denen eine Mehrzahl an den gleichen Benzolring bindet, gleich oder verschieden sein können und über eine Einfachbindung, substituiertes oder unsubstituiertes Methylen, ein Sauerstoffatom oder ein Schwefelatom unter Bildung eines Rings aneinander binden können; und A₁, A₂ und A₃ gleich oder verschieden sein können und eine zweiwertige Gruppe, dargestellt durch die nachstehenden Strukturformeln (B) bis (G), oder eine Einfachbindung darstellen, 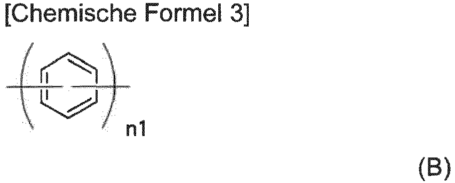 wobei n1 eine ganze Zahl von 1 bis 3 darstellt, 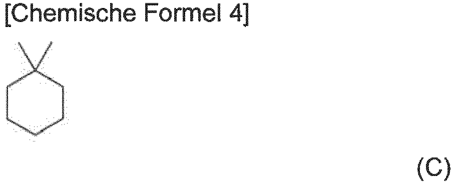 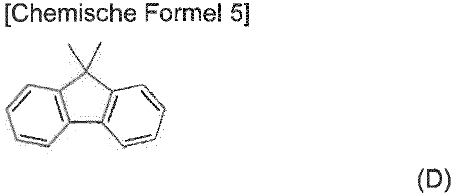 [Chemische Formel 6]
-CH₂- (E)

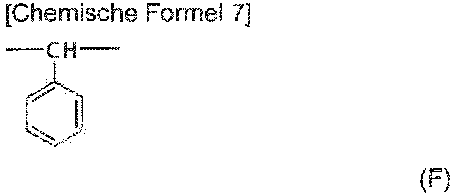

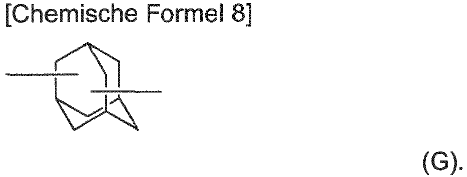

4. Organische elektrolumineszierende Vorrichtung nach Anspruch 1, wobei die erste Lochtransportschicht (4) eine Arylaminverbindung mit einer Struktur umfasst, in der zwei Triphenylaminstrukturen innerhalb eines Moleküls über eine Einfachbindung oder eine zweiwertige Gruppe, die kein Heteroatom enthält, verbunden sind.

5. Organische elektrolumineszierende Vorrichtung nach Anspruch 4, wobei die Arylaminverbindung mit einer Struktur, in der zwei Triphenylaminstrukturen innerhalb eines Moleküls über eine Einfachbindung oder eine zweiwertige Gruppe, die kein Heteroatom enthält, verbunden sind, eine Arylaminverbindung ist, dargestellt durch die nachstehende allgemeine Formel (3): 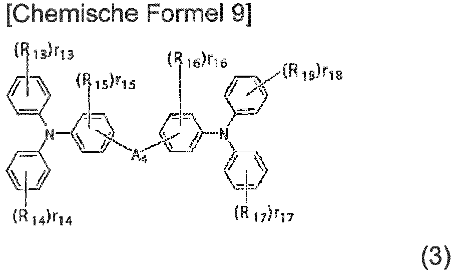 wobei R₁₃ bis R₁₈ ein Deuteriumatom, ein Fluoratom, ein Chloratom, Cyano, Nitro, unverzweigtes oder verzweigtes Alkyl mit 1 bis 6 Kohlenstoffatomen, das einen Substituenten aufweisen kann, Cycloalkyl mit 5 bis 10 Kohlenstoffatomen, das einen Substituenten aufweisen kann, unverzweigtes oder verzweigtes Alkenyl mit 2 bis 6 Kohlenstoffatomen, das einen Substituenten aufweisen kann, unverzweigtes oder verzweigtes Alkyloxy mit 1 bis 6 Kohlenstoffatomen, das einen Substituenten aufweisen kann, Cycloalkyloxy mit 5 bis 10 Kohlenstoffatomen, das einen Substituenten aufweisen kann, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder substituiertes oder unsubstituiertes Aryloxy darstellen; r₁₃ bis r₁₈ gleich oder verschieden sein können, r₁₃, r₁₄, r₁₇ und r₁₈ 0 oder eine ganze Zahl von 1 bis 5 darstellen, und r₁₅ und r₁₆ 0 oder eine ganze Zahl von 1 bis 4 darstellen, wobei wenn r₁₃, r₁₄, r₁₇ und r₁₈ eine ganze Zahl von 2 bis 5 sind, oder wenn r₁₅ und r₁₆ eine ganze Zahl von 2 bis 4 sind, R₁₃ bis R₁₈, von denen eine Mehrzahl an den gleichen Benzolring bindet, gleich oder verschieden sein können und über eine Einfachbindung, substituiertes oder unsubstituiertes Methylen, ein Sauerstoffatom oder ein Schwefelatom unter Bildung eines Rings aneinander binden können; und A4 eine zweiwertige Gruppe, dargestellt durch die nachstehenden Strukturformeln (C) bis (G), oder eine Einfachbindung darstellt, 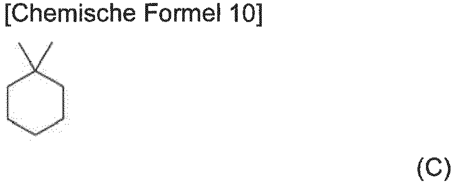 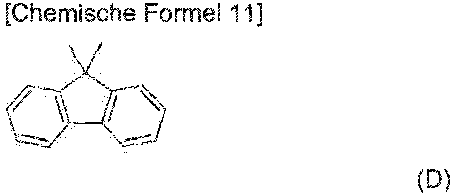 [Chemische Formel 12]
-CH₂- (E)

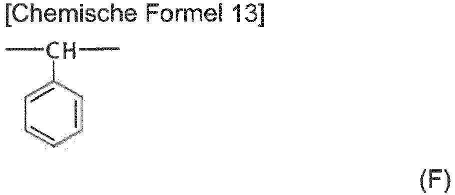

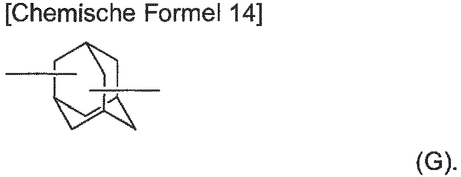

6. Organische elektrolumineszierende Vorrichtung nach Anspruch 1, wobei die Elektronentransportschicht (7) eine Verbindung der nachstehenden allgemeinen Formel (4) mit einer Anthracenringstruktur umfasst, 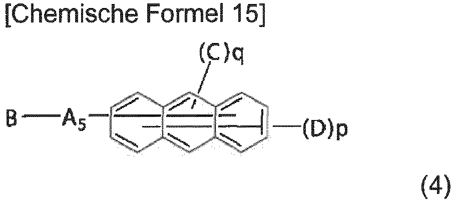 wobei A₅ eine zweiwertige Gruppe eines substituierten oder unsubstituierten aromatischen Kohlenwasserstoffs, eine zweiwertige Gruppe eines substituierten oder unsubstituierten aromatischen heterocyclischen Rings, eine zweiwertige Gruppe von substituierten oder unsubstituierten kondensierten polycyclischen Aromaten oder eine Einfachbindung darstellt; B eine substituierte oder unsubstituierte aromatische heterocyclische Gruppe darstellt; C eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellt; D gleich oder verschieden sein kann und ein Wasserstoffatom, ein Deuteriumatom, ein Fluoratom, ein Chloratom, Cyano, Trifluormethyl, unverzweigtes oder verzweigtes Alkyl mit 1 bis 6 Kohlenstoffatomen, eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellt; und p 7 oder 8 darstellt und q 1 oder 2 darstellt, während eine Beziehung aufrechterhalten wird, dass eine Summe von p und q 9 ist.

7. Organische elektrolumineszierende Vorrichtung nach Anspruch 6, wobei die Verbindung mit einer Anthracenringstruktur eine Verbindung der nachstehenden allgemeinen Formel (4a) mit einer Anthracenringstruktur ist, 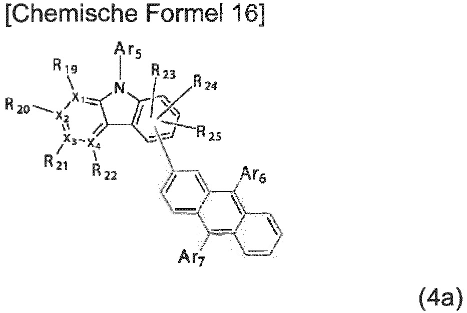 wobei Ar₅, Ar₆ und Ar₇ gleich oder verschieden sein können und eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellen; R₁₉ bis R₂₅ gleich oder verschieden sein können und ein Wasserstoffatom, ein Deuteriumatom, ein Fluoratom, ein Chloratom, Cyano, Nitro, unverzweigtes oder verzweigtes Alkyl mit 1 bis 6 Kohlenstoffatomen, das einen Substituenten aufweisen kann, Cycloalkyl mit 5 bis 10 Kohlenstoffatomen, das einen Substituenten aufweisen kann, unverzweigtes oder verzweigtes Alkenyl mit 2 bis 6 Kohlenstoffatomen, das einen Substituenten aufweisen kann, unverzweigtes oder verzweigtes Alkyloxy mit 1 bis 6 Kohlenstoffatomen, das einen Substituenten aufweisen kann, Cycloalkyloxy mit 5 bis 10 Kohlenstoffatomen, das einen Substituenten aufweisen kann, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder substituiertes oder unsubstituiertes Aryloxy darstellen, wobei R₁₉ bis R₂₅ über eine Einfachbindung, substituiertes oder unsubstituiertes Methylen, ein Sauerstoffatom oder ein Schwefelatom unter Bildung eines Rings aneinander binden können; und X₁, X₂, X₃ und X₄ ein Kohlenstoffatom oder ein Stickstoffatom darstellen, wobei nur eines von X₁, X₂, X₃ und X₄ ein Stickstoffatom ist, und in diesem Fall das Stickstoffatom nicht das Wasserstoffatom oder den Substituenten für R₁₉ bis R₂₂ aufweist.

8. Organische elektrolumineszierende Vorrichtung nach Anspruch 6, wobei die Verbindung mit einer Anthracenringstruktur eine Verbindung der nachstehenden allgemeinen Formel (4b) mit einer Anthracenringstruktur ist, 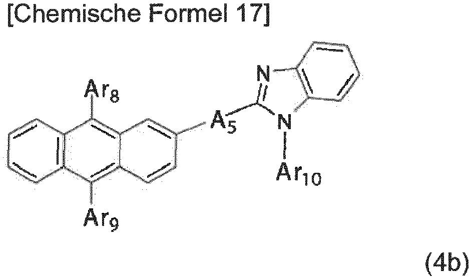 wobei A₅ eine zweiwertige Gruppe eines substituierten oder unsubstituierten aromatischen Kohlenwasserstoffs, eine zweiwertige Gruppe eines substituierten oder unsubstituierten aromatischen heterocyclischen Rings, eine zweiwertige Gruppe von substituierten oder unsubstituierten kondensierten polycyclischen Aromaten oder eine Einfachbindung darstellt; und Ar₈, Ar₉ und Ar₁₀ gleich oder verschieden sein können und eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellen.

## Revendications

1. Dispositif électroluminescent organique comprenant au moins une anode (2), une couche d'injection de trous (3), une première couche de transport de trous (4), une deuxième couche de transport de trous (5), une couche émettrice de lumière (6), une couche de transport d'électrons (7) et une cathode (9) en cet ordre, **caractérisé en ce que**
la deuxième couche de transport de trous (5) comprend un composé arylamine représenté par la formule générale (1c-a) ou (1c-b) suivante : 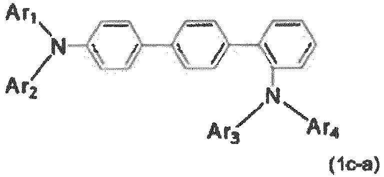 où Ar₁ à Ar₄ peuvent être identiques ou différents, et représentent un groupe hydrocarboné aromatique substitué ou non substitué, un groupe hétérocyclique aromatique substitué ou non substitué, ou un groupe polycyclique condensé aromatique substitué ou non substitué ; 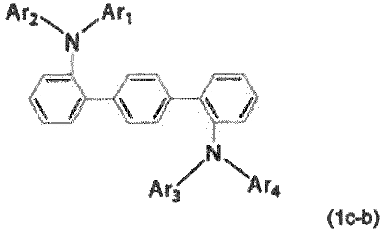 où Ar₁ à Ar₄ peuvent être identiques ou différents, et représentent un groupe hydrocarboné aromatique substitué ou non substitué, un groupe hétérocyclique aromatique substitué ou non substitué, ou un groupe polycyclique condensé aromatique substitué ou non substitué.

2. Dispositif électroluminescent organique selon la revendication 1, dans lequel la première couche de transport de trous (4) comprend un composé arylamine ayant une structure dans laquelle trois à six structures triphénylamine sont jointes au sein d'une molécule via une simple liaison ou un groupe bivalent qui ne contient pas d'hétéroatome.

3. Dispositif électroluminescent organique selon la revendication 2, dans lequel le composé arylamine ayant une structure dans laquelle trois à six structures triphénylamine sont jointes au sein d'une molécule via une simple liaison ou un groupe bivalent qui ne contient pas d'hétéroatome, est un composé arylamine de la formule générale (2) suivante, ayant quatre structures triphénylamine par molécule, 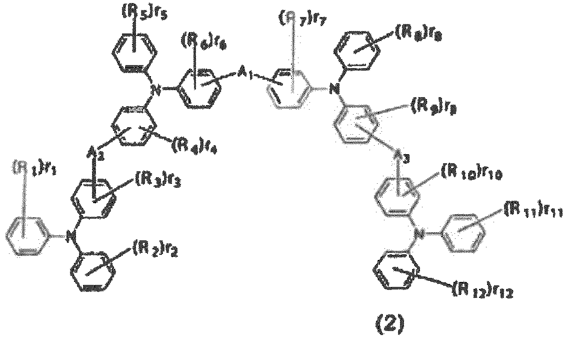 où R₁ à R₁₂ représentent un atome de deutérium, un atome de fluor, un atome de chlore, cyano, nitro, un alkyle linéaire ou ramifié ayant 1 à 6 atomes de carbone qui peut avoir un substituant, un cycloalkyle ayant 5 à 10 atomes de carbone qui peut avoir un substituant, un alcényle linéaire ou ramifié ayant 2 à 6 atomes de carbone qui peut avoir un substituant, un alkyloxy linéaire ou ramifié ayant 1 à 6 atomes de carbone qui peut avoir un substituant, un cycloalkyloxy ayant 5 à 10 atomes de carbone qui peut avoir un substituant, un groupe hydrocarboné aromatique substitué ou non substitué, un groupe hétérocyclique aromatique substitué ou non substitué, un groupe polycyclique condensé aromatique substitué ou non substitué, ou un aryloxy substitué ou non substitué ; r₁ à r₁₂ peuvent être identiques ou différents, r₁, r₂, r₅, r₈, r₁₁ et r₁₂ représentent 0 ou un entier allant de 1 à 5, et r₃, r₄, r₆, r₇, r₉ et r₁₀ représentent 0 ou un entier allant de 1 à 4, où lorsque r₁, r₂, r₅, r₈, r₁₁ et r₁₂ sont un entier allant de 2 à 5, ou lorsque r₃, r₄, r₆, r₇, r₉ et r₁₀ sont un entier allant de 2 à 4, R₁ à R₁₂, dont plusieurs sont liés au même cycle benzénique, peuvent être identiques ou différents et peuvent être liés les uns aux autres via une simple liaison, un méthylène substitué ou non substitué, un atome d'oxygène, ou un atome de soufre pour former un cycle ; et A₁, A₂ et A₃ peuvent être identiques ou différents, et représentent un groupe bivalent représenté par les formules de structure (B) à (G) suivantes, ou une simple liaison, 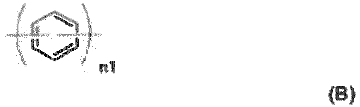 où n1 représente un entier allant de 1 à 3, 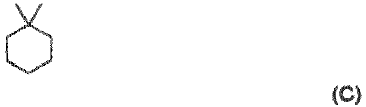 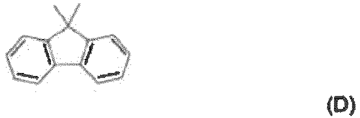
-CH₂- (E)

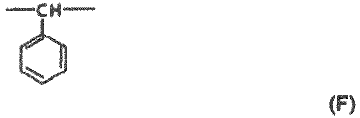

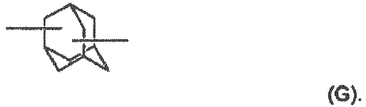

4. Dispositif électroluminescent organique selon la revendication 1, dans lequel la première couche de transport de trous (4) comprend un composé arylamine ayant une structure dans laquelle deux structures triphénylamine sont jointes au sein d'une molécule via une simple liaison ou un groupe bivalent qui ne contient pas d'hétéroatome.

5. Dispositif électroluminescent organique selon la revendication 4, dans lequel le composé arylamine ayant une structure dans laquelle deux structures triphénylamine sont jointes au sein d'une molécule via une simple liaison ou un groupe bivalent qui ne contient pas d'hétéroatome, est un composé arylamine représenté par la formule générale (3) suivante : 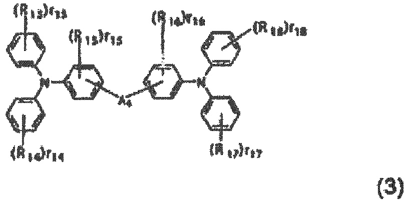 où R₁₃ à R₁₈ représentent un atome de deutérium, un atome de fluor, un atome de chlore, cyano, nitro, un alkyle linéaire ou ramifié ayant 1 à 6 atomes de carbone qui peut avoir un substituant, un cycloalkyle ayant 5 à 10 atomes de carbone qui peut avoir un substituant, un alcényle linéaire ou ramifié ayant 2 à 6 atomes de carbone qui peut avoir un substituant, un alkyloxy linéaire ou ramifié ayant 1 à 6 atomes de carbone qui peut avoir un substituant, un cycloalkyloxy ayant 5 à 10 atomes de carbone qui peut avoir un substituant, un groupe hydrocarboné aromatique substitué ou non substitué, un groupe hétérocyclique aromatique substitué ou non substitué, un groupe polycyclique condensé aromatique substitué ou non substitué, ou un aryloxy substitué ou non substitué ; r₁₃ à r₁₈ peuvent être identiques ou différents, r₁₃, r₁₄, r₁₇ et r₁₈ représentent 0 ou un entier allant de 1 à 5, et r₁₅ et r₁₆ représentent 0 ou un entier allant de 1 à 4, où lorsque r₁₃, r₁₄, r₁₇ et r₁₈ sont un entier allant de 2 à 5, ou lorsque r₁₅ et r₁₆ sont un entier allant de 2 à 4, R₁₃ à R₁₈, dont plusieurs sont liés au même cycle benzénique, peuvent être identiques ou différents et peuvent être liés les uns aux autres via une simple liaison, un méthylène substitué ou non substitué, un atome d'oxygène, ou un atome de soufre pour former un cycle ; et A₄ représente un groupe bivalent représenté par les formules de structure (C) à (G) suivantes, ou une simple liaison, 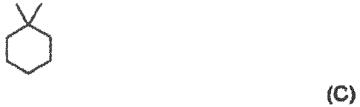 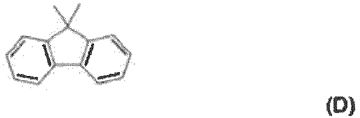
**-CH₂-** (E)

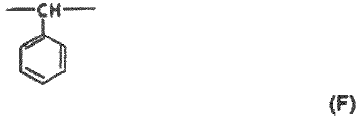

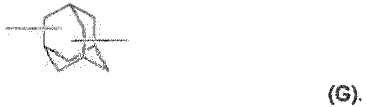

6. Dispositif électroluminescent organique selon la revendication 1, dans lequel la couche de transport d'électrons (7) comprend un composé de la formule générale (4) suivante ayant une structure de cycle anthracénique, 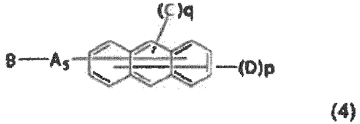 dans lequel A₅ représente un groupe bivalent d'un hydrocarbure aromatique substitué ou non substitué, un groupe bivalent d'un hétérocycle aromatique substitué ou non substitué, un groupe bivalent d'un aromatique polycyclique condensé substitué ou non substitué, ou une simple liaison ; B représente un groupe hétérocyclique aromatique substitué ou non substitué ; C représente un groupe hydrocarboné aromatique substitué ou non substitué, un groupe hétérocyclique aromatique substitué ou non substitué, ou un groupe polycyclique condensé aromatique substitué ou non substitué ; les D peuvent être identiques ou différents et représentent un atome d'hydrogène, un atome de deutérium, un atome de fluor, un atome de chlore, cyano, trifluorométhyle, un alkyle linéaire ou ramifié ayant 1 à 6 atomes de carbone, un groupe hydrocarboné aromatique substitué ou non substitué, un groupe hétérocyclique aromatique substitué ou non substitué, ou un groupe polycyclique condensé aromatique substitué ou non substitué ; et p représente 7 ou 8, et q représente 1 ou 2 tout en maintenant une relation telle que la somme de p et q fasse 9.

7. Dispositif électroluminescent organique selon la revendication 6, dans lequel le composé ayant une structure de cycle anthracénique est un composé ayant la formule générale (4a) suivante ayant une structure de cycle anthracénique, 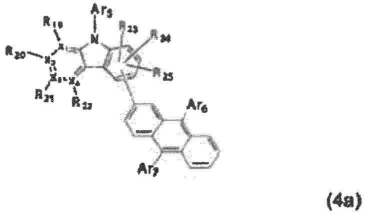 dans lequel Ar₅, Ar₆ et Ar₇ peuvent être identiques ou différents, et représentent un groupe hydrocarboné aromatique substitué ou non substitué, un groupe hétérocyclique aromatique substitué ou non substitué, ou un groupe polycyclique condensé aromatique substitué ou non substitué ; R₁₉ à R₂₅ peuvent être identiques ou différents, et représentent un atome d'hydrogène, un atome de deutérium, un atome de fluor, un atome de chlore, cyano, nitro, un alkyle linéaire ou ramifié ayant 1 à 6 atomes de carbone qui peut porter un substituant, un cycloalkyle ayant 5 à 10 atomes de carbone qui peut avoir un substituant, un alcényle linéaire ou ramifié ayant 2 à 6 atomes de carbone qui peut avoir un substituant, un alkyloxy linéaire ou ramifié ayant 1 à 6 atomes de carbone qui peut avoir un substituant, un cycloalkyloxy ayant 5 à 10 atomes de carbone qui peut avoir un substituant, un groupe hydrocarboné aromatique substitué ou non substitué, un groupe hétérocyclique aromatique substitué ou non substitué, un groupe polycyclique condensé aromatique substitué ou non substitué, ou un aryloxy substitué ou non substitué, où R₁₉ à R₂₅ peuvent être liés les uns aux autres via une simple liaison, un méthylène substitué ou non substitué, un atome d'oxygène, ou un atome de soufre pour former un cycle ; et X₁, X₂, X₃ et X₄ représentent un atome de carbone ou un atome d'azote, où seul l'un parmi X₁, X₂, X₃ et X₄ est un atome d'azote et dans ce cas, l'atome d'azote n'a pas d'atome d'hydrogène ni de substituant pour R₁₉ à R₂₂.

8. Dispositif électroluminescent organique selon la revendication 6, dans lequel le composé ayant une structure de cycle anthracénique est un composé de la formule générale (4b) suivante ayant une structure de cycle anthracénique, 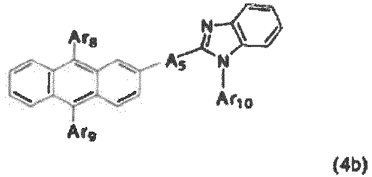 dans lequel A₅ représente un groupe bivalent d'un hydrocarbure aromatique substitué ou non substitué, un groupe bivalent d'un hétérocycle aromatique substitué ou non substitué, un groupe bivalent d'un aromatique polycyclique condensé substitué ou non substitué, ou une simple liaison ; et Ar₈, Arg et Ar₁₀ peuvent être identiques ou différents, et représentent un groupe hydrocarboné aromatique substitué ou non substitué, un groupe hétérocyclique aromatique substitué ou non substitué, ou un groupe polycyclique condensé aromatique substitué ou non substitué.
